(19) Europäisches Patentamt
European Patent Office
Office européen des brevets

(11) EP 4 674 840 A1

(12) EUROPEAN PATENT APPLICATION
published in accordance with Art. 153(4) EPC

(43) Date of publication:
07.01.2026 Bulletin 2026/02

(21) Application number: 24763063.5

(22) Date of filing: 23.02.2024

(51) International Patent Classification (IPC):
*C07D 211/96* (2006.01) *A61K 31/445* (2006.01)
*A61P 35/00* (2006.01) *A61P 37/00* (2006.01)

(52) Cooperative Patent Classification (CPC):
**A61K 31/445; A61P 35/00; A61P 37/00; C07D 211/96**

(86) International application number:
**PCT/CN2024/078293**

(87) International publication number:
**WO 2024/179381 (06.09.2024 Gazette 2024/36)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**

(30) Priority: **28.02.2023 CN 202310181337**
**22.09.2023 CN 202311231478**

(71) Applicant: **Sichuan Kelun-Biotech Biopharmaceutical Co., Ltd.**
**Chengdu, Sichuan 611138 (CN)**

(72) Inventors:
- **TIAN, Qiang**
  **Chengdu, Sichuan 611138 (CN)**
- **ZHANG, Yitao**
  **Chengdu, Sichuan 611138 (CN)**
- **YE, Jian**
  **Chengdu, Sichuan 611138 (CN)**
- **SONG, Hongmei**
  **Chengdu, Sichuan 611138 (CN)**
- **GE, Junyou**
  **Chengdu, Sichuan 611138 (CN)**

(74) Representative: **Meissner Bolte Partnerschaft mbB**
**Patentanwälte Rechtsanwälte**
**Postfach 86 06 24**
**81633 München (DE)**

# (54) CHEMICAL COUPLING LINKER AND USE THEREOF

(57) The present invention relates to a chemical coupling linker, and an antibody-drug conjugate prepared with the linker. The present invention further relates to a use of the antibody-drug conjugate in treating related disease of tumors, etc.

EP 4 674 840 A1

## Description

The present application is based on the application with CN application number 202310181337.6 and application date February 28, 2023, and the application with CN application number 202311231478.0 and application date September 22, 2023, and claims priority to these applications. The disclosures of the CN applications are hereby introduced into the present application in their entirety.

## Technical Field

The present invention relates to a novel linker for chemical coupling, antibody-drug conjugates prepared with the linker, and applications in the treatment of related diseases of tumors.

## Background Art

In recent years, antibody-drug conjugates have become one of the hot directions for the precise treatment of tumors, autoimmune diseases, etc.. Antibody-drug conjugates (ADCs) are formed by coupling monoclonal antibodies that target specific antigens with effector molecules (e.g., cytotoxic drugs) through linkers, and they possess both the biological activities of traditional small molecules and the targeting ability of antibodies.

Antibody-drug conjugates consist of three components: antibodies, linkers, and effector molecules. The methods for connecting antibodies and linker-effector molecules are mainly divided into non-site-specific conjugation methods and site-specific conjugation methods. In the early days, non-site-specific conjugation methods were used, i.e., the effector molecules were conjugated to the amino acid residues on antibodies by chemical methods, for example, the effector molecules were randomly conjugated to lysine and cysteine residues on antibodies. Though it did not involve the transformation or modification of antibodies, the number of effector molecules and the conjugation sites could not be determined, resulting in poor uniformity. Currently, the commonly used site-specific conjugation methods often achieve uniform linking of effector molecules at specific sites through specific conjugation. Site-specific conjugation-produced antibody-drug conjugates can reduce fluctuations in efficacy, pharmacokinetics, and quality control due to variations in conjugation sites and the number of conjugations.

Currently, common site-specific conjugation methods include THIOMAB technology, non-natural amino acid conjugation technology, glutamine enzymatic conjugation technology, transpeptidase conjugation technology, and ThioBridge technology. Among theses methods, antibody modification via antibody engineering or enzymatic coupling may affect antibody structural stability.

Patent application WO2012007896A1 discloses a linker structure based on polyethylene glycol carboxylic acid fluorophenyl ester, which is used to couple a polypeptide molecule to the K188 site of an antibody. Patent application WO2013173392A1 discloses a coupling linker structure based on piperidine amide that links to a lysine residue of an antibody via a piperidine linker. Although the coupling methods disclosed in WO2012007896A1 and WO2013173392A1 can achieve coupling to light chain lysine to some extent, they exhibit poor uniformity and have room for improvement in site-specific conjugation proportions.

The development of a site-specific, hydrophilic coupling linker structure remains highly significant for the development of antibody-conjugated drugs with good efficacy and safety.

## Contents of the invention

The present invention provides a novel linker for chemical coupling, comprising a piperidine sulfonyl carbamate moiety capable of selectively coupling with lysine at a defined site on the antibody light chain. The resulting antibody-drug conjugate exhibits high uniformity and clear anti-tumor efficacy. The coupling conditions are mild and the operations are simple.

Compounds

In one aspect, the present invention provides a compound or a pharmaceutically acceptable salt, ester, stereoisomer, tautomer, polymorph, solvate, N-oxide or isotope-labeled compound thereof, wherein the compound has a structure represented by Formula I:

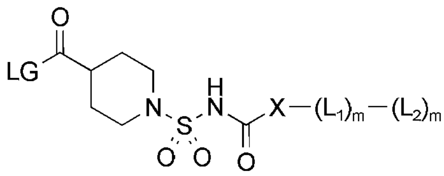

Formula I

wherein:

LG is a leaving group or a reactive group;

X is selected from the group consisting of -O-, $-NR^2-$ and $-CHR^3-$; $R^2$ and $R^3$ are each independently selected from the group consisting of hydrogen and $C_{1-6}$ alkyl; the alkyl is optionally substituted with one or more groups independently selected from the group consisting of amino, alkylamino, nitrogen-containing heterocyclyl, sulfonic acid group, carboxylic acid group, quaternary ammonium salt, hydroxyl and alkoxy;

each $L_1$ is independently selected from the group consisting of single bond, $C_{1-6}$ alkylene, $-(CH_2CH_2O)_y-$, $C_{2-6}$ alkenylene, an amino acid residue, a fragment of a polypeptide formed with 2 to 10 amino acids, 4- to 16-membered heterocyclylene, 5- to 16-membered heteroarylene, phenylene, amino, -CO-NH- and -NH-CO-; the $C_{1-6}$ alkylene, amino, $C_{2-6}$ alkenylene, 4- to 16-membered heterocyclylene, 5- to 16-membered heteroarylene, phenylene, -CO-NH- and -NH-CO- are optionally substituted with one or more groups independently selected from the group consisting of sulfonic acid group, phosphoric acid group, carboxylic acid group, amide group, quaternary ammonium salt, hydroxyl, alkyl, alkoxy, alkoxyalkyl, alkynyl and azido;

each $L_2$ is independently selected from the group consisting of $C_{2-6}$ alkynyl, tetrazinyl, methyltetrazinyl, trans-cyclooctenyl, benzoazacyclooctynyl, (1R,8S,9S)-bicyclo[6.1.0]non-4-yn-9-yl, azido, $C_{1-6}$ alkylacyl, aldehyde group, hydroxylamine group, oxime group, 4- to 16-membered heterocyclyl and 5- to 16-membered heteroaryl;

each m is independently an integer of 1 to 10;

y is an integer of 1 to 20.

In some embodiments, LG is selected from the group consisting of $-OR^1$, hydroxyl, halogen (e.g., chlorine), $C_{1-6}$ haloalkyl (e.g., chloromethyl) and 5- to 12-membered heteroaryl (e.g., imidazolyl), $R^1$ is selected from the group consisting of $C_{1-6}$ alkylacyl, maleimido, succinimido, sulfosuccinimido and phenyl, and the phenyl is optionally substituted with 1, 2, 3, 4 or 5 groups independently selected from the group consisting of nitro, cyano, sulfone group, sulfonic acid group, fluorine and chlorine.

In some embodiments, LG is selected from $-OR^1$ and imidazolyl, $R^1$ is selected from the group consisting of succinimido, sulfosuccinimido and phenyl, and the phenyl is optionally substituted with 1, 2, 3, 4 or 5 groups independently selected from the group consisting of nitro, sulfonic acid group and fluorine.

In some embodiments, LG is pentafluorophenoxy.

In some embodiments, X is selected from the group consisting of -O-, $-NR^2-$ and $-CHR^3-$; $R^2$ and $R^3$ are each independently selected from hydrogen and $C_{1-6}$ alkyl; and the alkyl is optionally substituted with one or more groups independently selected from the group consisting of amino, $C_{1-6}$ alkylamino, 5- to 12-membered nitrogen-containing heterocyclyl, sulfonic acid group, carboxylic acid group, quaternary ammonium salt, hydroxyl and $C_{1-6}$ alkoxy.

In some embodiments, X is selected from the group consisting of -O-, -NH- and $-CH_2-$.

In some embodiments, X is -O-.

In some embodiments, each $L_1$ is independently selected from the group consisting of single bond, $C_{1-6}$ alkylene, $-(CH_2CH_2O)_y-$, $C_{2-6}$ alkenylene, an amino acid residue, a fragment of a polypeptide formed with 2 to 10 amino acids, 4- to 16-membered heterocyclylene, 5- to 16-membered heteroarylene, phenylene, amino, -CO-NH- and -NH-CO-, wherein the $C_{1-6}$ alkylene, amino, 4- to 16-membered heterocyclylene, 5- to 16-membered heteroarylene, phenylene, -CO-NH- and -NH-CO- are optionally substituted with one or more groups independently selected from the group consisting of sulfonic acid group, phosphoric acid group, carboxylic acid group, amide group, quaternary ammonium salt, hydroxyl, $C_{1-6}$ alkyl, $C_{1-6}$ alkoxy, $C_{2-6}$ alkoxyalkyl, $C_{2-6}$ alkynyl and azido.

In some embodiments, each $L_1$ is independently selected from the group consisting of single bond, $C_{1-6}$ alkylene, $-(CH_2CH_2O)_y-$, phenyl, -CO-NH- and -NH-CO-, y is an integer of 1 to 10, such as an integer of 1 to 5, and m is an integer of 1

to 5.

In some embodiments, each $L_1$ is independently selected from single bond and $C_{1-4}$ alkylene, and m is 1 or 2.

In some embodiments, each $L_2$ is independently selected from the group consisting of $C_{2-6}$ alkynyl, methyltetrazinyl, trans-cyclooctenyl, benzoazacyclooctynyl, (1R,8S,9S)-bicyclo[6.1.0]non-4-yn-9-yl, azido and $C_{1-6}$ carbonyl, and m is an integer of 1 to 5.

In some embodiments, each $L_2$ is independently selected from single bond and $C_{2-6}$ alkynyl, and m is 1.

In some embodiments,

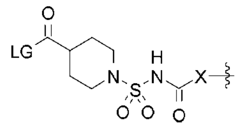

is selected from the group consisting of the following structures:

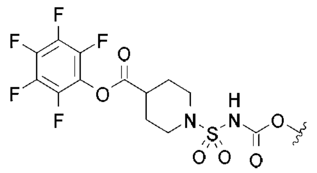 , 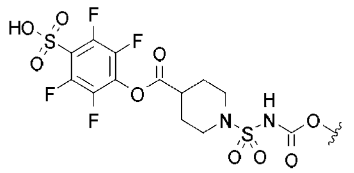 , 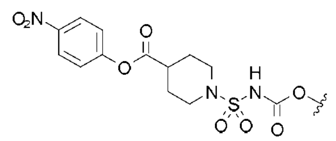 ,

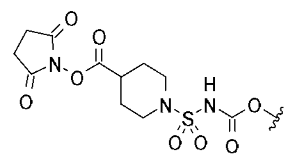 , 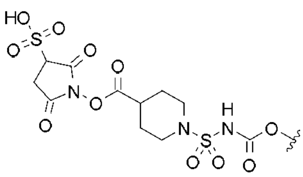 , 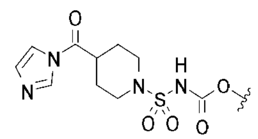 ,

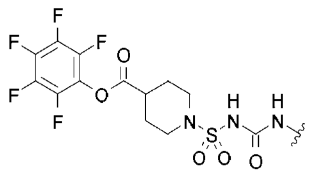 , 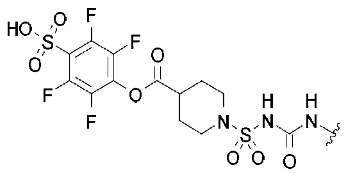 , 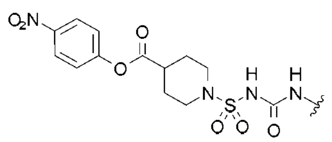 ,

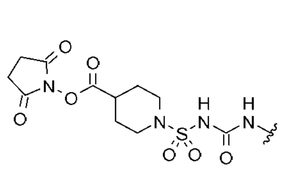 , 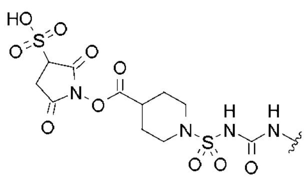 , 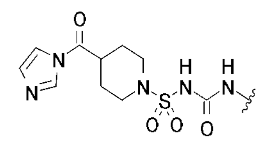 ,

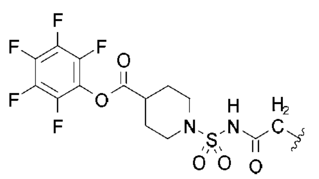 , 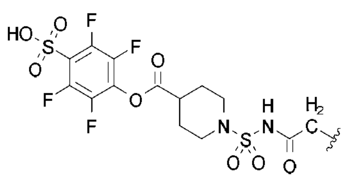 , 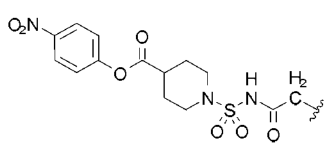 ,

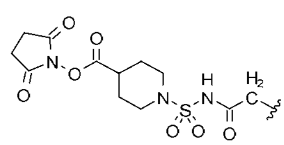 , 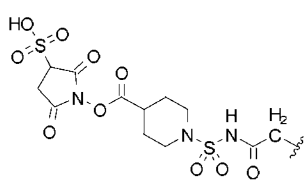 , 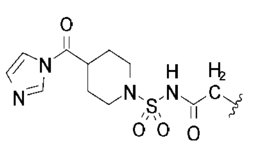 .

In some embodiments, the compound of Formula I is selected from:

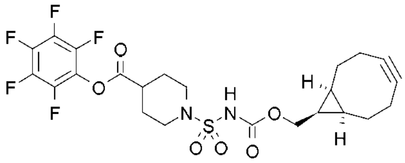
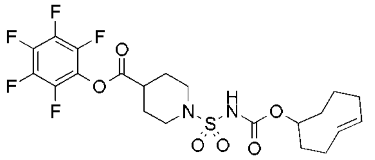
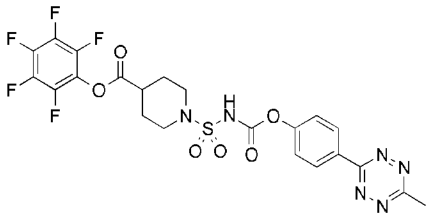
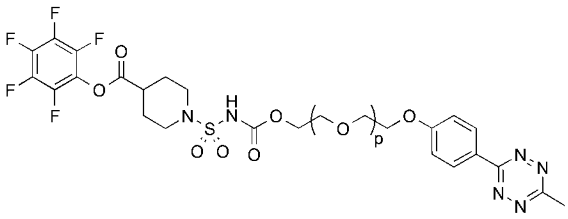
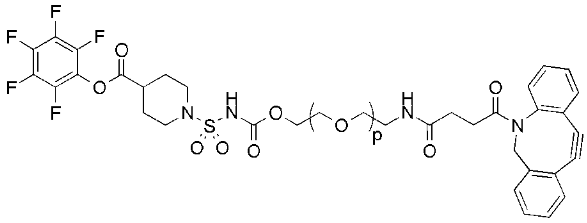
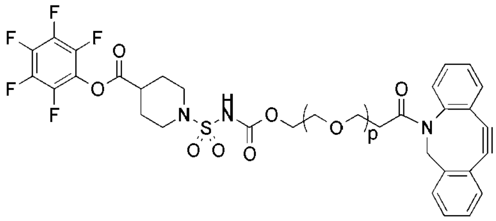
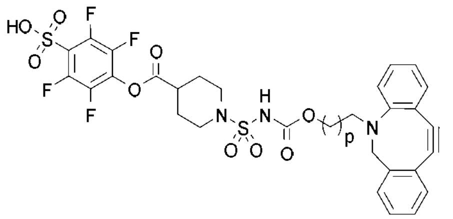
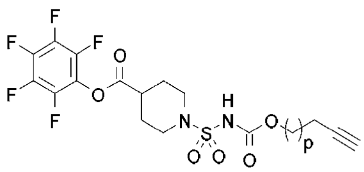
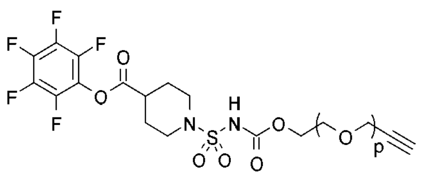
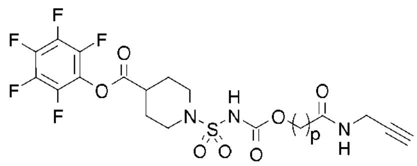
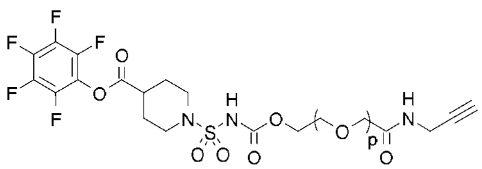
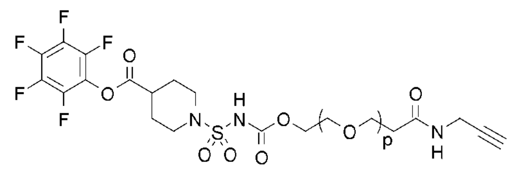
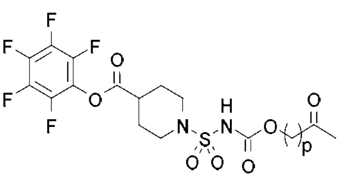
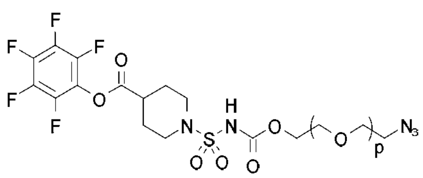

p is 0 or an integer of 1 to 20.

In another aspect, the present invention provides a compound or a pharmaceutically acceptable salt, ester, stereoisomer, tautomer, polymorph, solvate, N-oxide or isotope-labeled compound thereof, wherein the compound has a structure represented by Formula II:

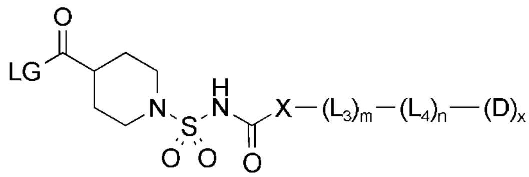

Formula II

wherein:

LG is a leaving group or a reactive group;

X is selected from the group consisting of -O-, $-NR^2-$ and $-CHR^3-$; $R^2$ and $R^3$ are each independently selected from the group consisting of hydrogen and $C_{1-6}$ alkyl; the $C_{1-6}$ alkyl is optionally substituted with one or more groups independently selected from the group consisting of amino, alkylamino, nitrogen-containing heterocyclyl, sulfonic acid group, carboxylic acid group, quaternary ammonium salt, hydroxyl and alkoxy;

each $L_3$ is independently selected from the group consisting of single bond, $C_{1-6}$ alkylene, -O-, $-(CH_2CH_2O)_y-$, oxime group, an amino acid residue, a fragment of a polypeptide formed with 2 to 10 amino acids, 4- to 16-membered heterocyclylene, 5- to 16-membered heteroarylene, amino, acyl, -CO-NH- and -NH-CO-; the $C_{1-6}$ alkylene, oxime group, amino, 4- to 16-membered heterocyclylene, 5- to 16-membered heteroarylene, -CO-NH- and -NH-CO- are optionally substituted with one or more groups independently selected from the group consisting of sulfonic acid group, phosphoric acid group, carboxylic acid group, amide group, quaternary ammonium salt, hydroxyl, alkyl, alkoxy, alkoxyalkyl, alkynyl and azido;

each $L_4$ is independently selected from the group consisting of an amino acid residue, a fragment of a polypeptide formed with 2 to 10 amino acids, glycosyl, phenylene, benzyl, benzyloxy, benzyloxycarbonyl, aminobenzyloxycarbonyl, acylbenzyloxycarbonyl, 5- to 16-membered heteroarylene, $C_{1-6}$ alkylene, $-(CH_2CH_2O)_y-$, acyl and amino; the $C_{1-6}$ alkylene and amino are optionally substituted with one or more groups independently selected from the group consisting of sulfonic acid group, phosphoric acid group, carboxylic acid group, amide group, sulfone group, sulfoxide group, quaternary ammonium salt, hydroxyl, alkyl, alkoxy, alkoxyalkyl, alkylaminoalkyl, alkynyl and azido, and the phenylene, benzyl, benzyloxy, benzyloxycarbonyl, aminobenzyloxycarbonyl, acylbenzyloxycarbonyl are optionally substituted with pyranosyl or furanosyl; each D is independently selected from the group consisting of a fragment of an effector molecule, and the effector molecule is, for example, a cytotoxin, an immunostimulant, a pro-apoptotic agent, a protein degrading agent or a hormone receptor modulator;

m is an integer of 1 to 20;

n is an integer of 1 to 20;

x is an integer of 1 to 10;

y is an integer of 1 to 20.

In some embodiments, LG is selected from the group consisting of $-OR^1$, hydroxyl, halogen (e.g., chlorine), $C_{1-6}$ haloalkyl (e.g., chloromethyl), 5- to 12-membered heteroaryl (e.g., imidazolyl), $R^1$ is selected from the group consisting of $C_{1-6}$ alkylacyl, maleimido, succinimido, sulfosuccinimido and phenyl, and the phenyl is optionally substituted with 1, 2, 3, 4 or 5 groups independently selected from the group consisting of nitro, cyano, sulfone group, sulfonic acid group, fluorine and chlorine.

In some embodiments, LG is selected from $-OR^1$ and imidazolyl, $R^1$ is selected from the group consisting of succinimido, sulfosuccinimido and phenyl, and the phenyl is optionally substituted with 1, 2, 3, 4, or 5 groups independently selected from the group consisting of nitro, sulfonic acid group and fluorine.

In some embodiments, LG is pentafluorophenoxy.

In some embodiments, X is selected from the group consisting of -O-, $-NR^2-$ and $-CHR^3-$; $R^2$ and $R^3$ are each independently selected from the group consisting of hydrogen and $C_{1-6}$ alkyl; the $C_{1-6}$ alkyl is optionally substituted with one or more groups independently selected from the group consisting of amino, $C_{1-6}$ alkylamino, 5- to 12-membered nitrogen-containing heterocyclyl, sulfonic acid group, carboxylic acid group, quaternary ammonium salt, hydroxyl and $C_{1-6}$

alkoxy.

In some embodiments, X is selected from the group consisting of -O-, -NH- and $-CH_2-$.

In some embodiments, X is -O-.

In some embodiments, each $L_3$ is independently selected from the group consisting of single bond, $C_{1-6}$ alkylene, -O-, $-(CH_2CH_2O)_y-$, oxime group, an amino acid residue, a fragment of a polypeptide formed with 2 to 10 amino acids, 4- to 16-membered heterocyclylene, 5- to 16-membered heteroarylene, amino, acyl, -CO-NH- and -NH-CO-; wherein the $C_{1-6}$ alkylene, amino, 4- to 16-membered heterocyclylene, 5- to 16-membered heteroarylene, -CO-NH- and -NH-CO- are optionally substituted with one or more groups independently selected from the group consisting of sulfonic acid group, phosphoric acid group, carboxylic acid group, amide group, quaternary ammonium salt, hydroxyl, $C_{1-6}$ alkyl, $C_{1-6}$ alkoxy, $C_{2-6}$ alkoxyalkyl, $C_{2-6}$ alkynyl and azido, y is an integer of 1 to 20, and m is an integer of 1 to 20.

In some embodiments, each $L_3$ is independently selected from the group consisting of $C_{1-6}$ alkylene, -O-, $-(CH_2CH_2O)_y-$, oxime group, 5- to 16-membered heteroarylene, acyl, -CO-NH- and -NH-CO-, wherein the $C_{1-6}$ alkylene, oxime group, 5- to 16-membered heteroarylene, acyl, -CO-NH- and -NH-CO- are optionally substituted with one or more groups independently selected from the group consisting of sulfonic acid group, phosphoric acid group, carboxylic acid group, amide group, quaternary ammonium salt, hydroxyl, alkyl, alkoxy, alkoxyalkyl, alkynyl and azido, y is an integer of 1 to 20, and m is an integer of 1 to 20.

In some embodiments, each $L_3$ is independently selected from the group consisting of $C_{1-6}$ alkylene, -O-, $-(CH_2CH_2O)_y-$, oxime group, 5- to 16-membered heteroarylene, acyl, -CO-NH- and -NH-CO-, wherein the $C_{1-6}$ alkylene, oxime group, 5- to 16-membered heteroarylene, acyl, -CO-NH- and -NH-CO- are optionally substituted with one or more groups independently selected from the group consisting of sulfonic acid group, phosphoric acid group, carboxylic acid group, amide group, quaternary ammonium salt, hydroxyl, $C_{1-6}$ alkyl, $C_{1-6}$ alkoxy, $C_{2-6}$ alkoxyalkyl, $C_{2-6}$ alkynyl and azido, y is an integer of 1 to 20, and m is an integer of 1 to 20.

In some embodiments, each $L_3$ is independently selected from the group consisting of $C_{1-6}$ alkylene, -O-, $-(CH_2CH_2O)_y-$, oxime group, 5- to 16-membered heteroarylene, acyl, -CO-NH- and -NH-CO-, y is an integer of 1 to 10, and m is an integer of 1 to 15.

In some embodiments, each $L_4$ is independently selected from the group consisting of an amino acid residue, a fragment of a polypeptide formed with 2 to 10 amino acids, glycosyl, phenylene, benzyl, benzyloxy, benzyloxycarbonyl, aminobenzyloxycarbonyl, acylbenzyloxycarbonyl, 5- to 6-membered heteroarylene, $C_{1-6}$ alkylene, $-(CH_2CH_2O)_y-$, acyl and amino; wherein the $C_{1-6}$ alkylene and amino are optionally substituted with one or more groups independently selected from the group consisting of sulfonic acid group, phosphoric acid group, carboxylic acid group, amide group, sulfone group, sulfoxide group, quaternary ammonium salt, hydroxyl, $C_{1-6}$ alkyl, $C_{1-6}$ alkoxy, $C_{2-6}$ alkoxyalkyl, $C_{1-6}$ alkylaminoalkyl, $C_{2-6}$ alkynyl and azido; wherein the phenylene, benzyl, benzyloxy, benzyloxycarbonyl, aminobenzyloxycarbonyl, acylbenzyloxycarbonyl are optionally substituted with glucuronic acid, galacturonic acid, glucose, galactose or mannose.

In some embodiments, each $L_4$ is independently selected from the group consisting of an amino acid residue, a fragment of a polypeptide formed with 2 to 10 amino acids, aminobenzyloxycarbonyl, $C_{1-6}$ alkylene and amino, wherein the $C_{1-6}$ alkylene and amino are optionally substituted with one or more groups independently selected from the group consisting of sulfonic acid group, phosphoric acid group, carboxylic acid group, amide group, sulfone group, sulfoxide group, quaternary ammonium salt, hydroxyl, alkyl, alkoxy, alkoxyalkyl, alkylaminoalkyl, alkynyl and azido, wherein the aminobenzyloxycarbonyl is optionally substituted with pyranosyl or furanosyl.

In some embodiments, each $L_4$ is independently selected from the group consisting of an amino acid residue, a fragment of a polypeptide formed with 2 to 10 amino acids, aminobenzyloxycarbonyl, $C_{1-6}$ alkylene and amino, wherein the $C_{1-6}$ alkylene and amino are optionally substituted with one or more groups independently selected from the group consisting of sulfonic acid group, phosphoric acid group, carboxylic acid group, amide group, sulfone group, sulfoxide group, quaternary ammonium salt, hydroxyl, $C_{1-6}$ alkyl, $C_{1-6}$ alkoxy, $C_{2-6}$ alkoxyalkyl, $C_{2-6}$ alkylaminoalkyl, $C_{2-6}$ alkynyl and azido, wherein the aminobenzyloxycarbonyl is optionally substituted with glucuronic acid, galacturonic acid, glucose, galactose or mannose.

In some embodiments, the amino acid is selected from the group consisting of L-type natural amino acids, D-type non-natural amino acids, and their analogs or derivatives.

In some embodiments, the amino acid is selected from the group consisting of Ala, Arg, Asn, Asp, Cit, Cys, Gln, Glu, Gly, His, Ile, Leu, Lys, Met, Phe, Pro, Ser, Thr, Trp, Tyr, Val, $Lys(COCH_2CH_2(OCH_2CH_2)_yOCH_3)$, y is an integer of 1 to 20.

In some embodiments, the polypeptide formed with 2 to 10 amino acids is selected from the group consisting of Ala-Ala, Ala-Lys, Ala-Lys(Ac), Ala-Pro, Gly-Glu, Gly-Gly, Phe-Lys, Phe-Lys(Ac), Val-Ala, Val-Lys, Val-Lys(Ac), Val-Cit, Val-Arg, Ala-Ala-Ala, Ala-Ala-Asn, Ala-Ala-Gly, Leu-Ala-Glu, Gly-Gly-Arg, Gly-Glu-Gly, Gly-Gly-Gly, Gly-Ser-Lys, Gly-Ala-Ala, Glu-Val-Ala, Glu-Val-Cit,Glu-Val-Arg, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, and Gly-Gly-Gly-Gly-Gly.

In some embodiments,

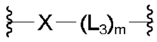

is selected from the group consisting of the following structures:

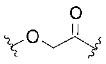

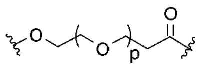

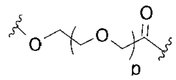

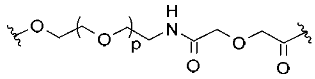

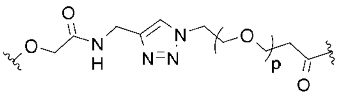

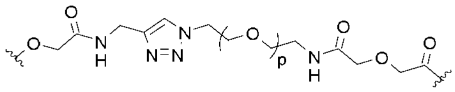

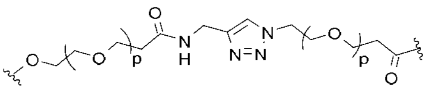

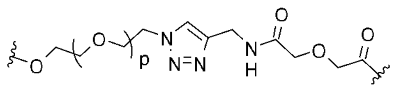

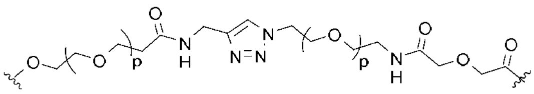

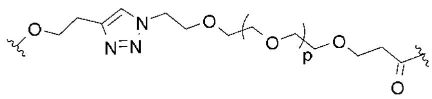

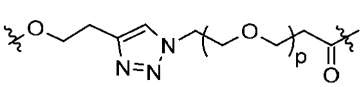

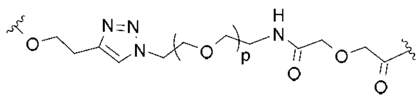

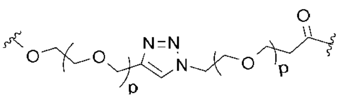

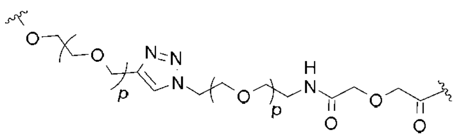

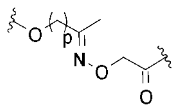

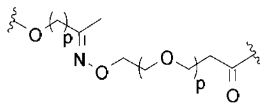

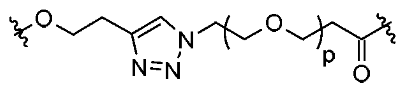 ;

p is 0 or an integer of 1 to 20;

In another aspect, the present invention provides a compound moiety

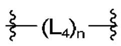 ,

which has the following structure:

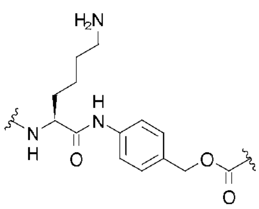

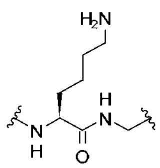

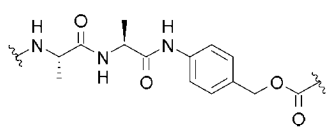

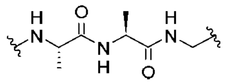
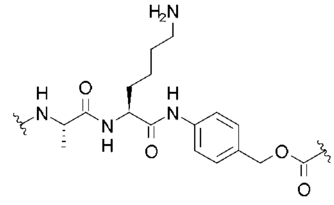
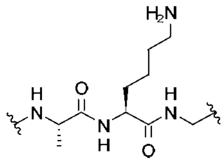

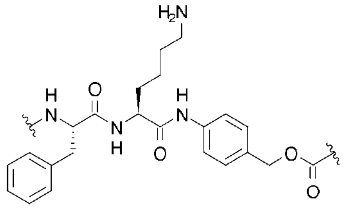
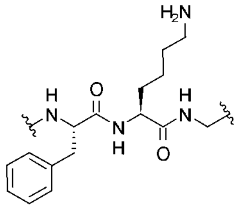
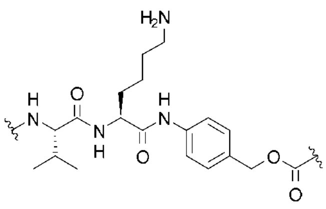

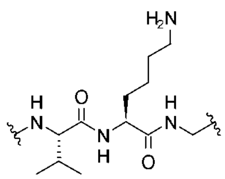
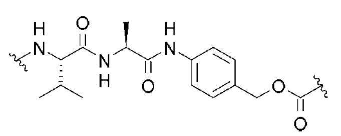
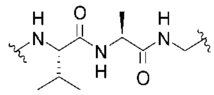

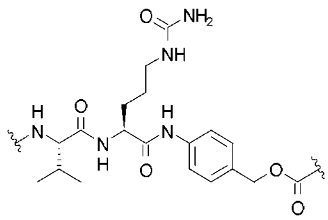
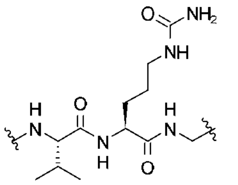
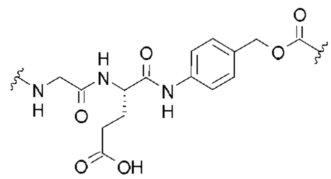

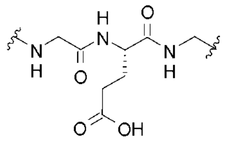
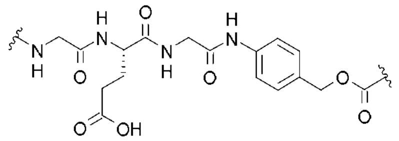
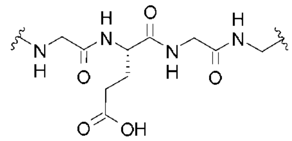

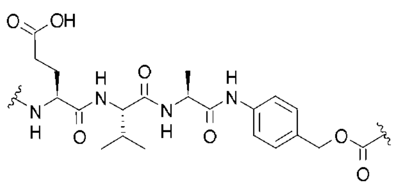
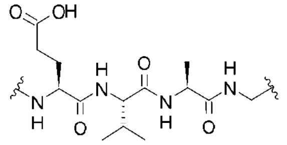
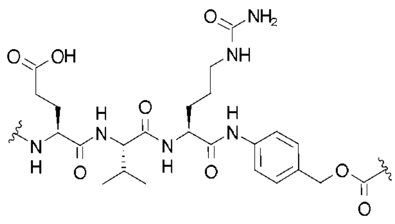

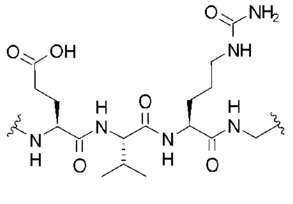
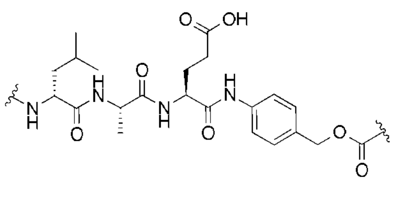
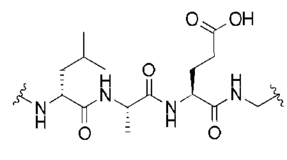

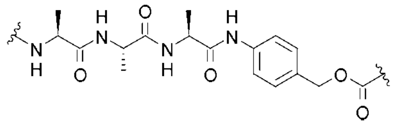
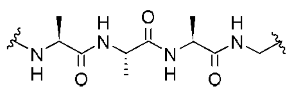
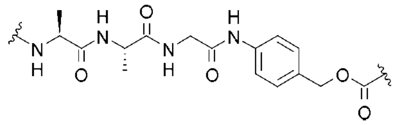

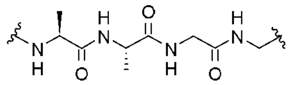
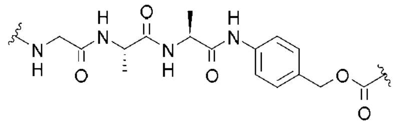
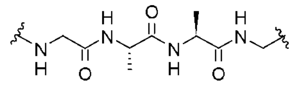

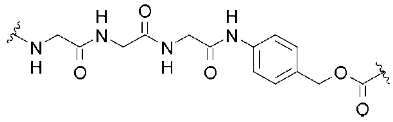
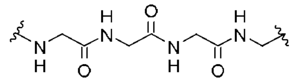
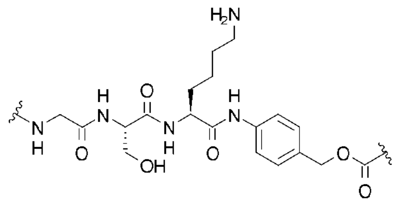

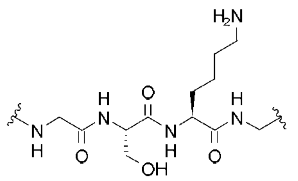
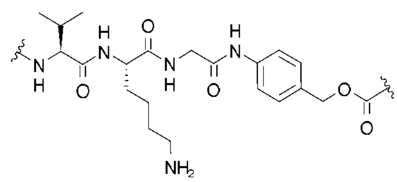
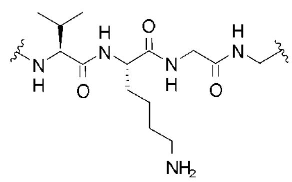

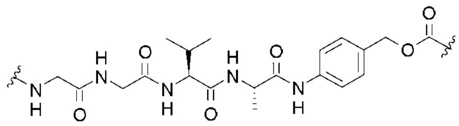
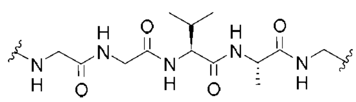

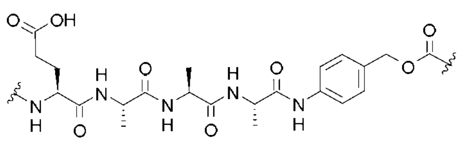
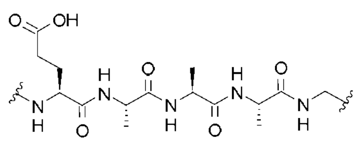

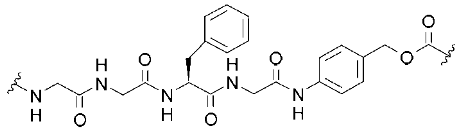
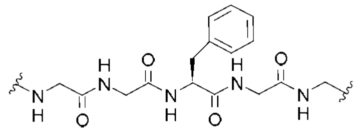

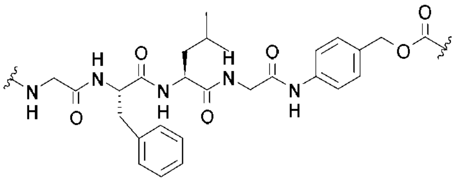
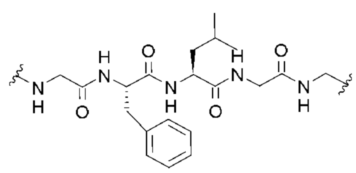
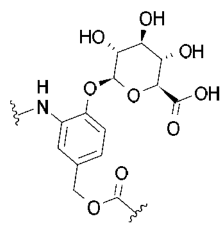

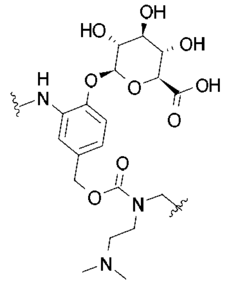
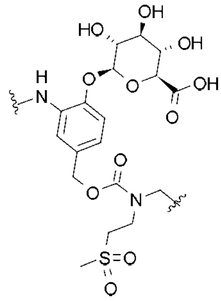
;

In some embodiments,

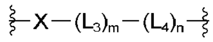

is selected from the group consisting of the following structures:

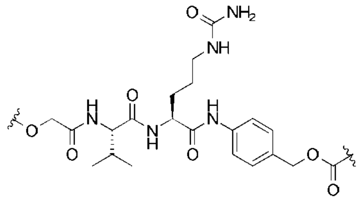

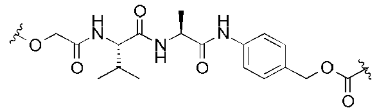

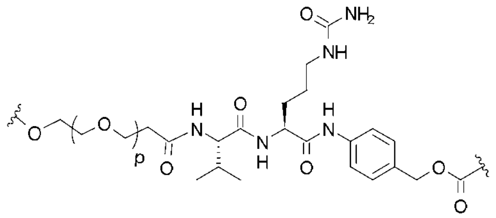

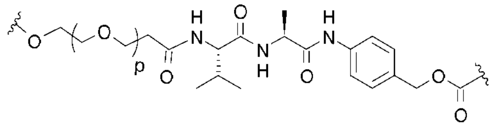

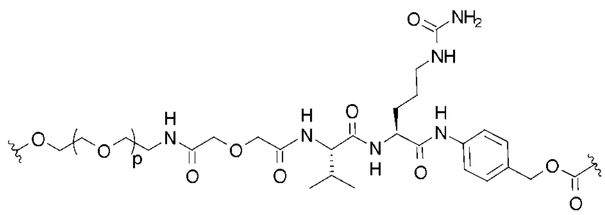

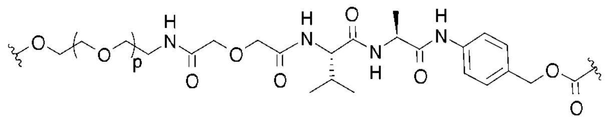

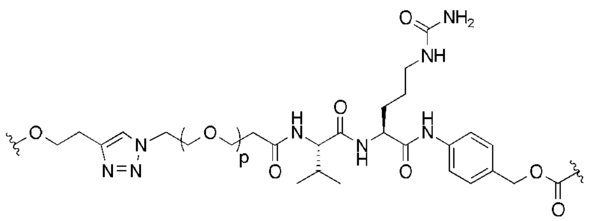

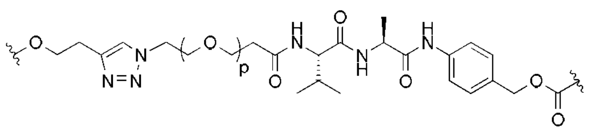

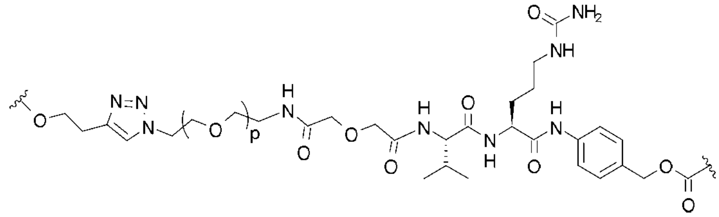

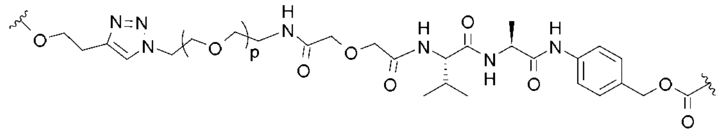

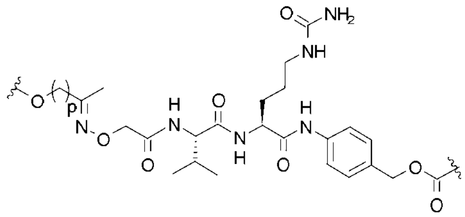

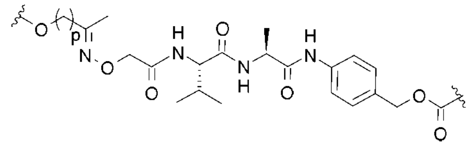

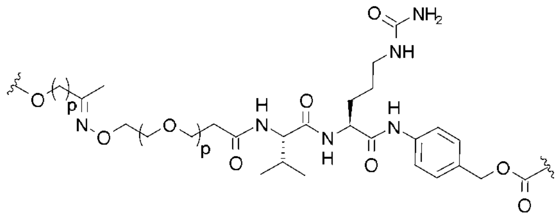

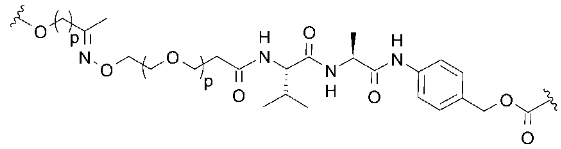

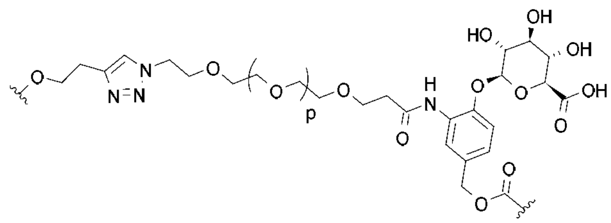
;

p is 0 or an integer of 1 to 20.

In some embodiments, p is 0 or an integer of 1 to 10, or p is 0 or an integer of 1 to 8, or p is 0, 1, 2, 3, 4, 5, 6, 7, 8, 9, 10, 11, 12, 13, 14, 15, 16, 17, 18, 19 or 20.

In some embodiments, the effector molecule is selected from the group consisting of microtubulin inhibitors, such as auristatins, maytansinoids; DNA intercalators, such as pyrrolobenzodiazepine (PBD); DNA topoisomerase inhibitors, such as topoisomerase I inhibitors (e.g., camptothecin, hydroxycamptothecin, 9-aminocamptothecin, SN-38, irinotecan, topotecan, belotecan, rubitecan, exatecan) or topoisomerase II inhibitors (e.g., doxorubicin, PNU-159682, duocarmycin, daunorubicin, mitoxantrone, podophyllotoxin or etoposide); RNA polymerase inhibitors, such as $\alpha$-amanitin; and pharmaceutically acceptable salts, esters and analogs of the above agents.

In some embodiments, the effector molecule is selected from the group consisting of topoisomerase I inhibitors (e.g., camptothecin, hydroxycamptothecin, 9-aminocamptothecin, SN-38, irinotecan, topotecan, belotecan, rubitecan, exatecan), MMAE, and MMAE derivatives.

In some embodiments, the effector molecule is selected from:

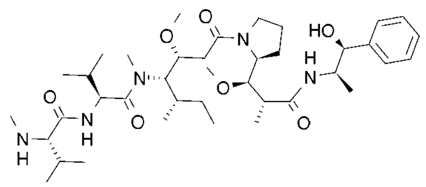
,
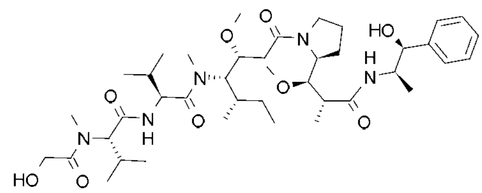
,

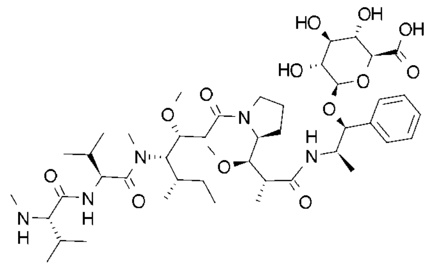, 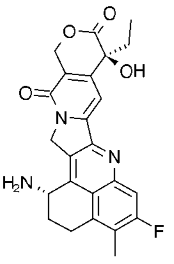, 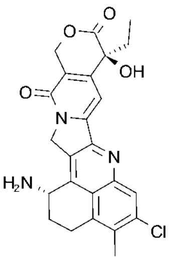, 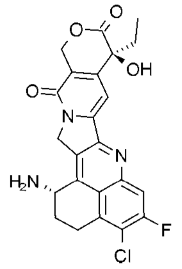,

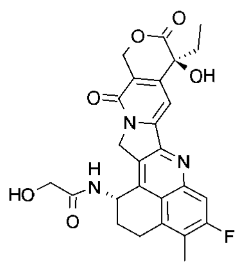, 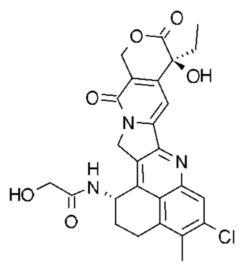, 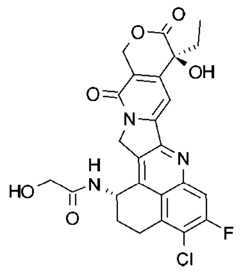, 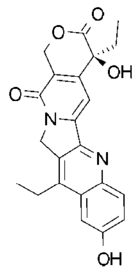, 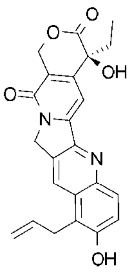,

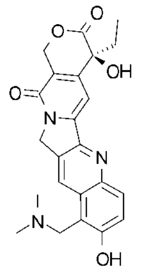, 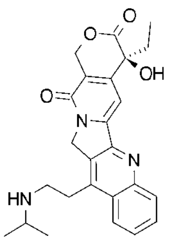, 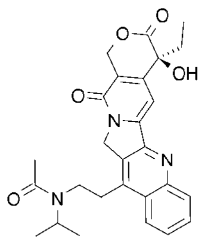, 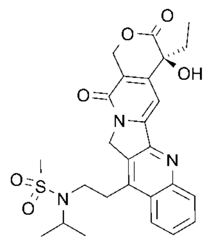, 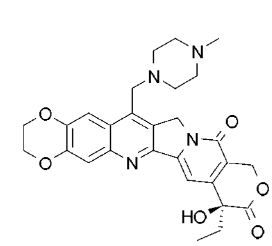,

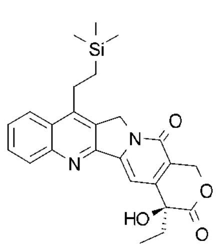, 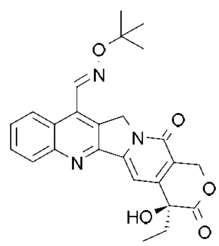.

In some embodiments, D is a structural moiety formed through the dehydrogenation of the effector molecule.

In some embodiments, the effector molecule is connected to $L_4$ through an amino or hydroxyl group on the effector molecule.

In some embodiments, D is selected from:

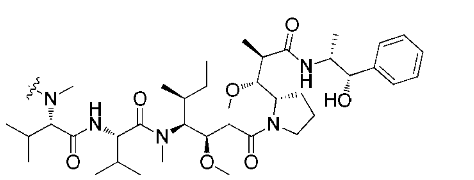, 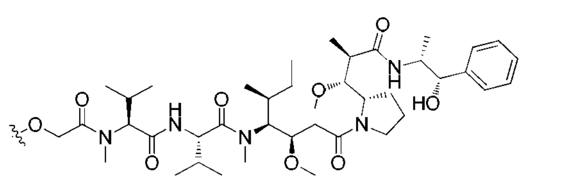,

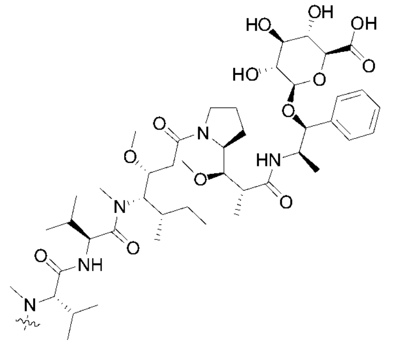, 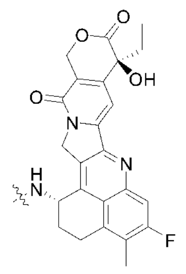, 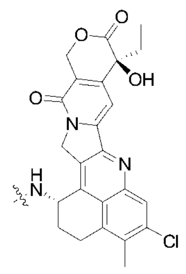, 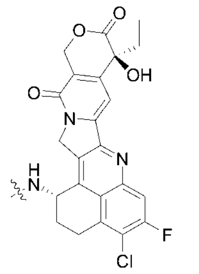,

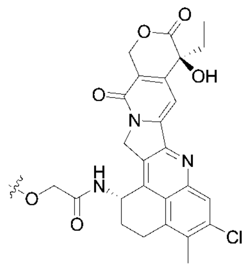, 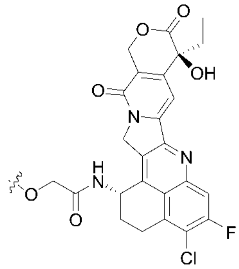, 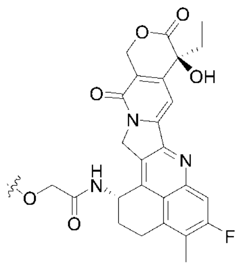, 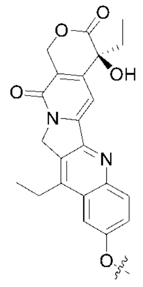, 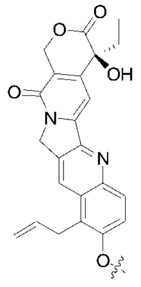,

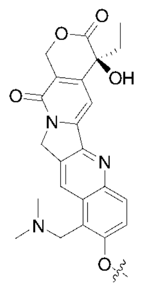, 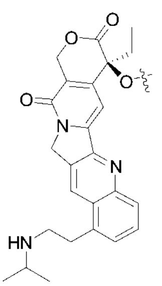, 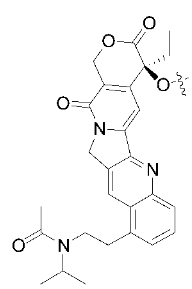, 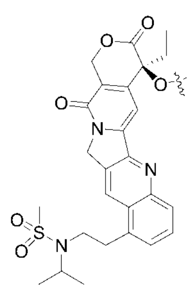, 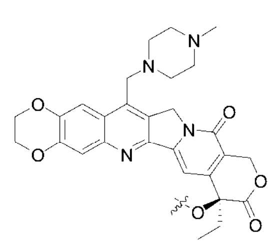,

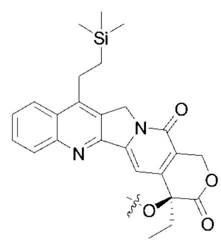, 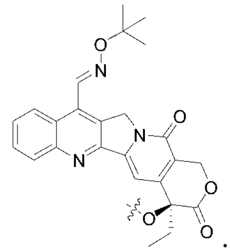.

In some embodiments, the compound of Formula II is selected from:

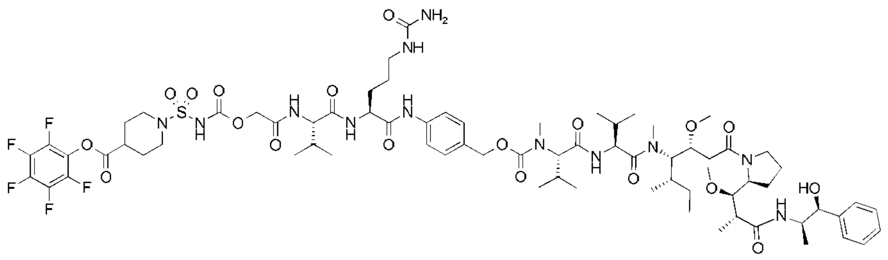

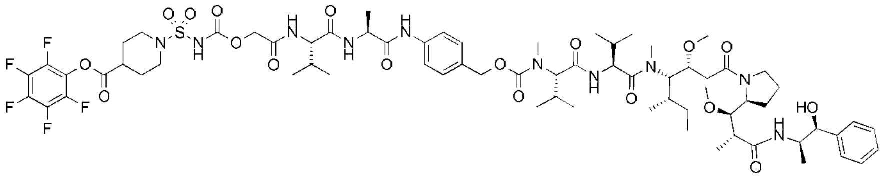

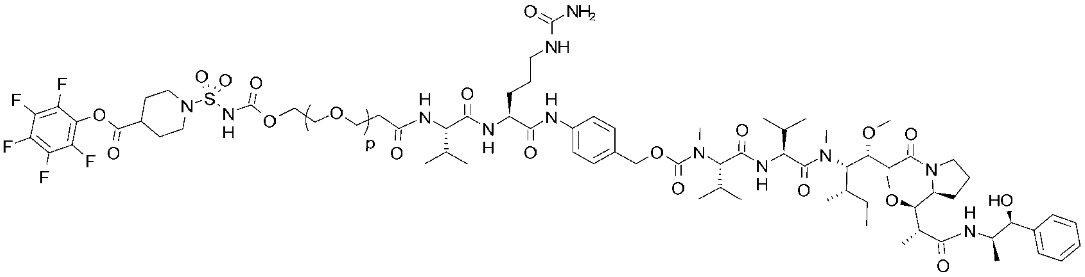

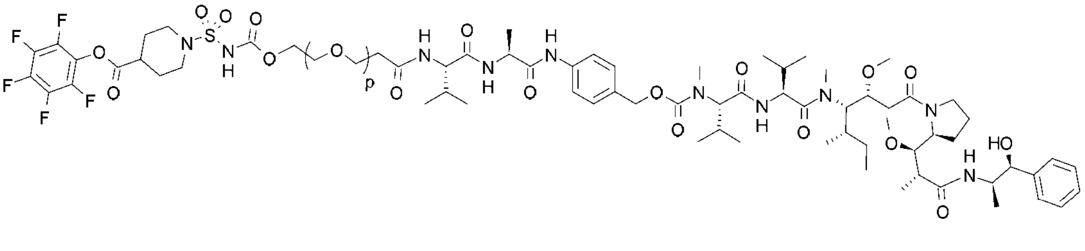

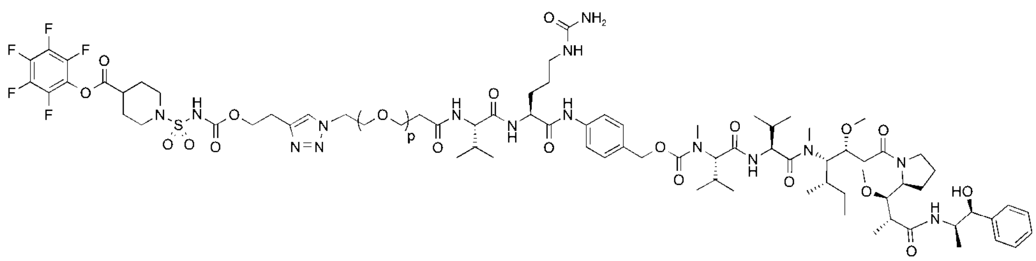

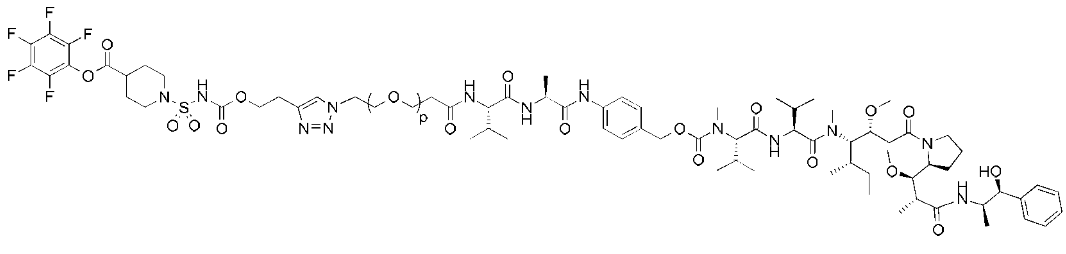

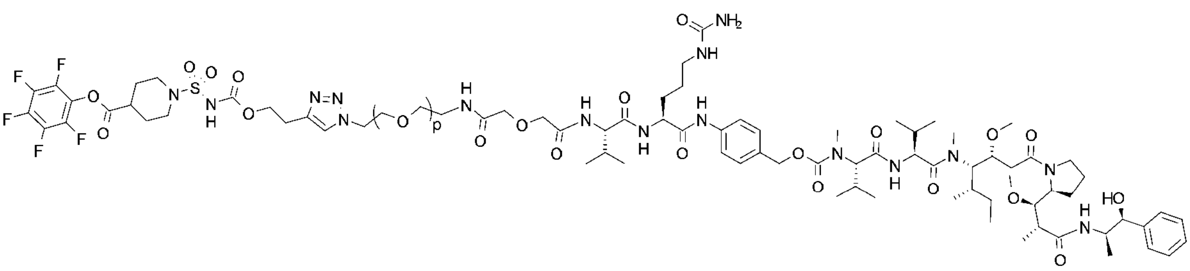

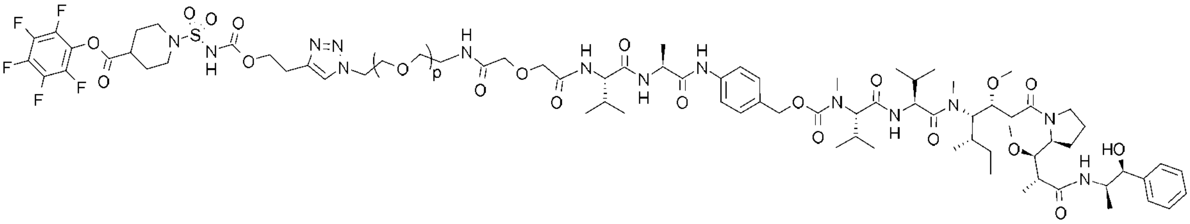

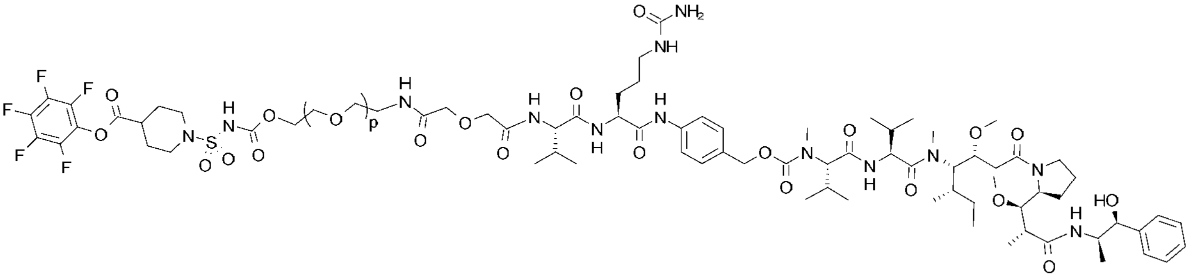

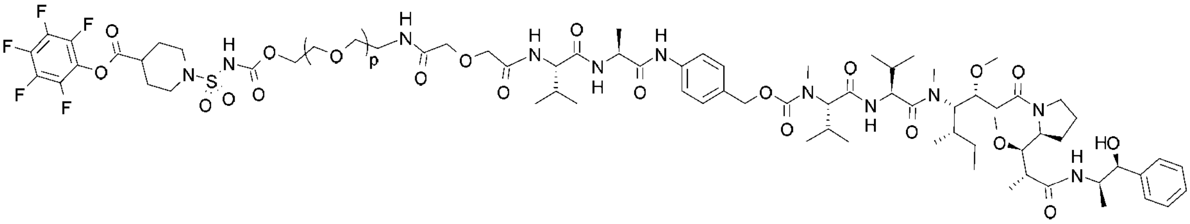

p is 0 or an integer of 1 to 20.

In some embodiments, the compound of Formula II is:

DL-1:

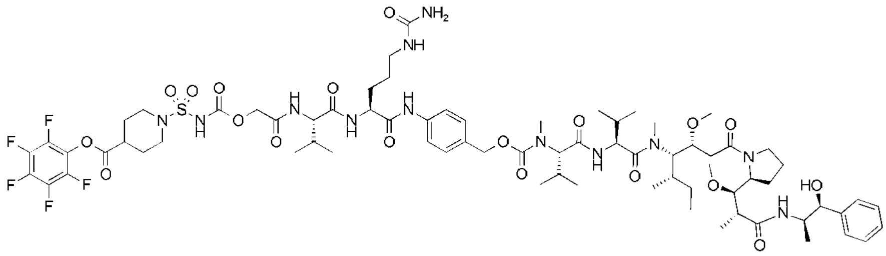

or

DL-2:

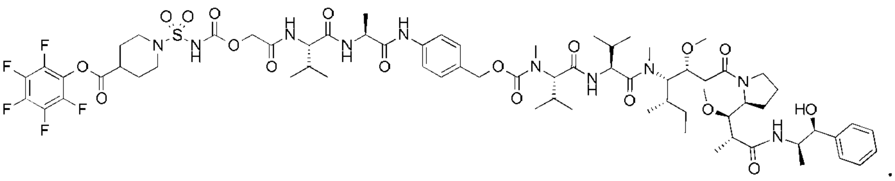

In another aspect, the present invention provides a bioactive conjugate, which has a structure represented by Formula III:

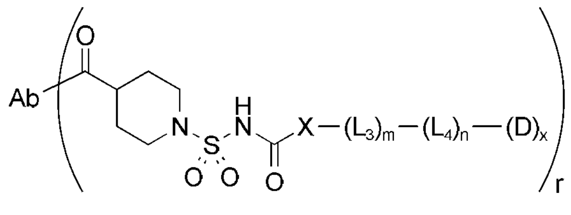

Formula III

wherein:

Ab is a targeting moiety (e.g., a small molecule ligand, a protein (e.g., an antibody), a polypeptide, a non-protein agent (e.g., a sugar, RNA, or DNA)); r is selected from the group consisting of 1 to 10;

X, $L_3$, $L_4$, D, m, x, and n are as defined in any of the above compound of Formula II.

In some embodiments, the target of Ab is selected from the group consisting of epidermal growth factor, Trop-2, CD37, HER2, CD70, EGFRvIII, Mesothelin, Folate receoptor1, Mucin 1, CD138, CD20, CD19, CD30, SLTRK6, Nectin 4, Tissue factor, Mucin16, Endothelinreceoptor, STEAP1, SLC39A6, Guanylylcyclase C, PSMA, CCD79b, CD22, Sodium phosphate cotransporter 2B, GPNMB, Trophoblast glycoprotein, AGS-16, EGFR, CD33, CD66e, CD74, CD56, PD-L1, TACSTD2, DR5, E16, 0772P, MPF, Napi3b, Sema 5b, PSCA hlg, ETBR, MSG783, STEAP2, TrpM4, CRIPTO, CD21, CD79b, FcRH2, NCA, MDP, IL20Rα, Brevican, EphB2R, ASLG659, PSCA, GEDA, BAFF-R, CD79a, CXCR5, HLA-DOB, P2X5, CD72, LY64, FcRH1, IRTA2, TENB2, integrins α5β6 and α4β7, FGF2, FGFR2, Her3, CA6, DLL3, DLL4, P-cadherin, EpCAM, pCAD, CD223, LYPD3, LY6E, EFNA4, ROR1, SLITRK6, 5T4, ENPP3, Claudin18.2, BMPR1B, Tyro7, c-Met, ApoE, CD11c, CD40, CD45(PTPRC), CD49D(ITGA4), CD80, CSFIR, CTSD, GZMB, Ly86, MS4A7, PIK3AP1, PIK3CD, CCR5, IFNG, IL10RA1, IL-6, ACTA2, COL7A1, LOX, LRRC15, MCPT8 , MMP10, NOG, SERPINE1, STAT1, TGFBR1, CTSS, PGF, VEGFA, C1QA, C1QB, ANGPTL4, EGLN, EGLN3, BNIP3, AIF1, CCL5, CXCL10, CXCL11, IFI6, PLOD2, KISS1R, STC2, DDIT4, PFKFB3, PGK1, PDK1, AKR1C1, AKR1C2, CADM1, CDH11, COL6A3, CTGF, HMOX1, KRT33A, LUM, WNT5A, IGFBP3, MMP14, CDCP1, PDGFRA, TCF4, TGF, TGFB1, TGFB2, CD1 lb, ADGRE1, EMR2, TNFRSF21, UPK1B, TNFSF9, MMP16, MFI2, IGF-1R, RNF43, NaPi2b, BCMA, B7H3 and TENB2.

In some embodiments, Ab is an antibody, and the bioactive conjugate is an antibody-drug conjugate (ADC).

In some embodiments, Ab is an antibody, and the antibody is conjugated through the terminal amino group of lysine to form a bioactive conjugate.

In some embodiments, Ab is an antibody, and the light chain of the antibody is connected to the remaining portion of the conjugate represented by Formula III via an amide bond formed between the terminal amino group of lysine and the remaining portion of the conjugate represented by Formula III.

In some embodiments, Ab is trastuzumab or pertuzumab.

In some embodiments, Ab is trastuzumab.

In some embodiments, the bioactive conjugate is selected from:

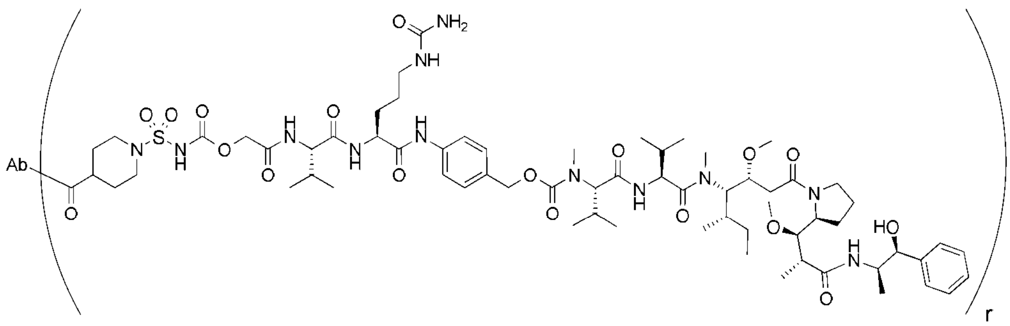

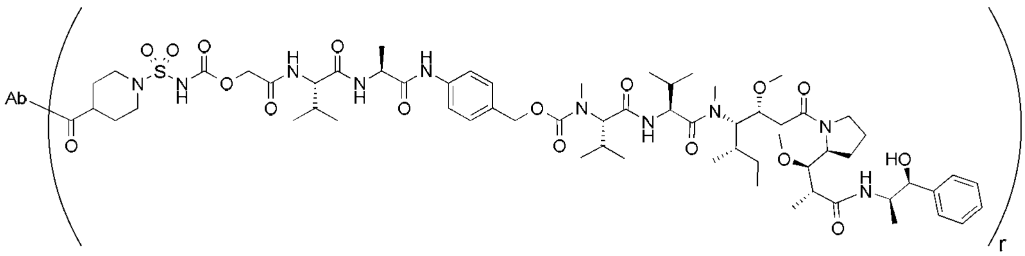

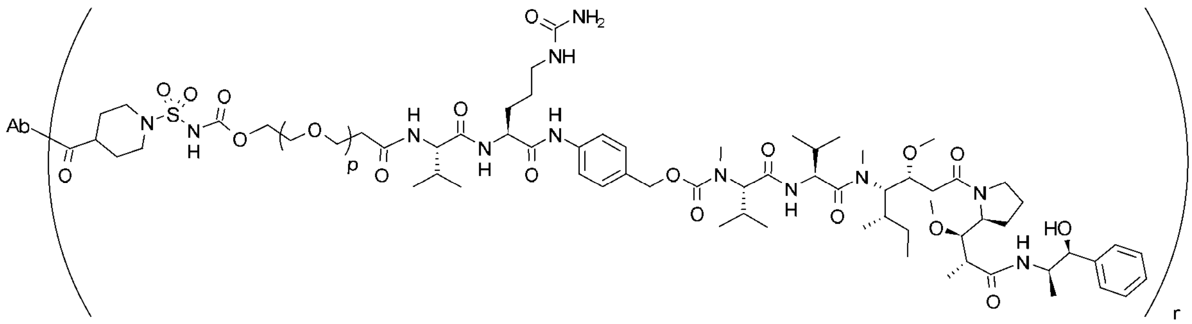

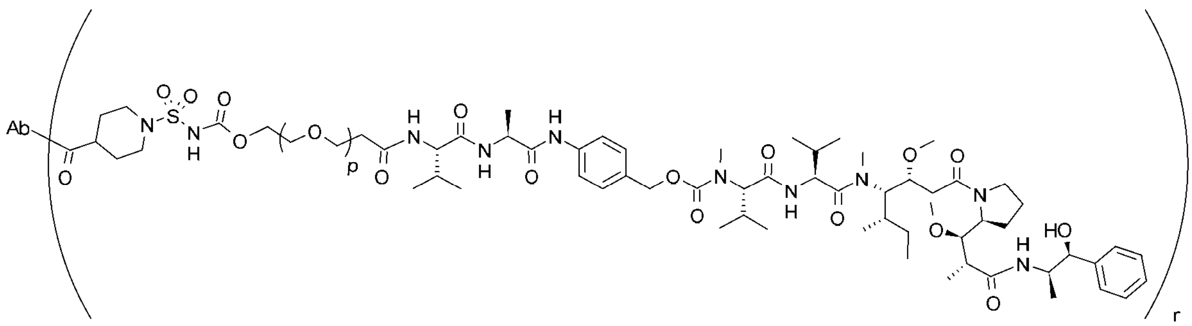

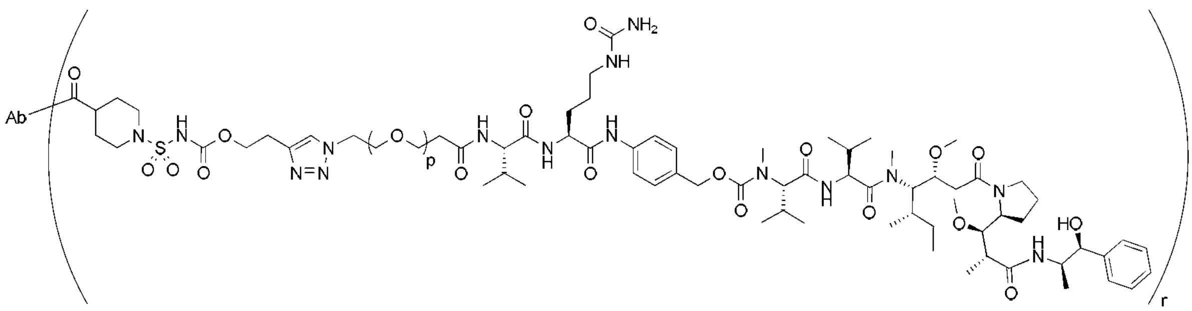

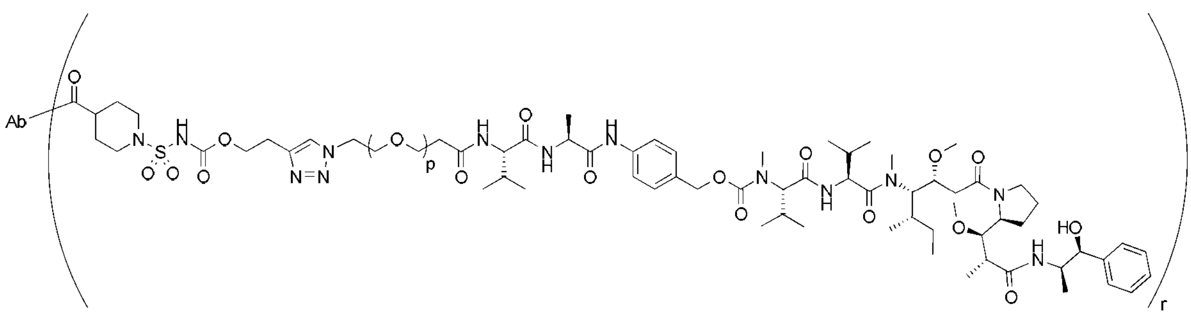

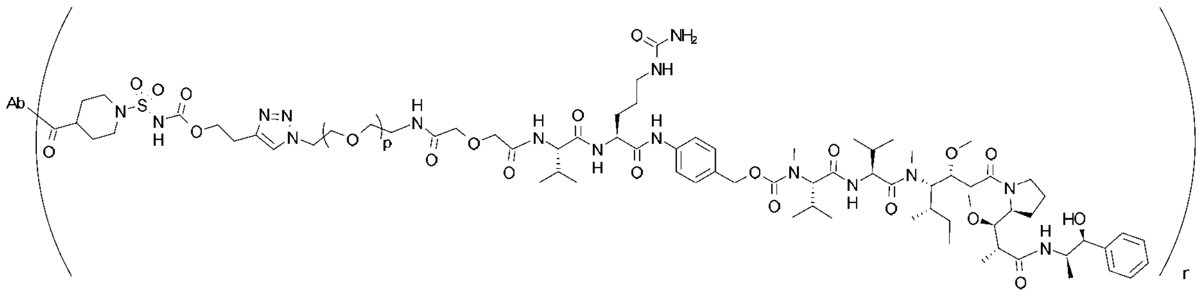

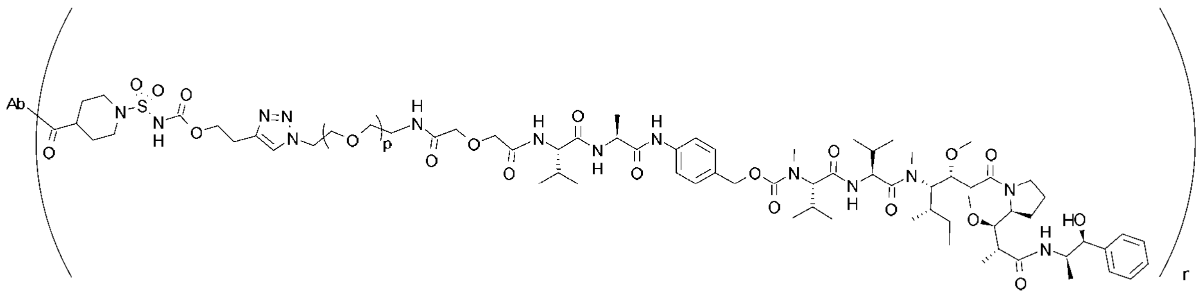

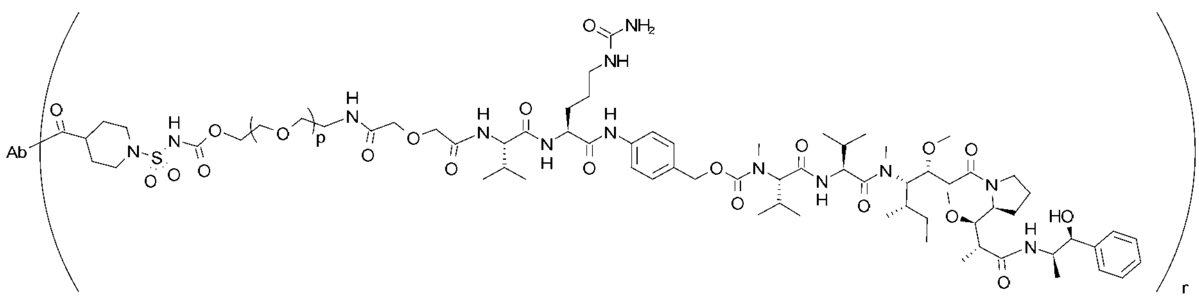

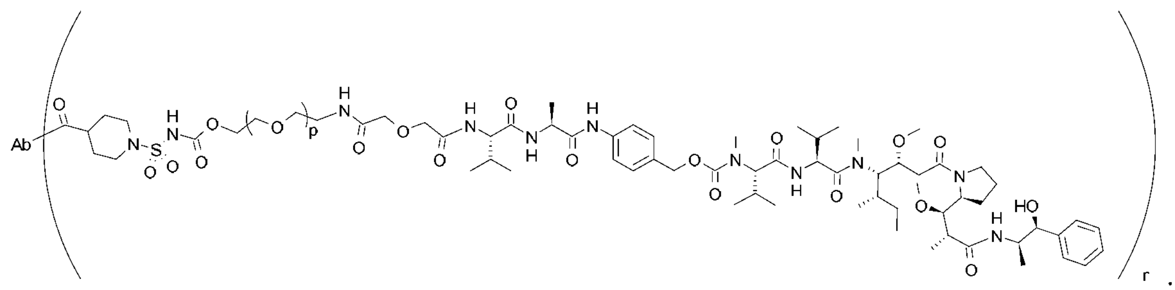

In some embodiments, the antibody-drug conjugate is:

Trastuzumab-DL-1:

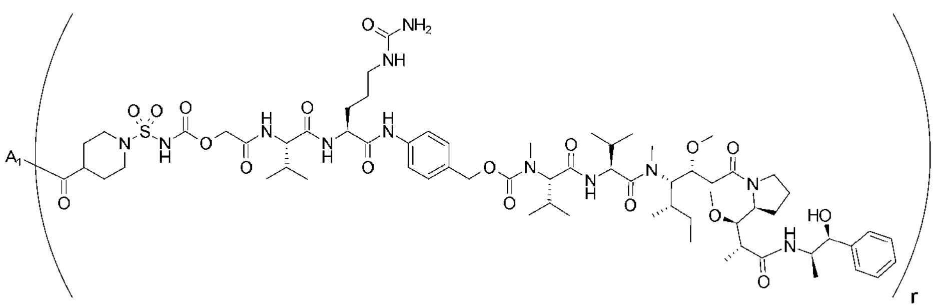

or

Trastuzumab-DL-2:

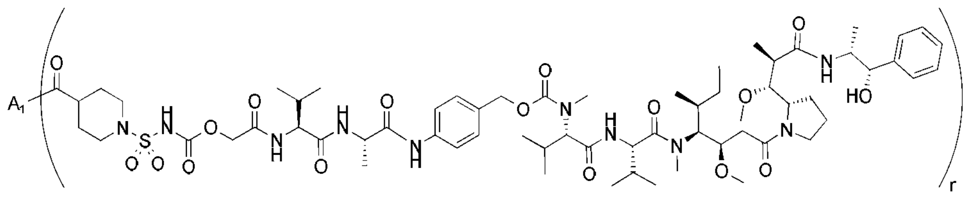

wherein, A1 is trastuzumab, r is 1 to 10, preferably 1 to 3, and more preferably about 2; preferably, trastuzumab is connected to the other portion of the bioactive conjugate through the lysine residue on trastuzumab.

Composition

In another aspect, the present application provides a composition comprising the bioactive conjugate (e.g., antibody-drug conjugate (ADC)) as described herein. Such a composition may comprise a plurality of conjugates as described herein, wherein each conjugate comprises a drug-linker as described herein, r is independently 1, 2, 3, 4, 5, 6, 7, 8, 9 or 10; preferably, r is independently 1, 2, 3, 4 or 5. In other words, each Ab (e.g., antibody) molecule in the composition may be conjugated to 1, 2, 3, 4, 5, 6, 7, 8, 9 or 10 (preferably 1, 2, 3, 4 or 5) drug-linkers. Consequently, the composition is characterized by a "drug-antibody" ratio (DAR) in the range of about 1 to about 10, preferably in the range of about 1 to about 5. Methods for determining DAR are well known to those skilled in the art, including techniques using reverse-phase chromatography or HPLC-MS.

For example, in any embodiment, the ADC composition described herein has a DAR of about 1 to about 10 or any subrange within this interval, for example: about 1 to 2, about 1 to 3, about 1 to 4, about 1 to 5, about 1 to 6, about 1 to 7, about 1 to 8, about 1 to 9, about 1 to 10, about 2 to 3, about 2 to 4, about 2 to 5, about 2 to 6, about 2 to 7, about 2 to 8, about 2 to 9, about 2 to 10.

In certain embodiments, the ADC compositions described herein has a DAR of about 1 to 10, for example, about 1.0 to 5.5, about 1.0 to 5.0, about 1.0 to 4.5, about 1.0 to 4.0, about 1.0 to 3.5, about 1.0 to 3.0, about 1.0 to 2.5, about 1.0 to 2.0, about 1.5 to 5.5, about 1.5 to 5.0, about 1.5 to 4.5, about 1.5 to 4.0, about 1.5 to 3.5, about 1.5 to 3.0, about 1.5 to 2.5, about 1.5 to 2.0, about 2.0 to 5.5, about 2.0 to 5.0, about 2.0 to 4.5, about 2.0 to 4.0, about 2.0 to 3.5, about 2.0 to 3.0, about 2.0 to 2.5, for example, 2.0, 2.1, 2.2, 2.3, 2.4 or 2.5.

Definitions

Unless otherwise defined below, the meanings of all technical and scientific terms used herein are intended to be the same as those generally understood by those skilled in the art. Reference to the technology used herein is intended to refer to the technology generally understood in the art, including those changes in technology or replacement of equivalent technology that are obvious to those skilled in the art. Although it is believed that the following terms are well understood by those skilled in the art, the following definitions are still set forth to better explain the present invention.

As used herein, the term "about" refers to a range of 1% to 10%, such as 1% to 5%, such as 1% to 2.5%, such as 1%, 1.5%, 2% or 2.5%, around the present value.

As used herein, the term "alkylene" refers to a saturated divalent hydrocarbyl, preferably a saturated divalent hydrocarbyl having 1, 2, 3, 4, 5 or 6 carbon atoms, such as $C_{1-6}$ alkylene, methylene, ethylene, propylene or butylene.

As used herein, the term "alkyl" is defined as a linear or branched saturated aliphatic hydrocarbyl. As used herein, the term "$C_{1-6}$ alkyl" refers to a linear or branched aliphatic hydrocarbyl having 1 to 6 carbon atoms (e.g., methyl, ethyl, n-propyl, isopropyl, n-butyl, isobutyl, sec-butyl, tert-butyl, n-pentyl, isopentyl, neopentyl or n-hexyl), which is optionally substituted with one or more (e.g., 1 to 3) suitable substituents (e.g., halogen) (in which case the group is referred to as a "haloalkyl", e.g., $C_{1-6}$ haloalkyl) (e.g., $CH_2F$, $CHF_2$, $CF_3$, $CCL_3$, $C_2F_5$, $C_2Cl_5$, $CH_2CF_3$, $CH_2Cl$ or $-CH_2CH_2CF_3$, etc.). The term "$C_{1-4}$ alkyl" refers to a linear or branched aliphatic hydrocarbyl having 1 to 4 carbon atoms (i.e., methyl, ethyl, n-propyl, isopropyl, n-butyl, isobutyl, sec-butyl or tert-butyl).

As used herein, the term "alkoxy" is defined as -O-alkyl, and the alkyl is as defined above. For example, as used herein, the term "$C_{1-6}$ alkoxy" refers to -O-$C_{1-6}$ alkyl.

As used herein, the term "alkoxyalkyl" is defined as an alkyl substituted with alkoxy, and the alkyl is as defined above. For example, as used herein, the term "$C_{2-6}$ alkoxyalkyl" refers to an alkyl substituted with alkoxy having 2 to 6 carbon atoms.

As used herein, the term "alkenylene" refers to a divalent hydrocarbyl comprising at least one carbon-carbon double bond, and preferably having 2, 3, 4, 5 or 6 carbon atoms, such as $C_{2-6}$ alkenylene, vinylene, propenylene or

butenylene.

As used herein, the term "alkenyl" refers to an aliphatic hydrocarbyl comprising at least one carbon-carbon double bond, and preferably having 2, 3, 4, 5 or 6 carbon atoms, such as $C_{2-6}$ alkenyl, vinyl, propenyl or butenyl.

As used herein, the term "alkynylene" refers to a divalent hydrocarbyl comprising at least one carbon-carbon triple bond, and preferably having 2, 3, 4, 5 or 6 carbon atoms, such as ethynylene, propynylene or butynylene.

As used herein, the term "alkynyl" refers to an aliphatic hydrocarbyl comprising at least one carbon-carbon triple bond, and preferably having 2, 3, 4, 5 or 6 carbon atoms, such as $C_{2-6}$ alkynyl, ethynyl, propynyl or butynyl.

As used herein, the term "acyl" refers to -C(=O)-.

As used herein, the term "alkylacyl" refers to -C(=O)-alkyl, and the alkyl is as defined above. For example, as used herein, the term "$C_{1-6}$ alkylacyl" refers to -C(=O)-$C_{1-6}$ alkyl.

As used herein, the term "sulfo" refers to

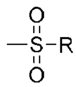 ,

wherein R is selected from the group consisting of H or alkyl, and the alkyl is as defined above.

As used herein, the term "sulfonic acid" refers to

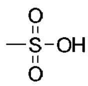 .

As used herein, the term "amino" refers to -$NH_2$, -NH- or

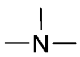 .

As used herein, the term "alkylamino" refers to -$NR_aR_b$, $R_a$ and $R_b$ are each independently selected from the group consisting of H or alkyl as defined above, and $R_a$ and $R_b$ are not H at the same time. For example, "$C_{1-6}$ alkylamino" refers to -$NR_aR_b$, $R_a$ and $R_b$ are each independently selected from the group consisting of H or $C_{1-6}$ alkyl.

As used herein, the term "carboxylic acid group" refers to -COOH.

As used herein, the term "phosphoric acid group" refers to

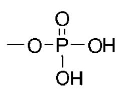 .

As used herein, the term "quaternary ammonium salt" refers to -$N^+R_cR_dR_e$, wherein $R_c$, $R_d$ and $R_e$ are each independently selected from the group consisting of alkyl, cycloalkyl, heterocyclyl, aryl or heteroaryl, or any two of $R_c$, $R_d$ and $R_e$ form a heterocyclyl together with the N atom to which they are connected, or $R_c$, $R_d$ and $R_e$ form a heteroaryl together with the N atom to which they are connected.

As used herein, the term "amide group" refers to -CO-$NR_fR_g$, wherein $R_f$ and $R_g$ are each independently selected from the group consisting of H or alkyl as defined above.

As used herein, the term "azido" refers to

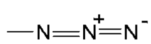 .

.

As used herein, the term "oxime group" refers to

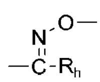 or 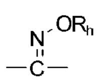 ,

wherein $R_h$ is selected from the group consisting of H, alkyl, cycloalkyl, heterocyclic, aryl or heteroaryl.

As used herein, the term "glycosyl" refers to a group that is obtained after removing a hemiacetal hydroxyl group

of a cyclic monosaccharide or oligosaccharide, and comprises furanosyl and pyranosyl, such as glucosyl, galactosyl, mannosyl, etc.

As used herein, the term "benzyl" refers to

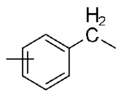

.

As used herein, the term "benzyloxy" refers to

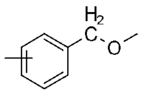

.

As used herein, the term "benzyloxycarbonyl" refers to

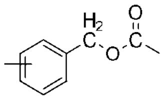

.

As used herein, the term "aminobenzyloxycarbonyl" refers to

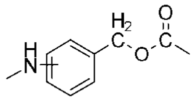

.

As used herein, the term "acylbenzyloxycarbonyl" refers to

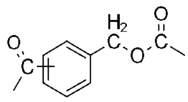

.

As used herein, the term "heterocyclyl" or "heterocyclic ring" refers to a saturated or partially unsaturated (i.e., having one or more double bonds and/or triple bonds in the ring) cyclic group in which at least one ring atom is a heteroatom selected from the group consisting of N, O and S, and the remaining ring atoms are C. For example, "4- to 16-membered heterocyclic ring (group)" refers to a saturated or partially unsaturated cyclic structure having 4 to 16 (e.g., 5 to 12, 2, 3, 4, 5, 6, 7, 8 or 9) ring atoms, in which at least one ring atom (e.g., 1, 2, 3 or 4) is a heteroatom selected from the group consisting of N, O and S. The term "nitrogen-containing heterocyclic ring (group)" refers to a heterocyclic ring (group) in which at least one of the ring atoms is N. Examples of heterocyclyl include, but are not limited to, oxiranyl, aziridinyl, azetidinyl, oxetanyl, tetrahydrofuranyl, dioxolinyl, pyrrolidinyl, pyrrolidonyl, imidazolidinyl, pyrazolidinyl, pyrrolinyl, tetrahydropyranyl, piperidinyl, morpholinyl, dithianyl, thiomorpholinyl, piperazinyl or trithianyl. The heterocyclyl may be optionally substituted with one or more (e.g., 1, 2, 3 or 4) suitable substituents, and may optionally form a fused ring structure with one or more aromatic rings or heteroaromatic rings.

As used herein, the term "aromatic ring" or "aryl" refers to a monocyclic or polycyclic aromatic ring system having, for example, 6, 8, 9, 10, 11, 12, 13 or 14 ring-forming carbon atoms, for example, a benzene ring or a naphthalene ring.

As used herein, the term "aromatic heterocyclic ring" or "heteroaryl" refers to a monocyclic or polycyclic aromatic ring system that has, for example, 5, 6, 8, 9, 10, 11, 12, 13 or 14 ring atoms, in particular 1 or 2 or 3 or 4 or 5 or 6 or 9 or 10 carbon atoms, and contains at least one heteroatom which may be identical or different (the heteroatom is, for example, oxygen, nitrogen or sulfur), and, in each case, may additionally be benzofused. For example, it is a 5- to 16-membered heteroaryl, 5- to 12-membered heteroaryl.

As used herein, the term "halogen" comprises F, Cl, Br and I.

As used herein, the term "amino acid residue" refers to an amino acid unit in a polypeptide, i.e., a remaining part formed when the amino acid participates in the formation of a peptide bond, resulting in the loss of a water molecule.

The term "substitution" means that one or more (e.g. one, two, three or four) hydrogen atoms on the designated atom are replaced by a selection from the indicated group, provided that the normal valence of the designated atom in the

present case is not exceeded and the substitution forms a stable compound. Combinations of substituents and/or variables are permitted only if such combinations result in stable compounds.

If a substituent is described as being "optionally substituted with," the substituent may be (1) unsubstituted or (2) substituted. If a carbon of a substituent is described as being optionally substituted with one or more of the substituents listed, one or more hydrogen atoms on the carbon (to the extent of any hydrogen atoms present) may be substituted individually and/or together with independently selected optional substituents. If a nitrogen of a substituent is described as being optionally substituted with one or more of the substituents listed, one or more hydrogen atoms on the nitrogen (to the extent of any hydrogen atoms present) may be substituted individually with independently selected optional substituents.

If a substituent is described as being "independently selected" from a group of groups, each substituent is selected independently of the other. Thus, each substituent may be the same as or different from another (other) substituent.

As used herein, the term "one or more" means one or more than one, such as 2, 3, 4, 5, or 10, as appropriate.

Unless otherwise indicated, as used herein, the site of attachment of a substituent may be from any convenient site of the substituent.

When a bond of a substituent is shown as passing through a bond connecting two atoms in a ring, then such a substituent may be bonded to any ring-forming atom in the substitutable ring.

The present invention also comprises all pharmaceutically acceptable isotopically labeled compounds that are identical to the compound of the present invention except that one or more atoms are replaced by an atom having the same atomic number but an atomic mass or mass number different from the atomic mass or mass number prevalent in nature. Examples of isotopes suitable for inclusion in the compounds of the present invention include, but are not limited to, isotopes of hydrogen (e.g., deuterium ($^{2}H$), tritium ($^{3}H$)); isotopes of carbon (e.g., $^{11}C$, $^{13}C$, and $^{14}C$); isotopes of chlorine (e.g., $^{36}Cl$); isotopes of fluorine (e.g., $^{18}F$); isotopes of iodine (e.g., $^{123}I$ and $^{125}I$); isotopes of nitrogen (e.g., $^{13}N$ and $^{15}N$); isotopes of oxygen (e.g., $^{15}O$, $^{17}O$, and $^{18}O$); isotopes of phosphorus (e.g., $^{32}P$); and isotopes of sulfur (e.g., $^{35}S$).

The term "stereoisomer" refers to an isomer formed due to at least one asymmetric center. For a compound having one or more (e.g., one, two, three, or four) asymmetric centers, racemic mixtures, single enantiomers, diastereomeric mixtures, and individual diastereomers may be produced. Specific individual molecules may also exist as geometric isomers (cis/trans). Similarly, the compound of the present invention may exist as mixtures of two or more structurally different forms in rapid equilibrium (commonly referred to as tautomers). Representative examples of tautomers include keto-enol tautomers, phenol-ketone tautomers, nitroso-oxime tautomers, imine-enamine tautomers, and the like. It is to be understood that the scope of the present application encompasses all such isomers or mixtures thereof in any proportion (e.g., 60%, 65%, 70%, 75%, 80%, 85%, 90%, 95%, 96%, 97%, 98%, 99%).

Solid lines (———), solid wedges ( ), or dashed wedges ( ) may be used herein to depict carbon-carbon bonds of the compounds of the present invention. The use of solid lines to depict bonds to asymmetric carbon atoms is intended to indicate that all possible stereoisomers at the carbon atom (e.g., specific enantiomers, racemic mixtures, etc.) are included. The use of solid or dashed wedges to depict bonds to asymmetric carbon atoms is intended to indicate the presence of the indicated stereoisomers. When present in a racemic mixture, solid and dashed wedges are used to define relative stereochemistry, not absolute stereochemistry. Unless otherwise indicated, the compounds of the present invention are intended to exist in the form of stereoisomers (which includes cis and trans isomers, optical isomers (e.g., R and S enantiomers), diastereomers, geometric isomers, rotational isomers, conformational isomers, atropisomers, and mixtures thereof). The compound of the present invention may exhibit more than one type of isomerism and consist of mixtures thereof (e.g., racemic mixtures and diastereomeric pairs).

The present invention encompasses all possible crystalline forms or polymorphs of the compounds of the present invention, which may be single polymorphs or mixtures of more than one polymorph in any proportion.

It should also be understood that certain compounds of the present invention may exist in free form for treatment or, where appropriate, in the form of pharmaceutically acceptable derivatives thereof. In the present invention, the pharmaceutically acceptable derivatives include, but are not limited to, pharmaceutically acceptable salts, esters, solvates, N-oxides, metabolites or prodrugs, which can directly or indirectly provide the compounds of the present invention or metabolites or residues thereof after being administered to a patient in need thereof. Therefore, when referring to "the compound of the present invention" herein, it is also intended to cover the above-mentioned various derivative forms of the compound.

Pharmaceutically acceptable salts of the compounds of the present invention include acid addition salts and base addition salts thereof.

Suitable acid addition salts are formed from acids that form pharmaceutically acceptable salts. Examples include aspartate, benzoate, bicarbonate/carbonate, glucoheptonate, gluconate, nitrate, orotate, palmitate and other similar salts.

Suitable base addition salts are formed from bases that form pharmaceutically acceptable salts. Examples include aluminum salt, arginine salt, choline salt, magnesium salt and other similar salt.

For a review of suitable salts, see Stahl and Wermuth, "Handbook of Pharmaceutical Salts: Properties, Selection,

and Use" (Wiley-VCH, 2002). Methods for preparing pharmaceutically acceptable salts of the compounds of the present invention are known to those skilled in the art.

As used herein, the term "ester" refers to an ester derived from the compounds of the general formulae of the present application, including physiologically hydrolyzable esters (which can be hydrolyzed under physiological conditions to release the compounds of the present invention in free acid or alcohol form). The compounds of the present invention themselves may also be esters.

The compounds of the present invention may exist in the form of solvates (preferably hydrates), wherein the compounds of the present invention contain a polar solvent, in particular water, for example, as a structural element of the crystal lattice of the compound. The amount of polar solvent, in particular water, may be present in a stoichiometric or non-stoichiometric ratio.

Those skilled in the art will appreciate that not all nitrogen-containing heterocycles are capable of forming N-oxides, as nitrogen requires an available lone pair of electrons to oxidize to form an oxide; those skilled in the art will recognize nitrogen-containing heterocycles that are capable of forming N-oxides. Those skilled in the art will also recognize that tertiary amines are capable of forming N-oxides. Synthetic methods for preparing N-oxides of heterocycles and tertiary amines are well known to those skilled in the art, and include oxidation of heterocycles and tertiary amines with peroxyacids such as peracetic acid and m-chloroperoxybenzoic acid (MCPBA), hydrogen peroxide, alkyl hydroperoxides such as tert-butyl hydroperoxide, sodium perborate, and dioxiranes such as dimethyldioxirane. These methods for preparing N-oxides have been extensively described and reviewed in the literature, see, for example: T. L. Gilchrist, Comprehensive Organic Synthesis, vol. 7, pp 748-750; A. R. Katritzky and A. J. Boulton, Eds., Academic Press; and G. W. H. Cheeseman and E. S. G. Werstiuk, Advances in Heterocyclic Chemistry, vol. 22, pp 390-392, A. R. Katritzky and A. J. Boulton, Eds., Academic Press.

Metabolites of the compounds of the present invention are also included within the scope of the present invention, which are substances formed in vivo upon administration of the compounds of the present invention. Such products may be produced, for example, by oxidation, reduction, hydrolysis, amidation, deamidation, esterification, enzymatic hydrolysis, etc. of the compounds being administered. Thus, the present invention comprises metabolites of the compounds of the present invention, including compounds made by contacting the compounds of the present invention with a mammal for a period of time sufficient to produce their metabolites.

The present invention further includes within its scope prodrugs of the compounds of the present invention, which are certain derivatives of the compounds of the present invention that may themselves have less pharmacological activity or no pharmacological activity and which, when administered to or on the body, may be converted, for example, by hydrolytic cleavage, into the compounds of the present invention having the desired activity. Typically, such prodrugs will be functional group derivatives of the compounds that are easily converted in vivo into the desired therapeutically active compounds. Additional information on the use of prodrugs may be found in "Pro-drugs as Novel Delivery Systems", Vol. 14, ACS Symposium Series (T. Higuchi and V. Stella). The prodrugs of the present invention can be prepared, for example, by replacing appropriate functional groups present in the compounds of the present invention with certain moieties known to those skilled in the art as "pro-moieties" (e.g., those described in "Design of Prodrugs", H. Bundgaard (Elsevier, 1985)).

The present invention also covers the compounds of the present invention that contain protecting groups. In any process for preparing the compounds of the present invention, it may be necessary and/or desirable to protect sensitive or reactive groups on any of the molecules involved, thereby forming chemically protected forms of the compounds of the present invention. This can be achieved by conventional protecting groups, for example, as described in T.W. Greene & P.G.M. Wuts, Protective Groups in Organic Synthesis, John Wiley & Sons, 1991, and these references are incorporated herein by reference. The protecting groups may be removed at an appropriate subsequent stage using methods known in the art.

In addition, the compounds of the present invention may be prepared in a variety of ways known to those skilled in the art of organic synthesis. The compounds of the present invention may be synthesized using the methods described below as well as synthetic methods known in the field of synthetic organic chemistry or variations thereof known to those skilled in the art. Preferred methods include (but are not limited to) those described above. The reaction may be carried out in a solvent or solvent mixture appropriate for the reagents and materials used and suitable for the transformation to be achieved. Those skilled in the art of organic synthesis will understand that the functional groups present on the molecules should be consistent with the proposed transformation. This will sometimes require the following judgment: modifying the order of synthetic steps or selecting another specific method route relative to one method route to obtain the desired compounds of the present invention.

It should also be recognized that another major consideration in the design of any synthetic route in the art is the correct choice of protecting groups used to protect the reactive functional groups present in the compounds described in the present invention. An authoritative description of many alternatives to the trained person is descrbed by Greene et al. (Protective Groups in Organic Synthesis, 4th Edition, Wiley-Interscience (2006)).

Unless otherwise specified, the substituents of the compounds in the above routes are as defined in the present invention. Those skilled in the art will understand that one or more steps in the above routes can be omitted according to the

desired product structure. Those skilled in the art can also adjust the order of the reaction steps appropriately as needed.

Pharmaceutical composition

The present invention also provides a pharmaceutical composition, which comprises the bioactive conjugate as described in the present invention, and one or more pharmaceutically acceptable carriers.

The pharmaceutical excipients described herein refer to excipients and additives used in the production of drugs and the preparation of prescriptions, and refer to substances that have been reasonably evaluated in terms of safety and are included in pharmaceutical preparations in addition to the active ingredients.

The pharmaceutical composition can be administered in any form as long as it prevents, alleviates, arrests or cures the symptoms of human or animal patients. For example, various suitable dosage forms can be prepared according to the route of administration.

The present application also provides a kit product, which contains the bioactive conjugate or pharmaceutical composition as described in the present invention, and optional medication instructions.

Therapeutic method and use

In another aspect, the present application provides the use of the bioactive conjugate in the manufacture of a medicament for preventing or treating a tumor disease.

In another aspect, the present application provides the bioactive conjugate, for use in preventing or treating a tumor disease.

In another aspect, the present application provides a method for preventing or treating a tumor disease, which comprises administering an effective amount of the bioactive conjugate or a pharmaceutical composition comprising the bioactive conjugate, to a subject in need thereof.

In one embodiment of the present invention, the tumor disease is a solid tumor or a hematological malignancy; for example, which is selected from the group consisting of colon cancer, gastric cancer, breast cancer, lung cancer (e.g., non-small cell lung cancer, specifically lung adenocarcinoma), lymphoma.

As used herein, the term "effective amount" refers to an amount of the conjugate that, after administration, alleviates one or more symptoms of the treated condition to a certain extent.

Unless otherwise specified, as used herein, the term "treatment" means reversing, alleviating the progression of the applied disease or condition or one or more symptoms of such disease or condition.

As used herein, "individual" or "subject" comprises human or non-human animals. Exemplary human individuals include human individuals (referred to as patients) suffering from a disease (e.g., a disease described herein) or normal individuals. In the present invention, "non-human animals" include all vertebrates, such as non-mammals (e.g., birds, amphibians, reptiles) and mammals, such as non-human primates, livestock and/or domesticated animals (e.g., sheep, dogs, cats, cows, pigs, etc.).

## Brief Description of the Drawings

The drawings described herein are used to provide a further understanding of the present invention and constitute a part of the present application. The schematic examples of the present invention and their descriptions are used to explain the present invention and do not constitute an improper limitation of the present invention. In the drawings:

Figure 1 shows the efficacy results of anti-human HER2 antibody-drug conjugate (ADC) on the JIMT-1 cell subcutaneous tumor mouse model.

Figure 2 shows the changes in the body weight of mice in each group of the human breast cancer cell JIMT-1 CDX model.

Figure 3 shows the efficacy results of anti-human HER2 antibody-drug conjugate (ADC) on the NCI-N87 cell subcutaneous tumor mouse model.

Figure 4 shows the changes in the body weight of mice in each group of the human gastric cancer cell NCI-N87 CDX model.

**Specific Models for Carrying Out the present invention**

In the following, examples of the present invention will be clearly and completely described in conjugation with the drawings. Apparently, the described examples are only part of the examples of the present invention, not all of them. The following description of at least one exemplary example is actually only illustrative and is by no means a limitation on the present invention and its application or use. Based on the examples of the present invention, all other examples obtained by ordinary technicians in this field without creative work are within the scope of the present invention.

The following examples and test examples are provided to further illustrate the present invention in detail, but they do not limit the scope of the present invention, and can be changed without departing from the scope of the present invention.

The abbreviations used herein have the following meanings:

| Abbrevi ation | Full Name | Abbrevi ation | Full Name |
|---|---|---|---|
| HOBt | 1-Hydroxybenzotriazole | DIPEA | N,N-Diisopropylethylamine |
| DIC | N,N-Diisopropylcarbodiimide | MMAE | Monomethyl auristatin E |
| Pd/C | Palladium on Carbon | $LiOH·H_2O$ | Lithium Hydroxide Monohydrate |
| EDCI | 1-(3-Dimethylaminopropyl)-3-et hylcarbodiimide Hydrochloride | HATU | O-(7-Azabenzotriazol-1-yl)-N,N,N',N'-tetramethyluronium Hexafluorophosphate |
| CuBr | Copper(I) Bromide | LC-MS | Liquid Chromatography-Mass Spectrometry |
| DCM | Dichloromethane | MeOH | Methanol |
| DMSO | Dimethyl Sulfoxide | DMF | N,N-Dimethylformamide |
| DAR | Drug-to-Antibody Ratio | Trastuzu mab | Trastuzumab |

The structures of the compounds described in the following examples were determined by nuclear magnetic resonance ($^1H$ NMR) or mass spectrometry (MS).

The nuclear magnetic resonance ($^1H$ NMR) was determined by a Bruker 400 MHz nuclear magnetic resonance instrument; deuterated chloroform ($CDCl_3$); and the internal standard substance was tetramethylsilane (TMS).

The abbreviations in the nuclear magnetic resonance (NMR) spectra used in the examples were shown below.

s: singlet, d: doublet, t: triplet, q: quartet, m: multiplet, br: broad, J: coupling constant, Hz: Hertz, $CDCl_3$: deuterated chloroform. δ values were expressed in ppm.

The mass spectrometry (MS) was determined by an Agilent (ESI) mass spectrometer, model Agilent 6120B.

Example 1: Pentafluorophenyl (SS,9S)-amino-6-((4-((5S,8S,11S,12R)-11-((S)-sec-butyl)-*12-(2-((S)-2-((1R,2R)-3-(((1S,2R)-1-hydroxy-1-phenylprop-2-yl)amino)-1-methoxy-2-methyl-3*-oxopropyl)pyrrolidin-1-yl)-2-oxoethyl)-5,8-diisopropyl-4,10-dimethyl-3,6,9-trioxo-2,13-dioxa-4, 7,10-triazatetradecyl)phenyl)carbamoyl)-9-isopropyl-1,8,11-trioxy-14,17-dioxa-2,7,10-triaza-eic osanate (DL-A)

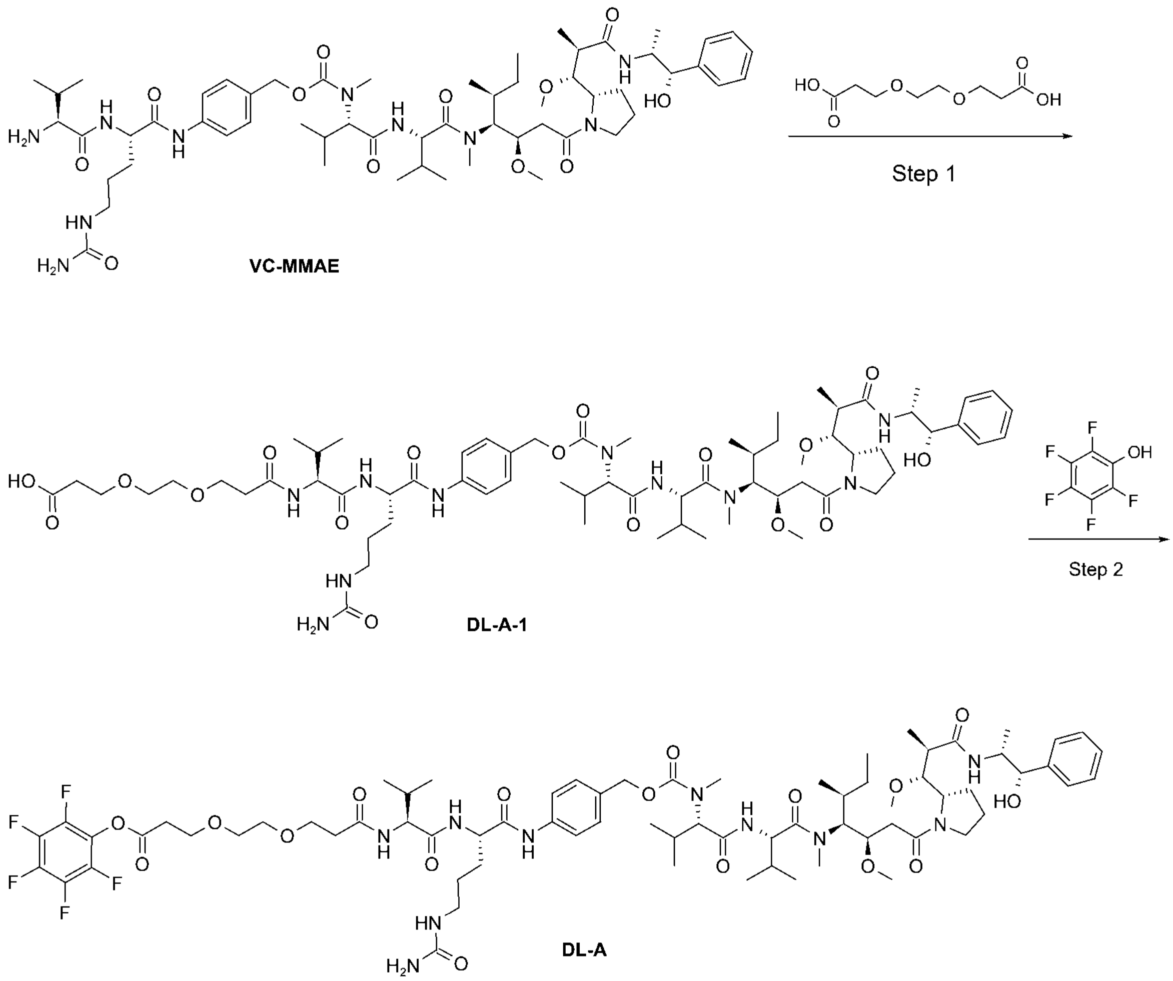


VC-MMAE

DL-A-1

DL-A

Step 1:

VC-MMAE (100 mg, 0.089 mmol), 3,3'-[1,2-ethanediylbis(oxy)]bispropanoic acid (19 mg, 0.089 mmol) and HATU (34 mg, 0.089 mmol) were dissolved in DMF (3 mL), added dropwise with diisopropylethylamine (35 mg, 0.267 mmol), and reacted under stirring for 1 hour. LC-MS was used to monitor the complete consumption of the raw materials. The reaction system was added with 0.1 mL of water, followed by separation and purification using HPLC (conditions were as follows) to obtain DL-A-1 (30 mg).

The structural characterization data were as follows:
ESI-MS (m/z): 1312.6$[M+1]^+$.
Chromatographic column: Waters XBridge Prep C18OBD (5 μm*19mm*150 mm)
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 10 | 90 | 25 |
| 2.00 | 10 | 90 | 25 |
| 18.00 | 90 | 10 | 25 |

Step 2:

DL-A-1 (30 mg, 0.023 mmol), pentafluorophenol (8 mg, 0.046 mmol) and EDCI (34 mg, 0.114 mmol) were dissolved in DMF (1 mL), and reacted under stirring for 4 hours. LC-MS was used to monitor the complete consumption of the raw materials. The reaction system was added with 0.1 mL of water, followed by separation and purification using HPLC (conditions are as follows) to obtain DL-A (3.6 mg).

Structural characterization data were as follows:
ESI-MS (m/z): 1478.7 [M+1]+.
Chromatographic column: Waters XBridge Prep C18OBD (5 μm*19mm*150 mm)
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 10 | 90 | 25 |
| 2.00 | 10 | 90 | 25 |
| 18.00 | 90 | 10 | 25 |

Example 2: Pentafluorophenyl *1-(6-(((S)-1-(((S)-1-((4-((5S,8S,11S,12R)-11-((S)-sec-butyl)-12-(2-((S)-2-((1R,2R)-3-(((1S,2R)-1-hydroxy-1-phenylprop-2-yl)amino)-1-methoxy-2-methyl-3*-oxopropyl)pyrrolidin-1-yl)-2-oxoethyl)-5,8-diisopropyl-4,10-dimethyl-3,6,9-trioxo-2,13-dioxa-4, 7,10-triazatetradecyl)phenyl)amino)-1-oxo-5-ureidopentan-2-yl)amino)-3-methyl-1-oxobut-2-yl) amino)-6-oxohexanoyl)piperidine-4-carboxylate (DL-B)

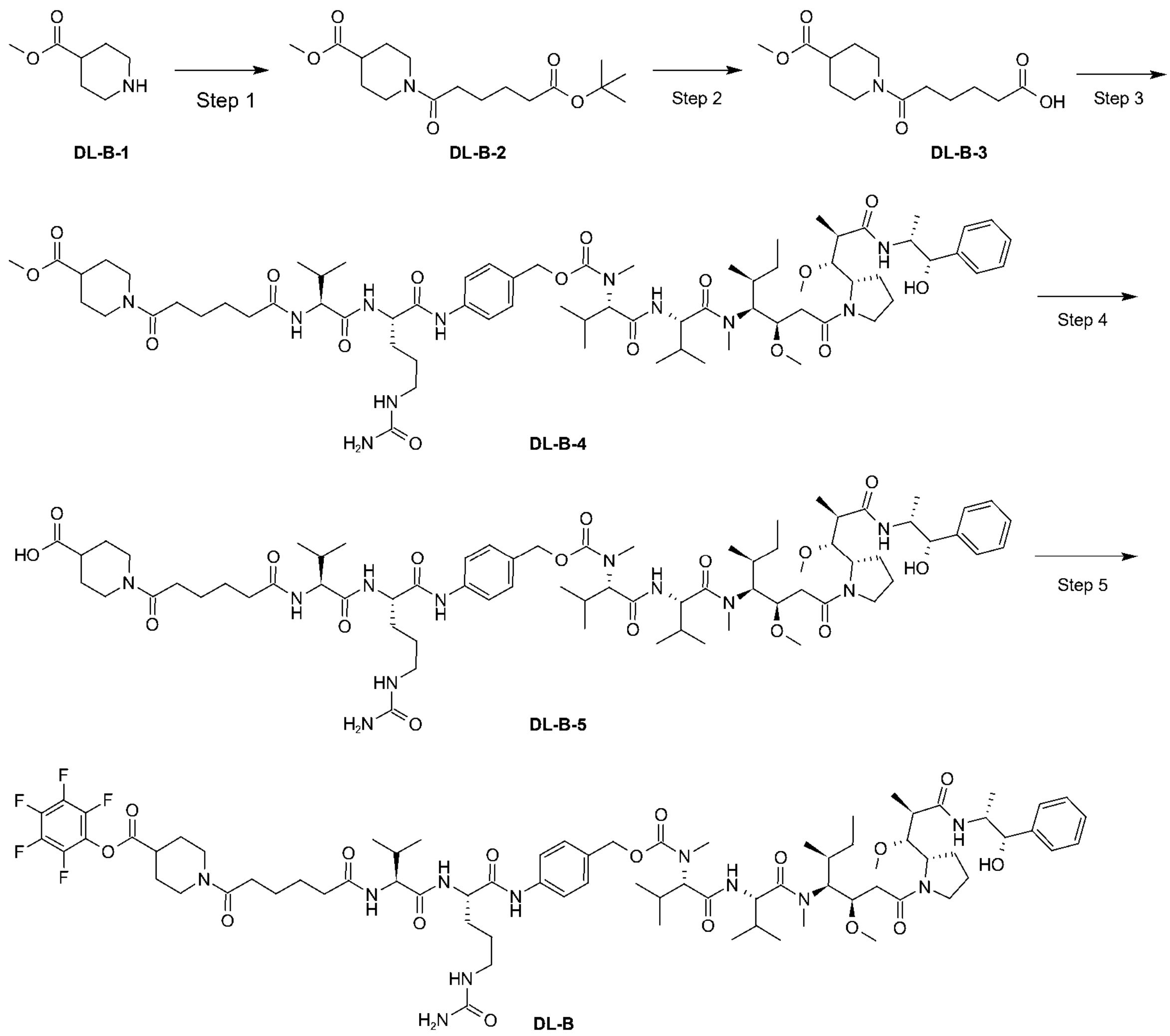

DL-B-1 DL-B-2 DL-B-3 DL-B-4 DL-B-5 DL-B

Step 1:

Methyl piperidine-4-carboxylate (DL-B-1, 500 mg, 3.49 mmol) and 6-(tert-butoxy)-6-oxohexanoic acid (706 mg, 3.49 mmol) were dissolved in DMF (2.00 mL), added with HATU (1.46 g, 3.84 mmol) and DIPEA (1.35 g, 10.48 mmol, 1.82

mL), then heated to 40°C and reacted for 18 hours. The reaction solution was added with water (30.0 mL), and extracted with dichloromethane 3 times (30 mL x 3). The combined organic phase was dried over anhydrous sodium sulfate, filtered and concentrated to obtain a crude product, which was purified by silica gel column chromatography (dichloromethane/methanol = 100/1 to 10/1), and then concentrated again to obtain compound DL-B-2 (500 mg, 1.53 mmol).

The structural characterization data were as follows:
ESI-MS (m/z): 328.5 $[M+H]^+$.

Step 2:

DL-B-2 (500 mg, 1.53 mmol) was dissolved in dichloromethane (5.00 mL), added with trifluoroacetic acid (1.54 g, 13.4 mmol, 1.00 mL), and then reacted at 25°C for 1 hour. The reaction solution was directly concentrated to obtain a crude product of trifluoroacetate salt of DL-B-3 (550 mg, 1.43 mmol).

The structural characterization data were as follows:
ESI-MS (m/z): 271.9 $[M+H]^+$.

Step 3:

VC-MMAE (60.0 mg, 53.4 μmol) and DL-B-3 (20.5 mg, 53.4 μmol, trifluoroacetate) were dissolved in DMF (10.0 mL), added with EDCI (37.5 mg, 195 μmol), HOBt (37.5 mg, 277 μmol) and DIPEA (34.5 mg, 267 μmol, 46.5 μL) in sequence, and then reacted under stirring at 25°C for 1 hour. The reaction solution was added with water (20.0 mL), and extracted with ethyl acetate 3 times (20.0 mL x 3). The organic phases were combined and dried over anhydrous sodium sulfate, filtered and concentrated to obtain a crude product, which was purified by silica gel column chromatography (dichloromethane/methanol = 100/1 to 10/1) and concentrated again to obtain compound DL-B-4 (10.0 mg, 7.26 μmol).

Structural characterization data were as follows:
ESI-MS (m/z): 1377.0 $[M+H]^+$.

Step 4:

DL-B-4 (10.0 mg, 7.26 μmol) was dissolved in a mixed solvent of tetrahydrofuran (0.50 mL), methanol (0.50 mL) and water (0.50 mL), added with lithium hydroxide (347 μg, 14.5 μmol), and stirred at 25°C for 2 hours. After adjusting the pH of the reaction solution to 5 to 6 with 1N dilute hydrochloric acid aqueous solution, solids were precipitated, which were filtered, and the filter cake was vacuum dried to obtain a crude product of compound DL-B-5 (10.0 mg).

The structural characterization data were as follows:
ESI-MS (m/z): 1363.0 $[M+H]^+$.

Step 5:

DL-B-5 (10.0 mg, 7.34 μmol) and pentafluorophenol (2.03 mg, 11.01 μmol) were dissolved in dichloromethane (2.50 mL), added with EDCI (6.33 mg, 33.0 μmol), and then stirred at 30°C for 1 hour. The reaction solution was added with water (10.0 mL), and extracted with dichloromethane 3 times (10.0 mL x 3). The combined organic phase was dried over anhydrous sodium sulfate, filtered and concentrated to obtain a crude product, which was separated and purified by high performance liquid chromatography, and freeze-dried to obtain compound DL-B (2.88 mg, 1.82 μmol).

The structural characterization data were as follows:
ESI-MS (m/z): 1529.6 $[M+H]^+$.

The separation and purification method was as follows:
Chromatographic column: Phenomenex luna C18 (10 μm *25mm*150 mm)
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 55 | 45 | 30 |
| 10.00 | 85 | 15 | 30 |

Example 3: Pentafluorophenyl 1-(*N*-((*6S,9S*)-14-amino-9-((4-((*5S,8S,11S,12R*)-11-((*S*)-*sec-butyl)-12-(2-((S)-2-((1R,2R)-3-(((1S,2R)-1-hydroxy-1-phenylprop-2-yl)amino)-1-methoxy-2* -methyl-3-oxopropyl)pyrrolidin-1-yl)-2-oxoethyl)-5,8-diisopropyl-4,10-dimethyl-3,6,9-trioxo-2, 13-dioxa-4,7,10-triazatetradecyl)phenyl)carbamoyl)-6-isopropyl-4,7,14-trioxo-2-oxa-5,8,13-triaz atetradecyl)aminosulfonyl)piperidine-4-carboxylate (DL-1)

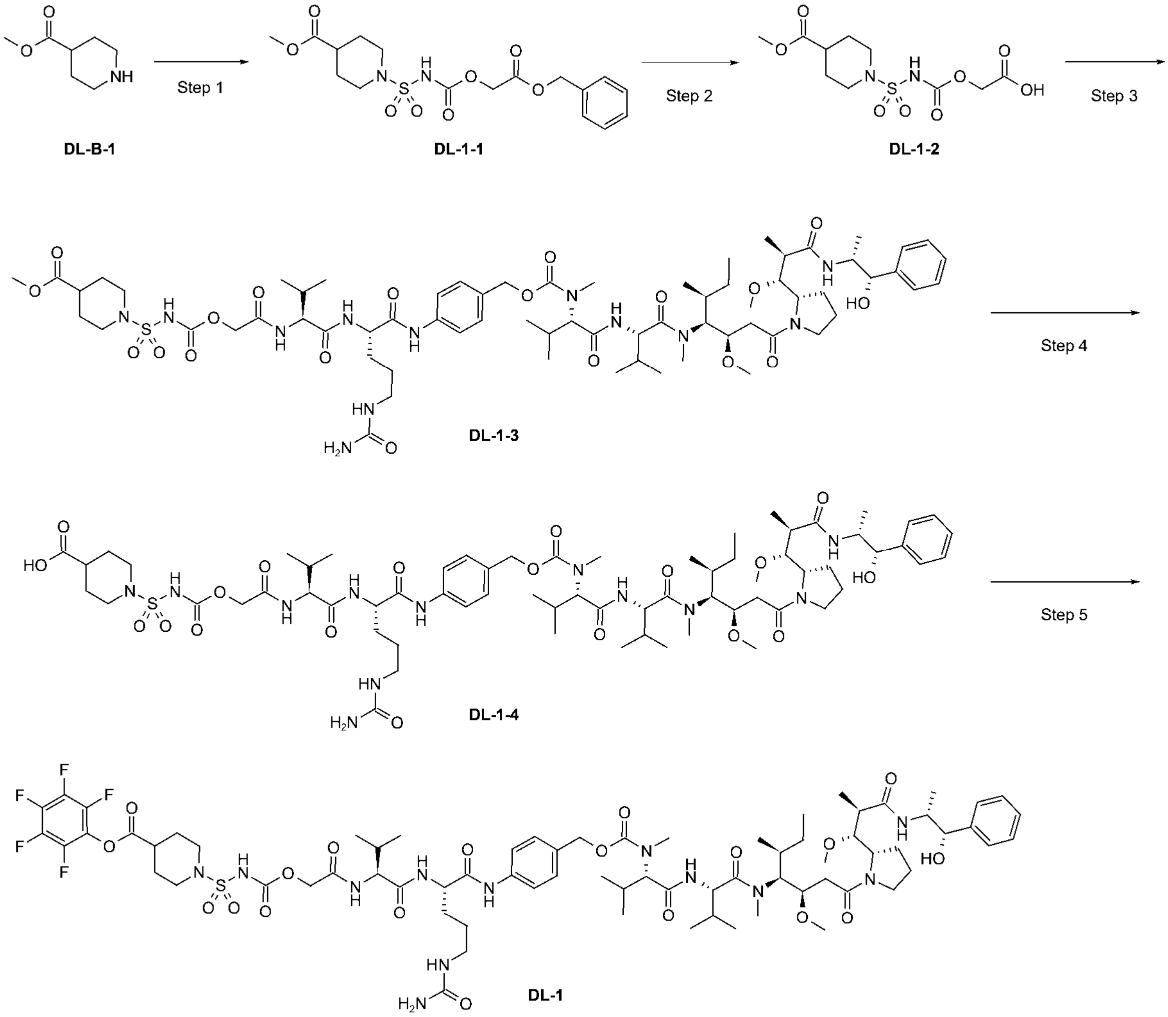

DL-B-1 DL-1-1 DL-1-2

DL-1-3

DL-1-4

DL-1

Step 1

Chlorosulfonyl isocyanate (500 mg, 3.53 mmol, 307 μL) was dissolved in dichloromethane (10.0 mL) at 0 °C, added with benzyl glycolate (533 mg, 3.21 mmol, 455 μL), and then stirred for 1 hour. A solution of methyl piperidine-4-carboxylate DL-B-1 (459 mg, 3.21 mmol) and triethylamine (974 mg, 9.63 mmol, 1.34 mL) in dichloromethane (5.00 mL) was then added to the above reaction solution, heated to 25 °C and stirred for 1 hour. The reaction solution was added with water (100 mL), and extracted with dichloromethane 3 times (50.0 mL x 3). The combined organic phase was washed with cooled 1N dilute hydrochloric acid (20.0 mL), dried over anhydrous sodium sulfate, filtered and concentrated to obtain a crude product of DL-1-1 (1.30 g, 3.14 mmol), which was used directly in the next step without purification.

The structural characterization data were as follows:
ESI-MS (m/z): 414.9 $[M+H]^+$.

Step 2:

Under nitrogen atmosphere, a solution of DL-1-1 (700 mg, 1.69 mmol) in methanol (20.0 mL) was added with Pd/C (0.70 g, 10%), subjected to gas replacement with hydrogen 3 times, and then reacted at 25°C for 3 hours (15 PSI). The reaction solution was filtered, the filter cake was rinsed with methanol 3 times (100 mL x3), and the filtrate was

concentrated to obtain a crude product of DL-1-2 (590 mg).

Step 3:

VC-MMAE (60 mg, 53.4 μmol) and DL-1-2 (17.3 mg, 53.4 μmol) were dissolved in DMF (10.0 mL), added with HOBt (36.0 mg, 267 μmol), DIPEA (34.5 mg, 267 μmol, 46.5 μL) and EDCI (30.7 mg, 160 μmol) in sequence, and then reacted at 25°C for 2 hours. The reaction solution was added with water (20.0 mL), and extracted with dichloromethane 3 times (20.0 mL x 3). The combined organic phase was dried over anhydrous sodium sulfate, filtered and concentrated to obtain a crude product of DL-1-3 (60.0 mg), which was used directly in the next step without purification.

The structural characterization data were as follows:
ESI-MS (m/z): 715.4 $[M/2+H]^+$.

Step 4:

DL-1-3 (50.0 mg, 34.9 μmol) was dissolved in tetrahydrofuran (2.50 mL), water (1.00 mL) and MeOH (1.00 mL), added with lithium hydroxide monohydrate (2.94 mg, 69.9 μmol), and stirred at 30°C for 1 hour. The reaction solution was adjusted to pH 6 with 1N dilute hydrochloric acid aqueous solution, and then directly concentrated to obtain a crude product of DL-1-4 (50.0 mg), which was used directly in the next step without purification.

The structural characterization data were as follows:
ESI-MS (m/z): 1416.6 $[M+H]^+$.

Step 5:

DL-1-4 (50.0 mg, 35.3 μmol) and pentafluorophenol (13.0 mg, 70.6 μmol) were dissolved in DCM (10.0 mL), added with EDCI (30.4 mg, 158 μmol), and stirred at 30°C for 2 hours. The reaction solution was concentrated at 25 °C to obtain a crude product, which was purified by HPLC and freeze-dried to obtain DL-1 (4.70 mg, 2.86 μmol).

The structural characterization data were as follows:
ESI-MS (m/z): 1582.1 $[M+H]^+$.

The separation and purification method was as follows:
Chromatographic column: Phenomenex luna C18 (10μm *25mm*150 mm)
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 56 | 44 | 30 |
| 10.00 | 86 | 14 | 30 |

Example 4: Pentafluorophenyl 1-(*N*-((2-(((*S*)-1-(((*S*)-1-((4-((*5S,8S,11S,12R*)-11-((*S*)-*sec-butyl)-12-(2-((S)-2-((1R,2R)-3-(((1S,2R)-1-hydroxy-1-phenylprop-2-yl)amino)-1-methoxy-2* -methyl-3-oxopropyl)pyrrolidin-1-yl)-2-oxoethyl)-5,8-diisopropyl-4,10-dimethyl-3,6,9-trioxo-2, 13-dioxa-4,7,10-triazatetradecyl)phenyl)amino)-1-oxoprop-2-yl)amino)-3-methyl-1-oxobut-2-yl) amino)-2-oxoethoxy)carbonyl)aminosulfonyl)piperidine-4-carboxylate (DL-2)

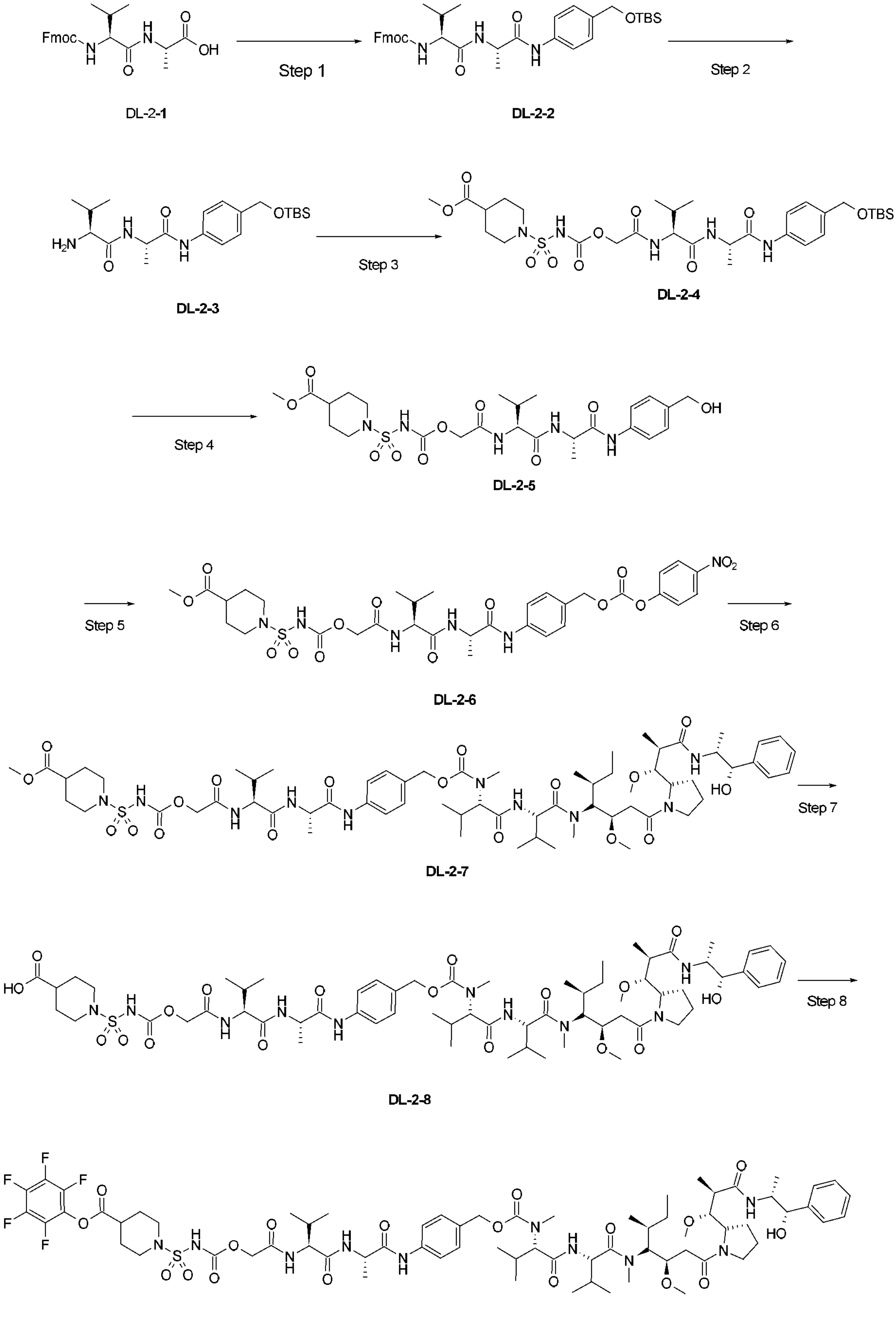


DL-2-1

DL-2-2

DL-2-3

DL-2-4

DL-2-5

DL-2-6

DL-2-7

DL-2-8

DL-2

Step 1:

(((9H-Fluoren-9-yl)methoxy)carbonyl)-L-valine-L-alanine (DL-2-1, 1.00 g, 2.44 mmol) and 4-(((tert-butyldimethylsilyl)oxy)methyl)aniline (1.16 g, 4.87 mmol) were dissolved in dichloromethane (10.0 mL) and methanol (5.00 mL), added with EEDQ (1.20 g, 4.87 mmol), and stirred at 25 °C for 5 hours. The reaction solution was concentrated to obtain a crude product, which was purified by silica gel column (petroleum ether/ethyl acetate = 100/1 to 0/100), and then concentrated again to obtain compound DL-2-2 (1.51 g, 2.37 mmol), which was used directly in the next step without purification.

Its structural characterization data were as follows:
ESI-MS (m/z): 630.3 $[M+H]^+$.

Step 2:

(9H-Fluoren-9-yl)methyl ((S)-1-(((S)-1-((4-(((tert-butyldimethylsilyl)oxy)methyl)phenyl) amino)-1-oxoprop-2-yl) amino)-3-methyl-1-oxobut-2-yl)carbamate (DL-2-2, 1.45 g, 2.30 mmol) was dissolved in DMF (10.0 mL), added with DBU (350 mg, 2.30 mmol, 347 μL), and stirred at 25 °C for 1 hour. The reaction solution was used directly in the next step without treatment.

Its structural characterization data were as follows:
ESI-MS (m/z): 408.2 $[M+H]^+$.

Step 3:

2-((((4-(Methoxycarbonyl)piperidin-1-yl)sulfonyl)carbamoyl)oxy)acetic acid (795 mg, 2.45 mmol) was dissolved in DMF (5.0 mL), added to the reaction solution of the previous step, then added with HOBt (1.66 g, 12.2 mmol), EDCI (1.41 g, 7.36 mmol) and DIPEA (1.59 g, 12.2 mmol, 2.14 mL), and stirred at 25 °C for 2 hours. The reaction solution was added with water (100 mL), and extracted with ethyl acetate 3 times (100 mL x 3). The combined organic phase was dried over anhydrous sodium sulfate, filtered and concentrated to obtain a crude product, which was purified by silica gel column (dichloromethane/methanol = 100/1 to 97/3) and concentrated again to obtain compound DL-2-4 (1.25 g, 1.57 mmol).

Its structural characterization data were as follows:
ESI-MS (m/z): 736.2 [M+Na]+.

Step 4:

Methyl 1-(*N*-((2-(((*S*)-1-(((*S*)-((4-(((tert-butyldimethylsilyl)oxy)methyl)phenyl)amino)-1-oxoprop-2-yl)amino)-3-methyl-1-oxobut-2-yl)amino)-2-oxoethoxy)carbonyl)aminosulfonyl)pipe ridine-4-carboxylate (DL-2-4, 1.15 g, 1.61 mmol) was dissolved in tetrahydrofuran (12.0 mL), added with TBAF (1 M, 4.83 mL), and stirred at 25 °C for 1 hour. The reaction solution was added with water (100 mL), and extracted with dichloromethane 3 times (50.0 mL x3). The combined organic phase was dried over anhydrous sodium sulfate, filtered and concentrated to obtain a crude product, which was purified by silica gel column (dichloromethane/methanol = 100/1 to 95/5) and concentrated again to obtain compound DL-2-5 (900 mg, 1.38 mmol).

Its structural characterization data were as follows:
ESI-MS (m/z): 600.1 $[M+H]^+$.

Step 5:

Methyl 1-(*N*-((2-(((*S*)-1-(((*S*)-1-((4-(hydroxymethyl)phenyl)amino)-1-oxoprop-2-yl) amino)-3-methyl-1-oxobut-2-yl)amino)-2-oxoethoxy)carbonyl)aminosulfonyl)piperidine-4-carbo xylate (DL-2-5, 850 mg, 1.42 mmol) and p-nitrophenyl chloroformate (428 mg, 2.13 mmol) were dissolved in dichloromethane (10 mL), added with trifluoroacetic acid (430 mg, 4.25 mmol, 591 μL), and then stirred at 25 °C for 1 hour. The reaction solution was added with water (100 mL), and extracted with dichloromethane 3 times (50.0 mL x 3). The combined organic phases were dried over anhydrous sodium sulfate, filtered and concentrated to obtain a crude product, which was purified by silica gel column (dichloromethane/methanol = 100/1 to 94/6) and concentrated again to obtain compound DL-2-6 (450 mg, 508.99 μmol).

Its structural characterization data were as follows:
ESI-MS (m/z): 765.2 $[M+H]^+$.

Step 6:

Methyl 1-(N-((2-(((S)-3-methyl-1-(((S)-1-((4-((((4-nitrophenoxy)carbonyl)oxy)methyl) phenyl)amino)-1-oxoprop-2-yl)amino)-1-oxobut-2-yl)amino)-2-oxoethoxy)carbonyl)aminosulfo nyl)piperidine-4-carboxylate (DL-2-6, 400 mg, 523 μmol) and MMAE (268 mg, 373 μmol) were dissolved in DMF (6.00 mL), added with HOBt (25.2 mg, 186 μmol) and DIPEA (169 mg, 1.31 mmol, 227 μL), and stirred at 25 °C for 10 hours. The reaction solution was added with water (100 mL), and extracted with dichloromethane 3 times (50.0 mL x 3). The organic phases were combined and dried over anhydrous sodium sulfate, filtered and concentrated to obtain a crude product, which was purified with a silica gel column (dichloromethane/methanol = 100/1 to 94/6), and concentrated again to obtain compound DL-2-7 (650 mg).

Its structural characterization data were as follows:
ESI-MS (m/z): 1343.9 $[M+H]^+$.

Step 7:

Methyl 1-(*N*-((2-(((*S*)-1-(((*S*)-1-((4-((*5S,8S,11S,12R*)-11-((S,sec-butyl)-12-(2-((*S*)-2-((1R,2R)-3-(((1S,2R)-1-hydroxy-1-phenylprop-2-yl)amino)-1-methoxy-2-methyl-3-oxypropyl)py rrolidin-1-yl)-2-oxoethyl)-5,8-diisopropyl-4,10-dimethyl-3,6,9-trioxo-2,13-dioxa-4,7,10-triazatet radecyl)phenyl)amino)-1-oxoprop-2-yl)amino)-3-methyl-1-oxobut-2-yl)amino)-2-oxoethoxy)car bonyl)aminosulfonyl)piperidine-4-carboxylate (DL-2-7,600 mg, 446 μmol) was dissolved in a mixed solvent of tetrahydrofuran (2.00 mL), methanol (2.00 mL) and water (2.00 mL), added with lithium hydroxide monohydrate (37.4 mg, 893 μmol), then heated to 45 °C and stirred for 2 hours. The reaction solution was adjusted to pH 6 with citric acid aqueous solution and filtered, the filter cake was vacuum dried to obtain the compound DL-2-8 (530 mg), which was directly used in the next step without purification.

Its structural characterization data were as follows:
ESI-MS (m/z): 665.3 $[M/2+H]^+$.

Step 8:

1-(*N*-(2-(((*S*)-1-(((*S*)-1-((4-((5*S*,8*S*,11*S*,12*R*)-11-((*S*,sec-butyl)-12-(2-((*S*)-2-((*1R,2R*)-3-(((*1S* ,*2R*)-1-hydroxy-1-phenylprop-2-yl)amino)-1-methoxy-2-methyl-3-oxypropyl)pyrrolidin-1-yl)-2-oxoethyl)-5,8-diisopropyl-4,10-dimethyl-3,6,9-trioxo-2,13-dioxa-4,7,10-triazatetradecyl)phenyl) amino)-1-oxoprop-2-yl)amino)-3-methyl-1-oxobut-2-yl) amino)-2-oxoethoxy)carbonyl)aminosul fonyl)piperidine-4-carboxylic acid (DL-2-8, 500 mg, 376 μmol) and pentafluorophenol (138 mg, 752 μmol) were dissolved in dichloromethane (10.0 mL), added with EDCI (324 mg, 1.69 mmol), and then stirred at 25 °C for 1 hour. The reaction solution was maintained at 30 °C and concentrated to obtain a crude product, which was purified by preparative HPLC and freeze-dried to obtain the title compound (18.5 mg, 8.45 μmol).

Its structural characterization data were as follows:
ESI-MS (m/z): 1496.2 $[M+H]^+$.

The purification and separation method was as follows:
Chromatographic column: Phenomenex luna C18 (10 μm*25mm*150 mm)
Mobile phase A: acetonitrile; Mobile phase B: water (0.05% formic acid)

| Time [min] | Mobile phase A [%] | Mobile phase B [%] | Flow rate [mL/min] |
|---|---|---|---|
| 0.00 | 52 | 48 | 28 |
| 11.00 | 82 | 18 | 28 |

Preparation Example (preparation of antibody-drug conjugates)

Preparation of Trastuzumab-DL-A (target DAR 2)

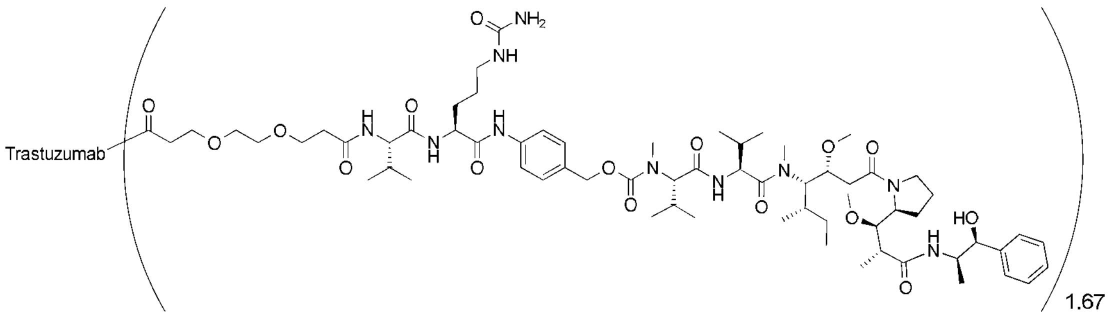

0.31mL of trastuzumab (16.2 mg/mL) was taken, adjusted to pH 7.40 with 1M $Na_2HPO_4$ solution, added with a solution of DL-A (17.77 uL, 10 mM, equivalent to 5 times the moles of antibody) in dimethyl sulfoxide, mixed well, and allowed to stand at room temperature for 18 hours. Upon completion, the buffer was exchanged using a NAP-5 gel column (Cytiva)with a 20 mM histidine buffer solution at pH 6.0 to obtain an antibody-drug conjugate (Trastuzumab-DL-A). The average loading (DAR value) as determined by mass spectrometry was 1.67, with DAR2 accounting for 36.11%.

Table 1: Measured molecular weight and DAR calculation of Trastuzumab-DL-A

| Loading | Molecular weight | Maximum signal intensity | Ratio | DAR |
|---|---|---|---|---|
| Naked antibody | 148054.79 | 605.36 | 13.96% | 1.67 |
| DAR1 | 149347.24 | 1294.39 | 29.86% | |
| DAR2 | 150640.68 | 1565.23 | 36.11% | |
| DAR3 | 151924.88 | 680.83 | 15.71% | |
| DAR4 | 153228.63 | 189.17 | 4.36% | |
| DAR5 | 154520.63 | 0.00 | 0.00% | |
| DAR6 | 155812.63 | 0.00 | 0.00% | |

Preparation of Trastuzumab-DL-B (target DAR 2)

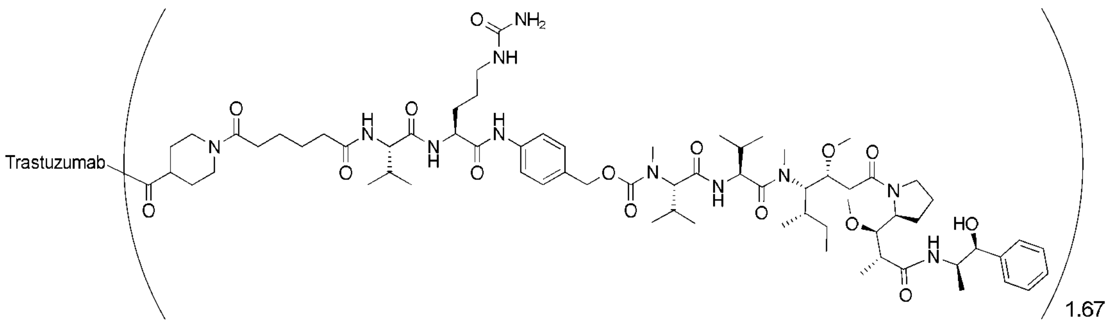

0.31mL of trastuzumab (16.2 mg/mL) was taken, adjusted to pH 7.40 with 1M $Na_2HPO_4$ solution, added with a solution of DL-B (17.24 uL, 10 mM, equivalent to 5 times the moles of antibody) in dimethyl sulfoxide, mixed well, and allowed to stand at room temperature for 18 hours. Upon completion, the buffer was exchanged using a NAP-5 gel column (Cytiva) with a 20 mM histidine buffer solution at pH 6.0 to obtain an antibody-drug conjugate (Trastuzumab-DL-B). The average loading (DAR value) as determined by mass spectrometry was 1.67, with DAR2 accounting for 44.88%.

Table 2: Measured molecular weight and DAR calculation of Trastuzumab-DL-B

| Loading | MWExp. | Maximum signal intensity | Ratio | DAR |
|---|---|---|---|---|
| Naked antibody | 148053.07 | 482.20 | 11.09% | 1.67 |
| DAR1 | 149398.09 | 1241.93 | 28.55% | |
| DAR2 | 150742.45 | 1952.01 | 44.88% | |
| DAR3 | 152088.68 | 577.81 | 13.28% | |
| DAR4 | 153430.23 | 95.45 | 2.19% | |
| DAR5 | 154774.23 | 0.00 | 0.00% | |
| DAR6 | 156118.23 | 0.00 | 0.00% | |

Preparation of Trastuzumab-DL-1 (target DAR 2)

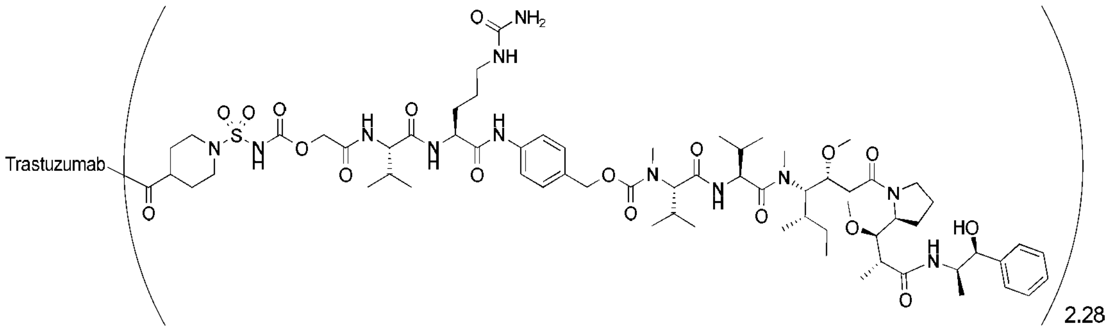

0.31mL of trastuzumab (16.2 mg/mL) was taken, adjusted to pH 7.40 with 1M $Na_2HPO_4$ solution, added with a solution of DL-1 (18.15 uL, 10 mM, equivalent to 5 times the moles of antibody) in dimethyl sulfoxide, mixed well, and allowed to stand at room temperature for 18 hours. Upon completion, the buffer was exchanged using a NAP-5 gel column (Cytiva) with a 20 mM histidine buffer solution at pH 6.0 to obtain an antibody-drug conjugate (Trastuzumab-DL-1). The average loading (DAR value) as determined by mass spectrometry was 2.28, with DAR2 accounting for 60.28%.

Table 3: Measured molecular weight and DAR calculation of Trastuzumab-DL-1

| Load | MWExp. | Maximum signal intensity | Ratio | DAR |
|---|---|---|---|---|
| Naked antibody | 148057.34 | 36.56 | 1.43% | 2.28 |
| DAR1 | 149456.22 | 176.03 | 6.87% | |
| DAR2 | 150851.01 | 1543.85 | 60.28% | |
| DAR3 | 152247.54 | 645.37 | 25.20% | |
| DAR4 | 153645.07 | 145.22 | 5.67% | |
| DAR5 | 155039.50 | 14.11 | 0.55% | |
| DAR6 | 156435.50 | 0.00 | 0.00% | |

Preparation of Trastuzumab-DL-2 (target DAR 2)

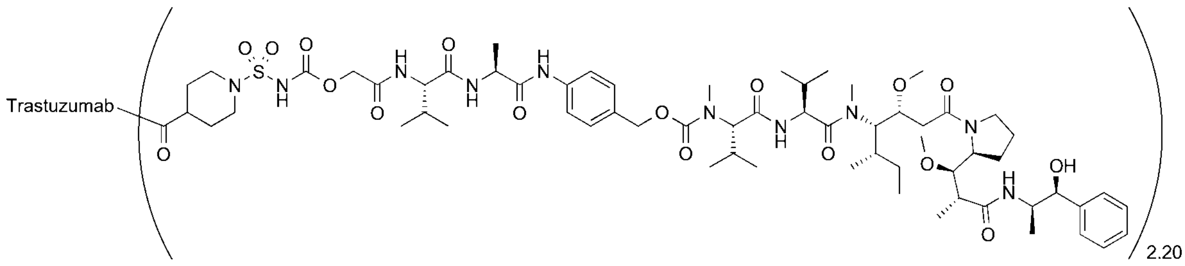

2.16mL of trastuzumab (16.2 mg/mL) was taken, adjusted to pH 7.40 with 1M $Na_2HPO_4$ solution, added with a solution of DL-2 (234 uL, 10 mM, equivalent to 5 times the moles of antibody) in dimethyl sulfoxide, mixed well, and allowed to stand at room temperature for 18 hours. Upon completion, the buffer was exchanged using a NAP-5 gel column (Cytiva) with a 20 mM histidine buffer solution at pH 6.0 to obtain an antibody-drug conjugate (Trastuzumab-DL-2). The average loading (DAR value) as determined by mass spectrometry was 2.20.

Table 4: Measured molecular weight and DAR calculation of Trastuzumab-DL-2

| Load | MWExp. | Maximum signal intensity | Ratio | DAR |
|---|---|---|---|---|
| Naked antibody | 148060.95 | 21.54 | 1.99% | 2.20 |
| DAR1 | 149374.75 | 109.51 | 10.11% | |
| DAR2 | 150684.05 | 635.59 | 58.65% | |
| DAR3 | 151995.59 | 268.82 | 24.81% | |
| DAR4 | 153310.32 | 48.18 | 4.45% | |
| DAR5 | 154620.32 | 0.00 | 0.00% | |
| DAR6 | 155930.32 | 0.00 | 0.00% | |

According to the loading (DAR value) and the proportion of DAR2 in the above coupling examples, it indicated that the chemical coupling linkers employed in the present application, especially the linkers featuring a piperidine sulfonyl carbamate structure, facilitated the enhancement of coupling efficiency and the attainment of antibody-drug conjugates with greater homogeneity and higher DAR2 ratios.

Test Example 1: Detection of *in vitro* activity of antibody-drug conjugates on cells

1. Inhibitory effects of antibody-drug conjugates on proliferation of HT-29 cells

(1) Cell plating: First, tumor cells HT-29 were cultured in the corresponding culture medium; the cells were digested with trypsin, centrifuged, resuspended and counted; and the cells were adjusted to a suitable concentration for plating. The source of tumor cells was shown in Table 5.

Table 5: Source of tumor cells

| Cell name | Tumor type | Source |
|---|---|---|
| HT-29 | Human colon cancer cell | Cell Bank of Chinese Academy of Sciences |

Co-incubation of the ADC of the present application and the tumor cells: After the cells were attached to the wall, the culture medium was removed, and the diluted bioactive molecule (the ADC of the present application) was added to wells of the above plate, and incubated for 96 hours.

Detection of *in vitro* cell activity: After the incubation, 50$\mu$L of Cell Counting-Lite™ 2.0 reagent (Vazyme/Novozyme) was added to each well, mixed well by oscillation in the dark, reacted for 10 min, and then detected using a microplate reader (manufacturer: BMG, model: PHERAStar-FS) for reading. Cell Counting-Lite™ without cell culture medium was used to obtain the background RLU, and Cell Counting-Lite™ with cell culture medium was used to obtain the solvent RLU. Cell inhibition rate = 1 - (sample RLU - background RLU)/(solvent RLU - background RLU) $\times$ 100%. According to the four-parameters model fitting curve, the half inhibitory concentration ($IC_{50}$) of compound was calculated. RLU: relative light unit.

(2) Data results: The detection results were shown in Table 6.

Table 6: Killing results of the antibody-drug conjugates on HT-29 cell line (96 hours)

| ADC No. | $IC_{50}$ (μg/mL) |
|---|---|
| Trastuzumab-DL-A | 42.43 |
| Trastuzumab-DL-B | 26.37 |
| Trastuzumab-DL-1 | 10.22 |

The detection results (inhibitory effect) of the antibody-drug conjugates (ADCs) on cell activity *in vitro* showed that the ADC molecules obtained by coupling with the new chemical coupling linkers had significant tumor cell killing effects. Compared with the control ADCs (Trastuzumab-DL-A and Trastuzumab-DL-B), the ADC (Trastuzumab-DL-1) disclosed in the present application exhibited better cell killing activity, indicating that the ADC molecules obtained by coupling using the new chemical coupling linkers were effective in inhibiting tumor growth.

Test Example 2: Evaluation of inhibitory effects of antibody-drug conjugates on tumor growth in subcutaneously transplanted tumor model mice

The preparations containing the ADCs of the present invention were respectively administered through tail vein injection to the CDX model mice with subcutaneously transplanted human breast cancer cells JIMT-1, and the changes in tumor volume and animal body weight were measured once a week to calculate the tumor inhibition efficacy of the ADCs of the present invention in the tumor-bearing mice.

Test drugs

Drug name, source, preparation method: An appropriate amount of the ADC of the present invention was taken, and the mother solution was diluted using 0.9% NaCl injection to obtain the administration solution according to the administration volume of 3 mg/kg. 0.9% NaCl injection was used as the solvent control (Vehicle).

Experimental animals and cell lines

NOD SCID mice (Gempharmatech, Chengdu)

Human breast cancer cells JIMT-1 (Nanjing Cobioer Biotechnology Co., Ltd.)

Experimental grouping and evaluation method

The tumor-bearing mice with an average tumor volume of 100 to 150 $mm^3$ were randomized into groups (the number of groups was determined according to the number of samples). According to the groups, 0.9% NaCl injection (hereinafter referred to as the solvent control, Vehicle) and the ADCs of the present invention were administrated respectively, and the administration frequencies thereof were shown in the specific examples. The administration method was tail vein injection, and the administration volume was 10 ml/kg. After administration, the tumor diameter was measured with a vernier caliper once a week, and the tumor volume was calculated according to the following calculation formula: $V = 0.5\ a \times b^2$, wherein a and b represented the long diameter and short diameter of the tumor, respectively. The death of the animals was observed and recorded every day.

The tumor growth inhibition rate TGI (%) was calculated using the following formula to evaluate the tumor inhibition efficacy of the ADCs of the present invention:

VT end>VT0, TGI (%) = [1-(VT end - VT0)/(VC end - VC0)]*100%; or, VT end≤VT0, TGI (%) = [1 - (VT end - VT0)/VT0] * 100%.

wherein VT end: mean of tumor volume at the end of experiment in treatment group

VT0: mean of tumor volume at the beginning of administration in treatment group
VC end: mean of tumor volume at the end of experiment in the negative control group

VC0: mean of tumor volume at the beginning of administration in the negative control group

The following formula was used to calculate the tumor relative proliferation rate T/C (%), which was used to evaluate the tumor inhibition efficacy of the ADCs of the present invention:

$$T/C = (VT\ end/VT0)/(VC\ end/VC0).$$

(1) Pharmacological efficacy test of anti-human HER2 antibody-drug conjugate in JIMT-1 model

JIMT-1 cells were cultured in DMEM medium containing 10% fetal bovine serum at 37°C and 5% $CO_2$. The JIMT-1 cells in the exponential growth phase were collected, resuspended to a suitable concentration with matrix gel: PBS = 1:1 (v/v), and inoculated subcutaneously in female NOD SCID mice to establish a breast cancer model. When the average tumor volume was about 150 $mm^3$, the mice were randomized into groups according to the tumor size, including in sequence: a vehicle control group (i.e., negative control, Vehicle group), and groups of Trastuzumab-DL-A, Trastuzumab-DL-B, Trastuzumab-DL-1 and Trastuzumab-DL-2 of the present invention. Each group was injected via tail vein (i.v.), and the drug was administered on Day 0, for a total of 1 dose. After administration, the mouse weight was measured once a week, and the long and short diameters of tumor were measured with a vernier caliper, and the tumor volume was calculated according to the following calculation formula: $V = 0.5\ a \times b^2$, wherein a and b represented the long and short diameters of tumor, respectively, and the death of animals was observed and recorded every day.

The ADCs of the present invention had significant inhibitory effects on tumor growth in the JIMT-1 human breast cancer transplanted tumor model. Compared with the Vehicle group, the tumor growth inhibition rates (TGI) of the groups of Trastuzumab-DL-A, Trastuzumab-DL-B, Trastuzumab-DL-1 and Trastuzumab-DL-2 of the present invention were 38.54%, 15.04%, 75.08% and 80.90%, respectively. On Day 21, there was no animal death or significantly loss of animal weight in each treatment group, and no obvious drug toxicity was observed. During the treatment period, the mice had good tolerance to the ADCs of the present invention. The specific results were shown in Table 7 and Figures 1 and 2.

Table 7: Human breast cancer cell JIMT-1 CDX model

| Group | Dose (mg/kg) | Day 21 | | | |
|---|---|---|---|---|---|
| | | Tumor volume ($mm^3$) ($\bar{x}\pm$SEM) | TGI (%) | T/C (%) | P value (vs.Vehicle) |
| Vehicle | - | 926.94±39.42 | - | - | - |
| Trastuzumab-DL-A | 3 | 667.33±60.57 | 38.54 | 71.52 | <0.01 |
| Trastuzumab-DL-B | 3 | 825.50±31.21 | 15.04 | 88.88 | <0.1 |
| Trastuzumab-DL-1 | 3 | 421.60±64.96 | 75.08 | 44.84 | <0.001 |
| Trastuzumab-DL-2 | 3 | 380.68±61.71 | 80.90 | 40.73 | <0.001 |
| Note: TGI was tumor growth inhibition rate, T/C was relative tumor proliferation rate, the same below. | | | | | |

Test Example 3: Evaluation of inhibitory effects of antibody-drug conjugates on tumor growth in subcutaneously transplanted tumor model mice

The preparations containing the ADCs of the present invention were respectively administered through tail vein injection to the CDX model mice with subcutaneously transplanted human gastric cancer cells NCI-N87, and the changes in tumor volume and animal body weight were measured twice a week to calculate the tumor inhibition efficacy of the ADCs of the present invention in the tumor-bearing mice.

Test drug

Drug name, source, preparation method: An appropriate amount of the ADC of the present invention was taken, and the mother solution was diluted using 0.9% NaCl injection to obtain the administration solution according to the administration volume of 1 mg/kg. 0.9% NaCl injection was used as the solvent control (Vehicle).

Experimental animal and cell line

Balb/c Nude mice (Gempharmatech, Chengdu, production license number: SCXK (Chuan) 2020-0034, animal qualification certificate number: 511214900025102)

Human gastric cancer cells NCI-N87 (ATCC)

Experimental grouping and evaluation method

The tumour-bearing mice with an average tumor volume of about 150 $mm^3$ were randomized into groups (the number of groups was determined according to the number of samples). According to the groups, 0.9% NaCl injection (hereinafter referred to as the vehicle control, Vehicle) and the ADCs of the present invention were administrated respectively, and the frequency of administration was shown in the specific example. The administration method was tail vein injection, and the administration volume was 10 ml/kg. After administration, the tumor diameter was measured with a vernier caliper twice a week, and the tumor volume was calculated according to the following calculation formula: $V = 0.5\ a \times b^2$, wherein a and b represented the long diameter and short diameter of the tumor, respectively. The death of animals was observed and recorded every day.

The tumor growth inhibition rate TGI (%) was calculated using the following formula, and used to evaluate the anti-tumor efficacy of the ADCs of the present invention:

VT end>VT0, TGI (%) = [1 - (VT end - VT0)/(VC end - VC0)]*100%; or, VT end≤VT0, TGI (%) = [1 - (VT end - VT0)/VT0] * 100%,

wherein VT end: mean of tumor volume at the end of experiment in treatment group

VT0: mean of tumor volume at the beginning of administration in treatment group
VC end: mean of tumor volume at the end of experiment in the negative control group
VC0: mean of tumor volume at the beginning of administration in the negative control group

The following formula was used to calculate the tumor relative proliferation rate T/C (%), which was used to evaluate the anti-tumor efficacy of the ADCs of the present invention:

$$T/C = (VT\ end/VT0)/(VC\ end/VC0).$$

(1) Detection of efficacy anti-human HER2 antibody-drug conjugates in NCI-N87 model

NCI-N87 cells were cultured under 5% $CO_2$ conditions in RPMI 1640 culture medium containing 10% fetal bovine serum at 37°C. The NCI-N87 cells in the exponential growth phase were collected, resuspended in PBS to a suitable concentration, and inoculated subcutaneously into female Balb/c-nu mice to establish a gastric cancer model. When the average tumor volume was about 150 $mm^3$, the mice were randomized into groups according to the tumor size, including in order: a vehicle control group (i.e., negative control, Vehicle group), groups of Trastuzumab-DL-1 1 mg/kg, and Trastuzumab-DL-2 group of the present invention; the mice were injected via tail vein injection (i.v.) on Day 0, Day 7, and Day 14, for a total of 3 injections. After administration, the weight of mice was measured twice a week, and the long and short diameters of tumors were measured with a vernier caliper, and the tumor volume was calculated according to the following calculation formula: $V = 0.5\ a \times b^2$, wherein a and b represented the long diameter and short diameter of tumor, respectively. The death of animals was observed and recorded every day.

The ADCs of the present invention had a significant inhibitory effect on tumor growth in the NCI-N87 gastric cancer transplant model. Compared with the Vehicle group, the tumor growth inhibition rates (TGI) of the Trastuzumab-DL-1 1 mg/kg group and the Trastuzumab-DL-2 1 mg/kg group of the present invention were 66.23% and 63.87%, respectively. On Day 20, there was no animal death or significantly loss of animal weight in the treatment groups, and no obvious drug toxicity was observed. During the treatment period, the mice had good tolerance to the ADCs of the present invention. The specific results were shown in Table 8 and Figures 3 and 3.

Table 8: Human gastric cancer cell NCI-N87 CDX model

| Group | Dose (mg/kg) | Day 20 | | | |
|---|---|---|---|---|---|
| | | Tumor volume ($mm^3$) ($\bar{x}$±SEM) | TGI (%) | T/C (%) | P value (vs.Vehicle) |
| Vehicle | - | 583.55±79.39 | - | - | - |
| Trastuzumab-DL-1 | 1 | 314.11±58.87 | 66.23 | 53.48 | <0.05 |
| Trastuzumab-DL-2 | 1 | 324.17±16.65 | 63.87 | 55.07 | <0.05 |

Note: TGI was tumor growth inhibition rate, T/C was relative tumor proliferation rate.

In addition to those described herein, various modifications of the present invention will be obvious to those skilled in the art based on the foregoing description. Such modifications are also intended to fall within the scope of the appended claims. The references cited in the present application (including all patents, patent applications, journal articles, books and any other disclosures) were incorporated herein by reference in their entirety.

## Claims

**1.** A compound or a pharmaceutically acceptable salt, ester, stereoisomer, tautomer or isotope-labeled compound thereof, wherein the compound has a structure represented by Formula I:

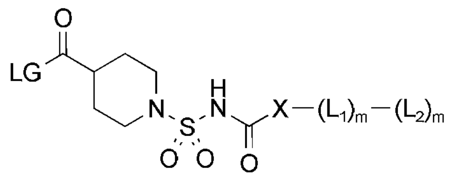

Formula I

wherein:

LG is a leaving group or a reactive group;
X is selected from the group consisting of -O-, -$NR^2$- and -$CHR^3$-; $R^2$ and $R^3$ are each independently selected from the group consisting of hydrogen and $C_{1-6}$ alkyl; the $C_{1-6}$ alkyl is optionally substituted with one or more groups independently selected from the group consisting of amino, alkylamino, nitrogen-containing heterocyclyl, sulfonic acid group, carboxylic acid group, quaternary ammonium salt, hydroxyl and alkoxy;
each $L_1$ is independently selected from the group consisting of single bond, $C_{1-6}$ alkylene, -$(CH_2CH_2O)_y$-, $C_{2-6}$ alkenylene, an amino acid residue, a fragment of a polypeptide formed with 2 to 10 amino acids, 4- to 16-membered heterocyclylene, 5- to 16-membered heteroarylene, phenylene, amino, -CO-NH- and -NH-CO-; the $C_{1-6}$ alkylene, amino, $C_{2-6}$ alkenylene, 4- to 16-membered heterocyclylene, 5- to 16-membered heteroarylene, phenylene, -CO-NH- and -NH-CO- are optionally substituted with one or more groups independently selected from the group consisting of sulfonic acid group, phosphoric acid group, carboxylic acid group, amide group, quaternary ammonium salt, hydroxyl, alkyl, alkoxy, alkoxyalkyl, alkynyl and azido;
each $L_2$ is independently selected from the group consisting of $C_{2-6}$ alkynyl, tetrazinyl, methyltetrazinyl, trans-cyclooctenyl, benzoazacyclooctynyl, (*1R,8S,9S*)-bicyclo[6.1.0]non-4-yn-9-yl, azido, $C_{1-6}$ alkylacyl, aldehyde group, hydroxylamine group, oxime group, 4- to 16-membered heterocyclyl and 5- to 16-membered heteroaryl;
each m is independently an integer of 1 to 10;
y is an integer of 1 to 20.

**2.** A compound or a pharmaceutically acceptable salt, ester, stereoisomer, tautomer or isotope-labeled compound thereof, wherein the compound has a structure represented by Formula II:

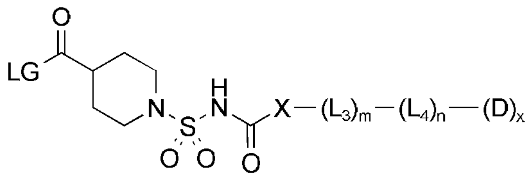

Formula II

wherein:

LG is a leaving group or a reactive group;
X is selected from the group consisting of -O-, -$NR^2$- and -$CHR^3$-; $R^2$ and $R^3$ are each independently selected from the group consisting of hydrogen and $C_{1-6}$ alkyl; the $C_{1-6}$ alkyl is optionally substituted with one or more groups independently selected from the group consisting of amino, alkylamino, nitrogen-containing heterocyclyl, sulfonic

acid group, carboxylic acid group, quaternary ammonium salt, hydroxyl and alkoxy;
each $L_3$ is independently selected from the group consisting of single bond, $C_{1-6}$ alkylene, -O-, $-(CH_2CH_2O)_y-$, oxime group, an amino acid residue, a fragment of a polypeptide formed with 2 to 10 amino acids, 4- to 16-membered heterocyclylene, 5- to 16-membered heteroarylene, amino, acyl, -CO-NH- and -NH-CO-; the $C_{1-6}$ alkylene, oxime group, amino, 4- to 16-membered heterocyclylene, 5- to 16-membered heteroarylene, -CO-NH- and -NH-CO- are optionally substituted with one or more groups independently selected from the group consisting of sulfonic acid group, phosphoric acid group, carboxylic acid group, amide group, quaternary ammonium salt, hydroxyl, alkyl, alkoxy, alkoxyalkyl, alkynyl and azido;
each $L_4$ is independently selected from the group consisting of an amino acid residue, a fragment of a polypeptide formed with 2 to 10 amino acids, glycosyl, phenylene, benzyl, benzyloxy, benzyloxycarbonyl, aminobenzyloxycarbonyl, acylbenzyloxycarbonyl, 5- to 16-membered heteroarylene, $C_{1-6}$ alkylene, $-(CH_2CH_2O)_y-$, acyl and amino; the $C_{1-6}$ alkylene and amino are optionally substituted with one or more groups independently selected from the group consisting of sulfonic acid group, phosphoric acid group, carboxylic acid group, amide group, sulfone group, sulfoxide group, quaternary ammonium salt, hydroxyl, alkyl, alkoxy, alkoxyalkyl, alkylaminoalkyl, alkynyl and azido, and the phenylene, benzyl, benzyloxy, benzyloxycarbonyl, aminobenzyloxycarbonyl, acylbenzyloxycarbonyl are optionally substituted with pyranosyl or furanosyl;
each D is independently selected from the group consisting of a fragment of an effector molecule, and the effector molecule is, for example, a cytotoxin, an immunostimulant, a pro-apoptotic agent, a protein degrading agent or a hormone receptor modulator; preferably, the effector molecule is connected to $L_4$ via an amino or hydroxyl thereon;
m is an integer of 1 to 20;
n is an integer of 1 to 20;
x is an integer of 1 to 10;
y is an integer of 1 to 20.

**3.** The compound according to claim 1, wherein LG is selected from the group consisting of $-OR^1$, hydroxyl, halogen (e.g., chlorine), $C_{1-6}$ haloalkyl (e.g., chloromethyl), 5- to 12-membered heteroaryl (e.g., imidazolyl), $R^1$ is selected from the group consisting of $C_{1-6}$ alkylacyl, maleimido, succinimido, sulfosuccinimido and phenyl, and the phenyl is optionally substituted with 1, 2, 3, 4 or 5 groups independently selected from the group consisting of nitro, cyano, sulfonyl, sulfonic acid group, fluorine and chlorine; preferably, LG is selected from the group consisting of $-OR^1$ and imidazolyl, $R^1$ is selected from the group consisting of succinimido, sulfosuccinimido and phenyl, and the phenyl is optionally substituted with 1, 2, 3, 4 or 5 groups independently selected from the group consisting of nitro, sulfonic acid group and fluorine; preferably, LG is pentafluorophenoxy;

preferably, each $L_1$ is independently selected from the group consisting of single bond, $C_{1-6}$ alkylene, $-(CH_2CH_2O)_y-$, $C_{2-6}$ alkenylene, an amino acid residue, a fragment of a polypeptide formed with 2 to 10 amino acids, 4- to 16-membered heterocyclylene, 5- to 16-membered heteroarylene, phenylene, amino, -CO-NH- and -NH-CO-, wherein the $C_{1-6}$ alkylene, amino, 4-to 16-membered heterocyclylene, 5- to 16-membered heteroarylene, phenylene, -CO-NH- and -NH-CO- are optionally substituted with one or more groups independently selected from the group consisting of sulfonic acid group, phosphoric acid group, carboxylic acid group, amide group, quaternary ammonium salt, hydroxyl, $C_{1-6}$ alkyl, $C_{1-6}$ alkoxy, $C_{2-6}$ alkoxyalkyl, $C_{2-6}$ alkynyl and azido; preferably, each $L_1$ is independently selected from the group consisting of single bond, $C_{1-6}$ alkylene, $-(CH_2CH_2O)_y-$, phenyl, -CO-NH- and -NH-CO -, y is an integer of 1 to 10, such as an integer of 1 to 5, and m is an integer of 1 to 5; preferably, each $L_1$ is independently selected from the group consisting of single bond and $C_{1-4}$ alkylene, and m is 1 or 2;
preferably, each $L_2$ is independently selected from the group consisting of $C_{2-6}$ alkynyl, methyltetrazinyl, trans-cyclooctenyl, benzoazacyclooctynyl, (IR,8S,9S)-bicyclo[6.1.0]non-4-yn-9-yl, azido and $C_{1-6}$ alkylacyl, and m is an integer of 1 to 5; preferably, each $L_2$ is independently selected from the group consisting of single bond and $C_{2-6}$ alkynyl, and m is 1.

**4.** The compound according to claim 1, wherein,

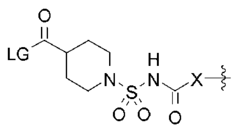

is selected from the group consisting of the following structures:

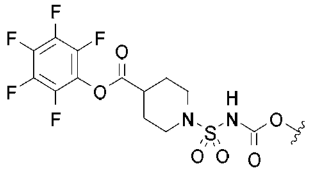 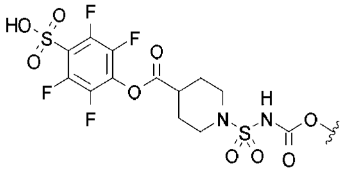 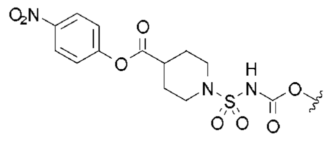

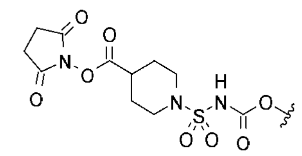 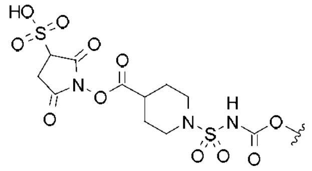 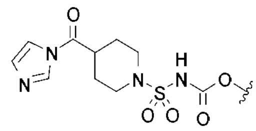

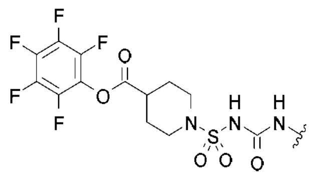 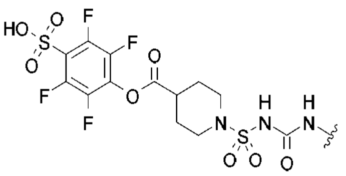 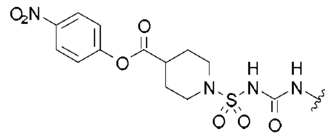

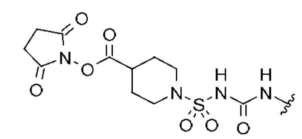 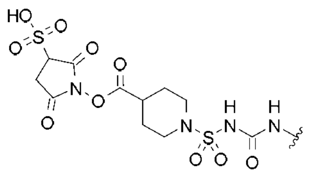 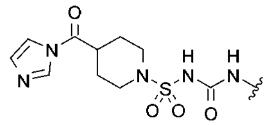

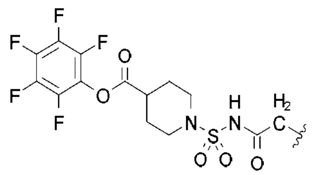 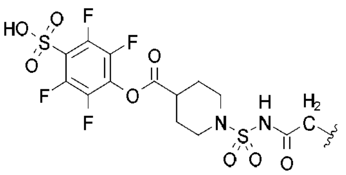 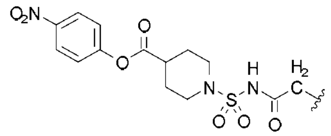

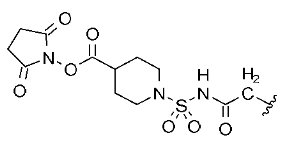 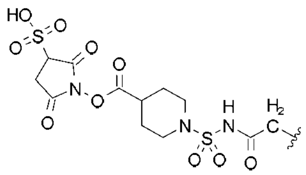 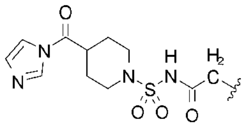 ;

preferably

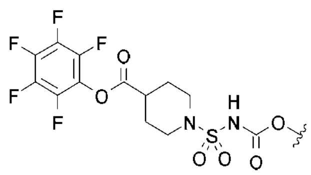 .

5. The compound of Formula I according to claim 1, which is selected from the group consisting of the following structures:

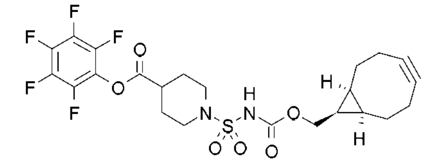

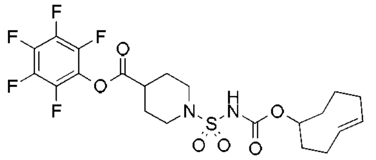

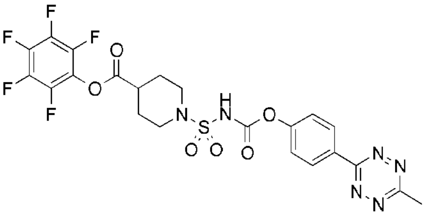

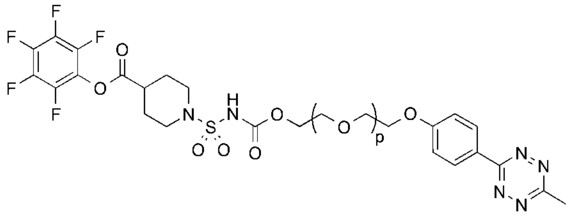

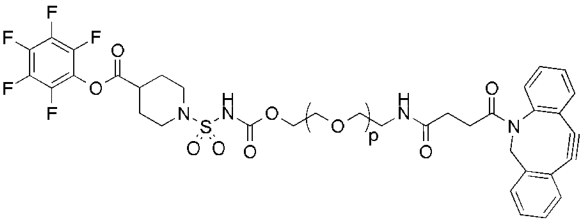

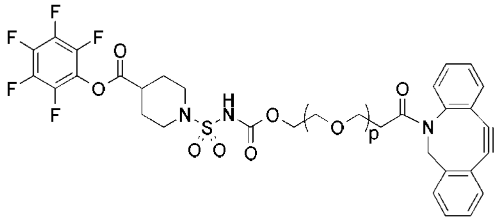

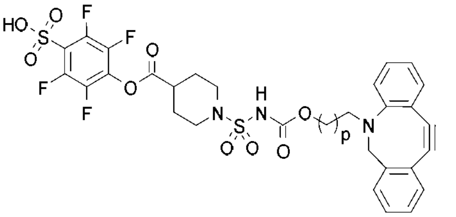

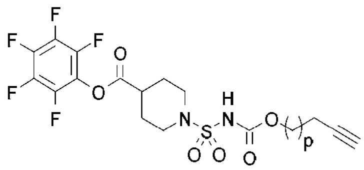

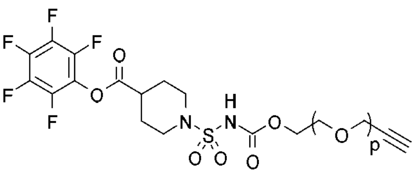

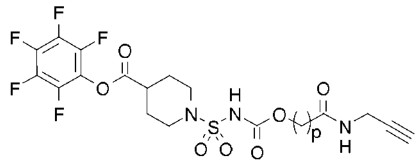

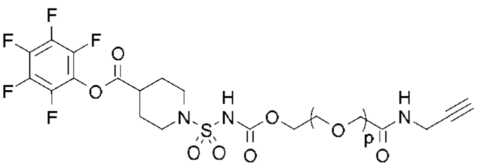

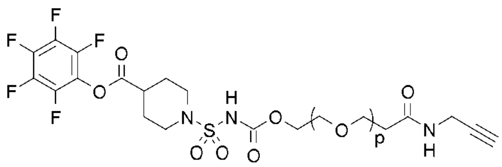

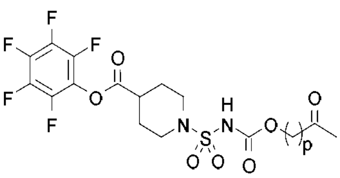

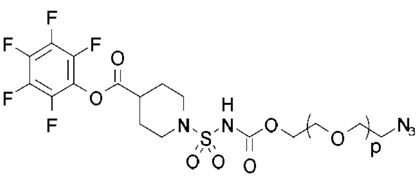

;

p is 0 or an integer of 1 to 20.

**6.** The compound according to claim 2, wherein LG is selected from the group consisting of -OR$^1$, hydroxyl, halogen (e.g., chlorine), $C_{1-6}$ haloalkyl (e.g., chloromethyl), 5- to 12-membered heteroaryl (e.g., imidazolyl), R$^1$ is selected from the group consisting of $C_{1-6}$ alkylacyl, maleimido, succinimido, sulfosuccinimido and phenyl, and the phenyl is optionally substituted with 1, 2, 3, 4 or 5 groups independently selected from the group consisting of nitro, cyano,

sulfone group, sulfonic acid group, fluorine and chlorine; preferably, LG is selected from the group consisting of $-OR^1$ and imidazolyl, $R^1$ is selected from the group consisting of succinimido, sulfosuccinimido and phenyl, and the phenyl is optionally substituted with 1, 2, 3, 4 or 5 groups independently selected from the group consisting of nitro, sulfonic acid group and fluorine; preferably, LG is pentafluorophenoxy;

preferably, X is selected from the group consisting of -O-, $-NR^2-$ and $-CHR^3-$; $R^2$ and $R^3$ are each independently selected from the group consisting of hydrogen and $C_{1-6}$ alkyl; the $C_{1-6}$ alkyl is optionally substituted with one or more groups independently selected from the group consisting of amino, $C_{1-6}$ alkylamino, 5- to 12-membered nitrogen-containing heterocyclyl, sulfonic acid group, carboxylic acid group, quaternary ammonium salt, hydroxyl and $C_{1-6}$ alkoxy; X is selected from the group consisting of -O-, -NH- and $-CH_2-$; preferably, X is -O-;

preferably, each $L_3$ is independently selected from the group consisting of single bond, $C_{1-6}$ alkylene, -O-, $-(CH_2CH_2O)_y-$, oxime group, an amino acid residue, a fragment of a polypeptide formed with 2 to 10 amino acids, 4- to 16-membered heterocyclylene, 5- to 16-membered heteroarylene, amino, acyl, -CO-NH- and -NH-CO-; wherein the $C_{1-6}$ alkylene, amino, 4- to 16-membered heterocyclylene, 5- to 16-membered heteroarylene, -CO-NH- and -NH-CO- are optionally substituted with one or more groups independently selected from the group consisting of sulfonic acid group, phosphoric acid group, carboxylic acid group, amide group, quaternary ammonium salt, hydroxyl, $C_{1-6}$ alkyl, $C_{1-6}$ alkoxy, $C_{2-6}$ alkoxyalkyl, $C_{2-6}$ alkynyl and azido, y is an integer of 1 to 20, and m is an integer of 1 to 20; preferably, each $L_3$ is independently selected from the group consisting of $C_{1-6}$ alkylene, -O-, $-(CH_2CH_2O)_y-$, oxime group, 5- to 16-membered heteroarylene, acyl, -CO-NH- and -NH-CO-, wherein the $C_{1-6}$ alkylene, oxime group, 5- to 16-membered heteroarylene, acyl, -CO-NH- and -NH-CO- are optionally substituted with one or more groups independently selected from the group consisting of sulfonic acid group, phosphoric acid group, carboxylic acid group, amide group, quaternary ammonium salt, hydroxyl, $C_{1-6}$ alkyl, $C_{1-6}$ alkoxy, $C_{2-6}$ alkoxyalkyl, $C_{2-6}$ alkynyl and azido, y is an integer of 1 to 20, and m is an integer of 1 to 20; preferably, each $L_3$ is independently selected from the group consisting of $C_{1-6}$ alkylene, oxime group, 5- to 16-membered heteroarylene, acyl, -CO-NH- and -NH-CO-, wherein the $C_{1-6}$ alkylene, oxime group, 5- to 16-membered heteroarylene, acyl, -CO-NH- and -NH-CO- are optionally substituted with one or more groups independently selected from the group consisting of sulfonic acid group, phosphoric acid group, carboxylic acid group, amide group, quaternary ammonium salt, hydroxyl, $C_{1-6}$ alkyl, $C_{1-6}$ alkoxy, $C_{2-6}$ alkoxyalkyl, $C_{2-6}$ alkynyl and azido, y is an integer of 1 to 20, and m is an integer of 1 to 20; preferably, each $L_3$ is independently selected from the group consisting of $C_{1-6}$ alkylene, -O-, $-(CH_2CH_2O)_y-$, oxime group, 5- to 16-membered heteroarylene, acyl, -CO-NH- and -NH-CO-, y is an integer of 1 to 10, and m is an integer of 1 to 15;

each $L_4$ is independently selected from the group consisting of an amino acid residue, a fragment of a polypeptide formed with 2 to 10 amino acids, glycosyl, phenylene, benzyl, benzyloxy, benzyloxycarbonyl, aminobenzyloxycarbonyl, acylbenzyloxycarbonyl, 5- to 6-membered heteroarylene, $C_{1-6}$ alkylene, $-(CH_2CH_2O)_y-$, acyl and amino; wherein the $C_{1-6}$ alkylene and amino are optionally substituted with one or more groups independently selected from the group consisting of sulfonic acid group, phosphoric acid group, carboxylic acid group, amide group, sulfone group, sulfoxide group, quaternary ammonium salt, hydroxyl, $C_{1-6}$ alkyl, $C_{1-6}$ alkoxy, $C_{2-6}$ alkoxyalkyl, $C_{1-6}$ alkylaminoalkyl, $C_{2-6}$ alkynyl and azido; wherein the phenylene, benzyl, benzyloxy, benzyloxycarbonyl, aminobenzyloxycarbonyl, acylbenzyloxycarbonyl are optionally substituted with glucuronic acid, galacturonic acid, glucose, galactose or mannose; preferably, each $L_4$ is independently selected from the group consisting of an amino acid residue, a fragment of a polypeptide formed with 2 to 10 amino acids, aminobenzyloxycarbonyl, $C_{1-6}$ alkylene and amino, wherein the $C_{1-6}$ alkylene and amino are optionally substituted with one or more groups independently selected from the group consisting of sulfonic acid group, phosphoric acid group, carboxylic acid group, amide group, sulfone group, sulfoxide group, quaternary ammonium salt, hydroxyl, alkyl, alkoxy, alkoxyalkyl, alkylaminoalkyl, alkynyl and azido, wherein the aminobenzyloxycarbonyl is optionally substituted with pyranosyl or furanosyl; preferably, each $L_4$ is independently selected from the group consisting of an amino acid residue, a fragment of a polypeptide formed with 2 to 10 amino acids, aminobenzyloxycarbonyl, $C_{1-6}$ alkylene and amino, wherein the $C_{1-6}$ alkylene and amino are optionally substituted with one or more groups independently selected from the group consisting of sulfonic acid group, phosphoric acid group, carboxylic acid group, amide group, sulfone group, sulfoxide group, quaternary ammonium salt, hydroxyl, $C_{1-6}$ alkyl, $C_{1-6}$ alkoxy, $C_{2-6}$ alkoxyalkyl, $C_{2-6}$ alkylaminoalkyl, $C_{2-6}$ alkynyl and azido, wherein the aminobenzyloxycarbonyl is optionally substituted with glucuronic acid, galacturonic acid, glucose, galactose or mannose;

preferably, the amino acid is selected from the group consisting of L-type natural amino acid, D-type non-natural amino acid, and an analog or derivative thereof; preferably, the amino acid is selected from the group consisting of Ala, Arg, Asn, Asp, Cit, Cys, Gln, Glu, Gly, His, Ile, Leu, Lys, Met, Phe, Pro, Ser, Thr, Trp, Tyr, Val, $Lys(COCH_2CH_2(OCH_2CH_2)_yOCH_3)$, y is an integer of 1 to 20; preferably, the polypeptide formed with 2 to 10 amino acids is selected from the group consisting of Ala-Ala, Ala-Lys, Ala-Lys(Ac), Ala-Pro, Gly-Glu, Gly-Gly, Phe-Lys, Phe-Lys(Ac), Val-Ala, Val-Lys, Val-Lys(Ac), Val-Cit, Val-Arg, Ala-Ala-Ala, Ala-Ala-Asn, Ala-Ala-Gly, Leu-Ala-Glu, Gly-Gly-Arg, Gly-Glu-Gly, Gly-Gly-Gly, Gly-Ser-Lys, Gly-Ala-Ala, Glu-Val-Ala, Glu-Val-Cit,Glu-Val-Arg, Ser-Ala-Pro, Val-Leu-Lys, Val-Lys-Ala, Val-Lys-Gly, Gly-Gly-Phe-Gly, Gly-Gly-Val-Ala, Gly-Phe-Leu-Gly, Glu-Ala-Ala-Ala, and Gly-Gly-Gly-Gly-Gly.

**7.** The compound according to claim 2 or 6, wherein

$$-X-(L_3)_m-$$

is selected from the group consisting of the following structures:

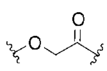
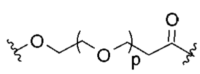
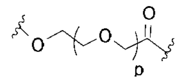
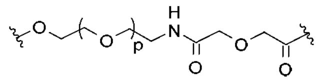
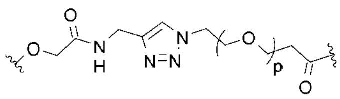
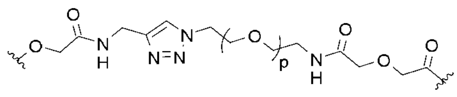
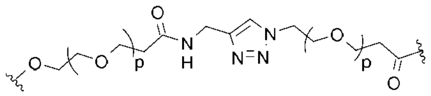
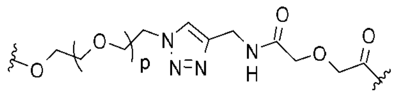
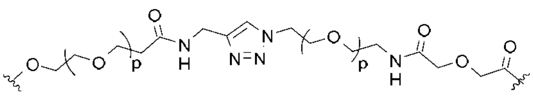
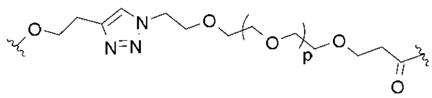
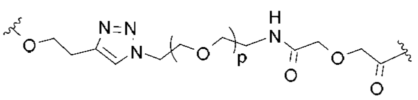
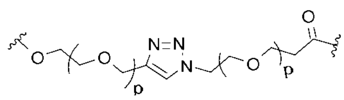
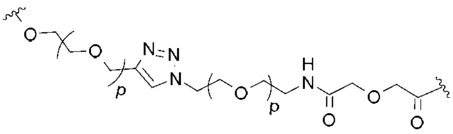
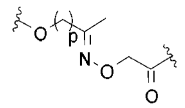
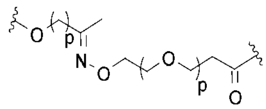
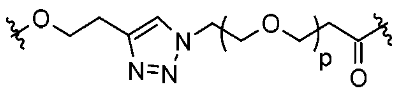 ;

p is 0 or an integer of 1 to 20.

**8.** The compound according to claim 2 or 6, wherein

$$-(L_4)_n-$$

is selected from the group consisting of the following structures:

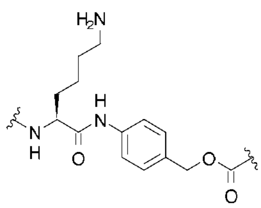
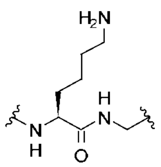
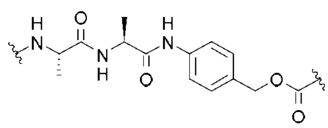
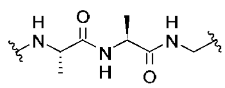

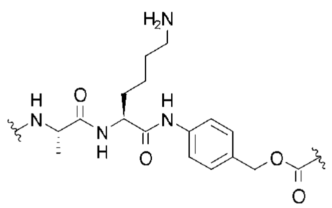
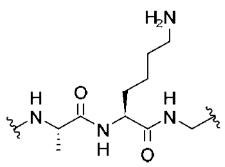
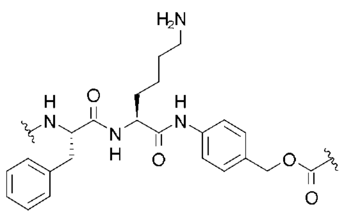
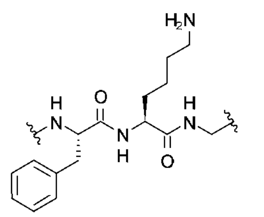
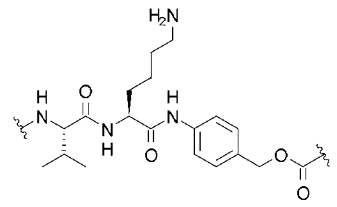
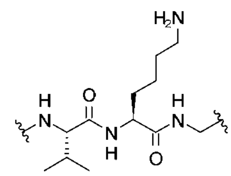
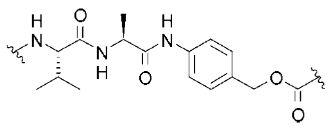
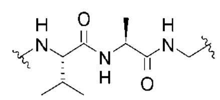
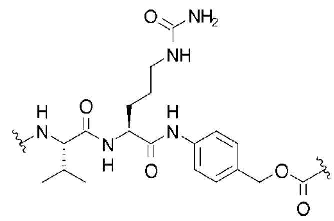
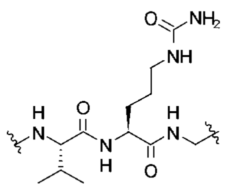
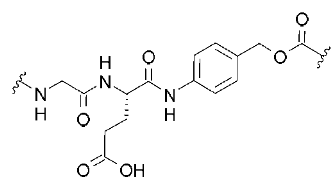
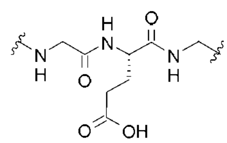
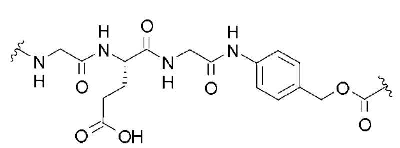
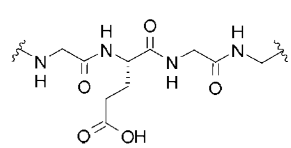
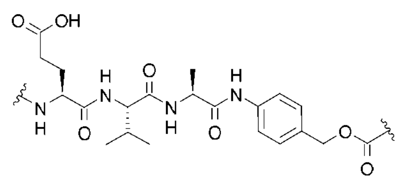
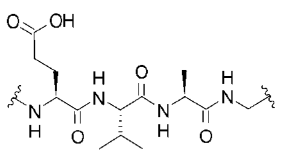
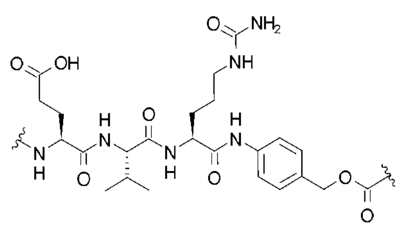
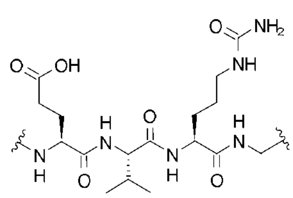
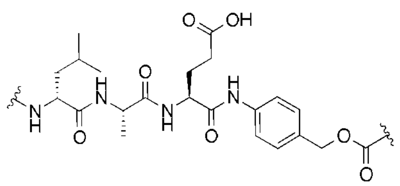
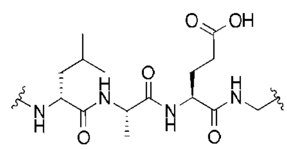
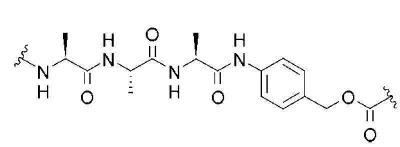

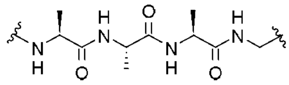
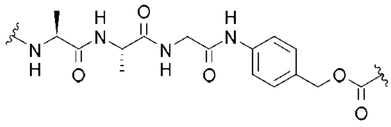
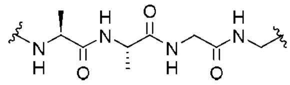

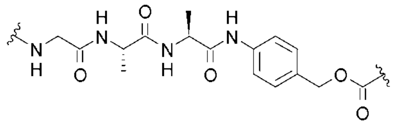
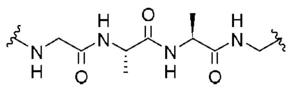
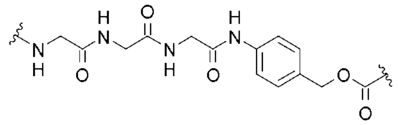

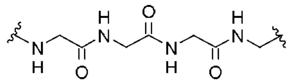
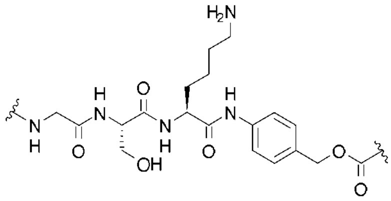
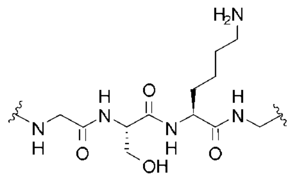

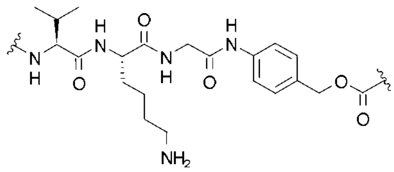
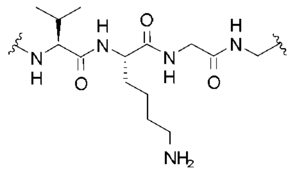

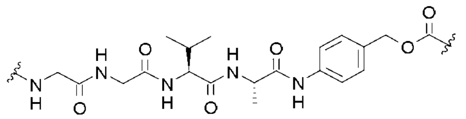
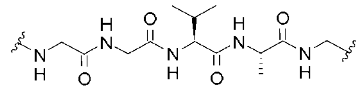

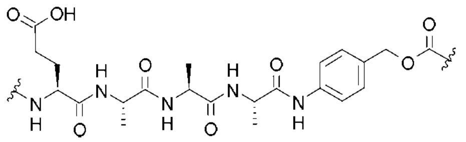
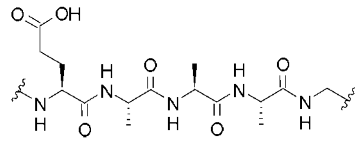

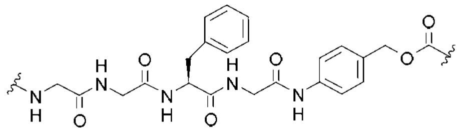
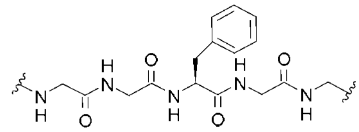

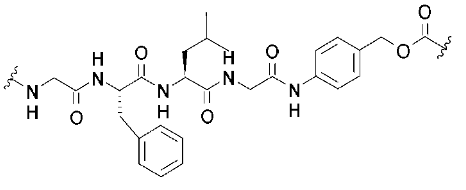
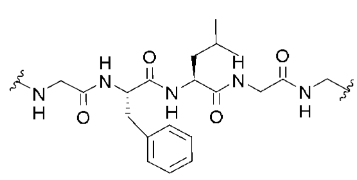
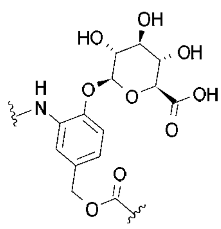

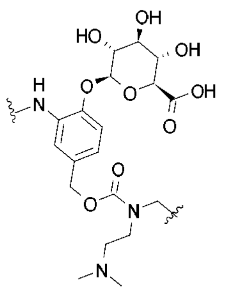 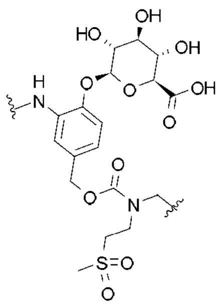.

**9.** The compound according to any one of claims 2, 6 to 8, wherein

$$-X-(L_3)_m-(L_4)_n-$$

is selected from the group consisting of the following structures:

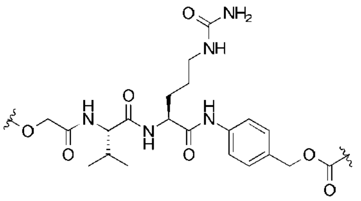 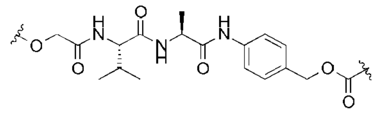

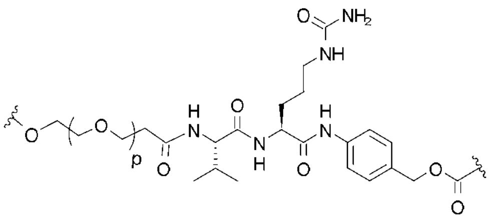 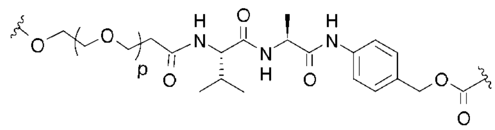

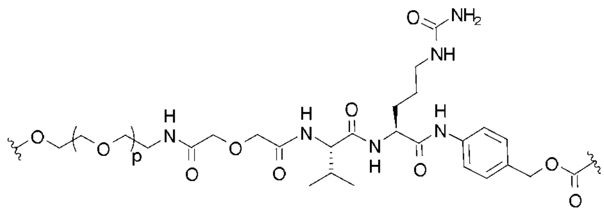

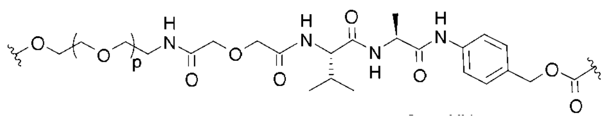

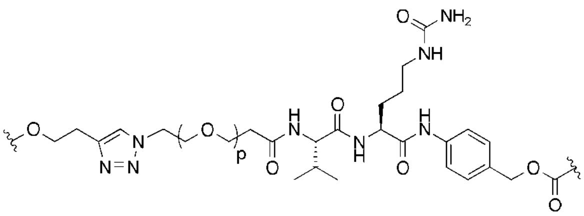

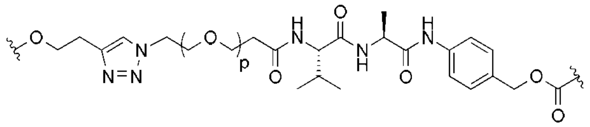

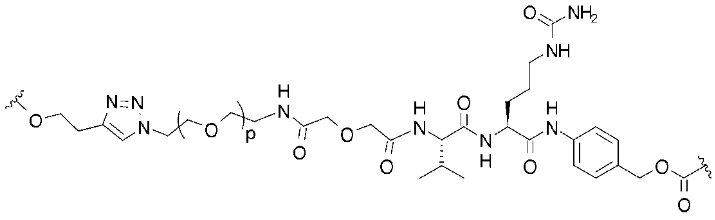

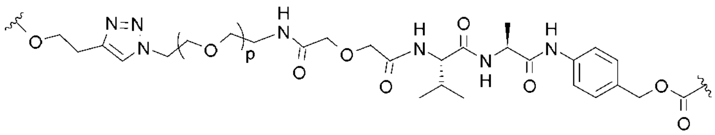

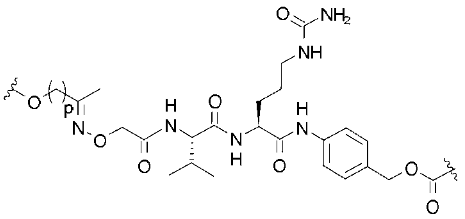

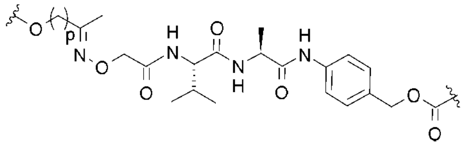

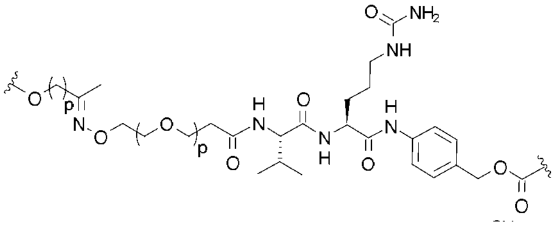

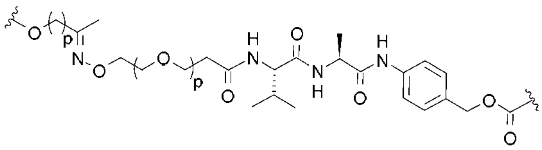

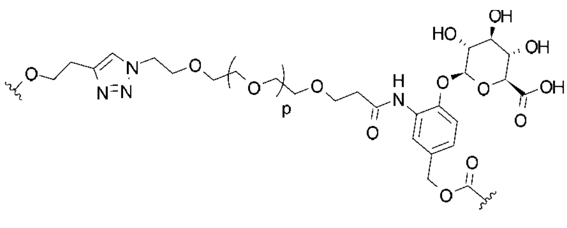

;

p is 0 or an integer of 1 to 20.

**10.** The compound according to claim 2, wherein the effector molecule is selected from the group consisting of microtubulin inhibitors, such as auristatins, maytansinoids; DNA intercalators, such as pyrrolobenzodiazepine (PBD); DNA topoisomerase inhibitors, such as topoisomerase I inhibitors (e.g., camptothecin, hydroxycamptothecin, 9-aminocamptothecin, SN-38, irinotecan, topotecan, belotecan, rubitecan, exatecan) or topoisomerase II inhibitors (e.g., doxorubicin, PNU-159682, duocarmycin, daunorubicin, mitoxantrone, podophyllotoxin or etoposide); RNA polymerase inhibitors, such as α-amanitin; and pharmaceutically acceptable salts, esters and analogs of the above agents;

preferably, the effector molecule is selected from the group consisting of topoisomerase I inhibitors (e.g., camptothecin, hydroxycamptothecin, 9-aminocamptothecin, SN-38, irinotecan, topotecan, belotecan, rubitecan, exatecan), MMAE, and MMAE derivatives;
preferably, the effector molecule is selected from:

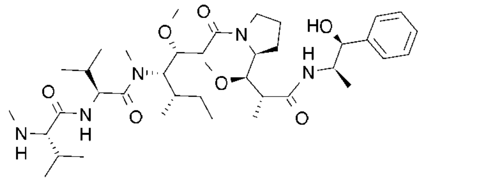,
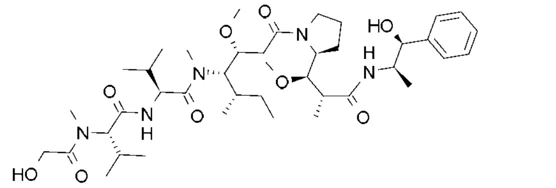,

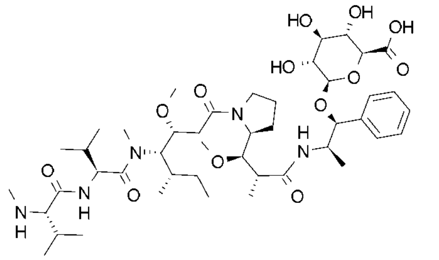,
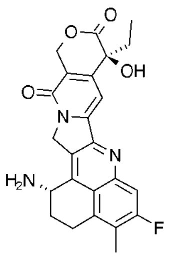,
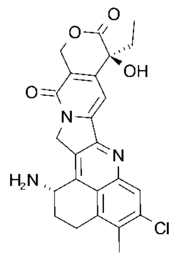,
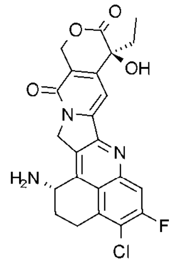,

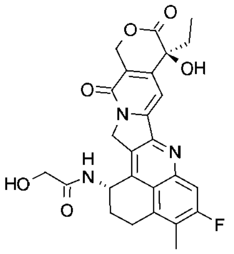,
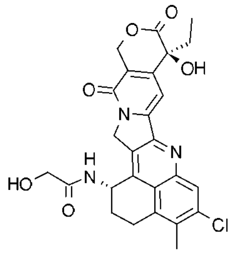,
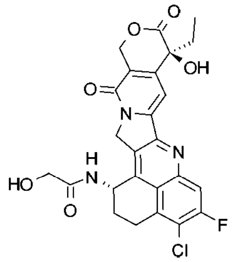,
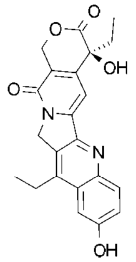,
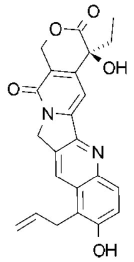,

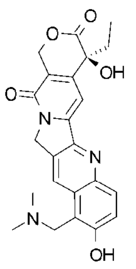,
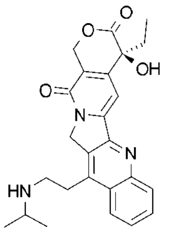,
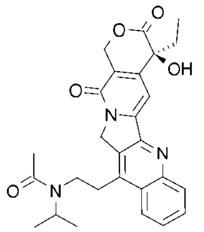,
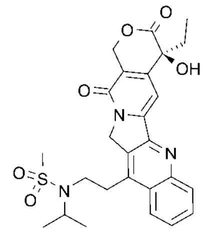,
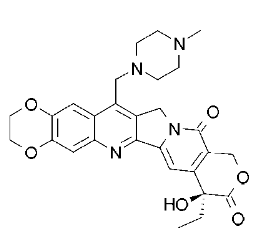,

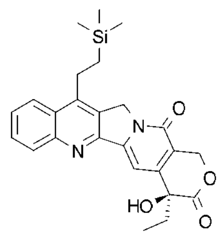,
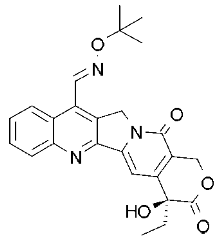;

preferably, D is selected from:

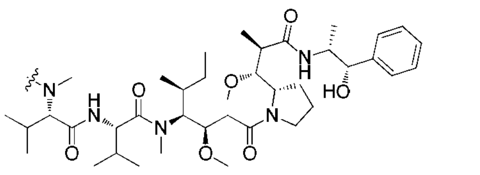,
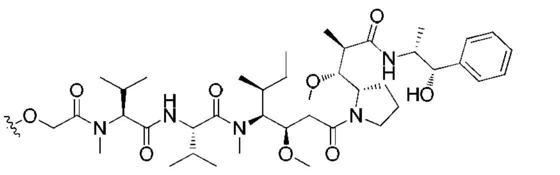,

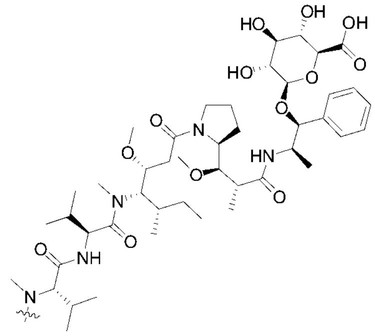 , 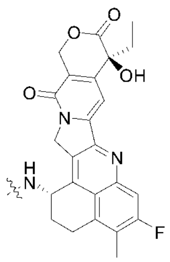 , 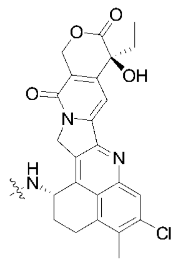 , 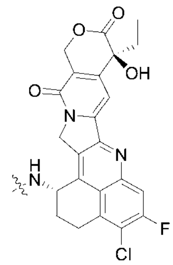 ,

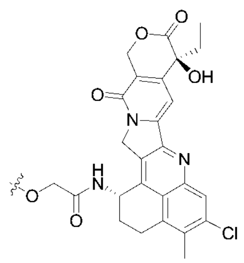 , 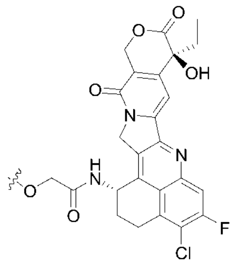 , 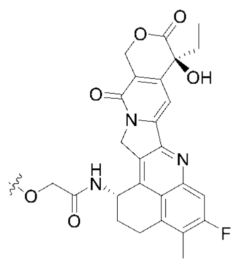 , 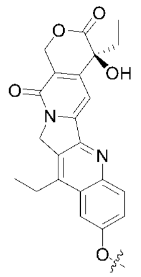 , 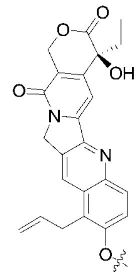 ,

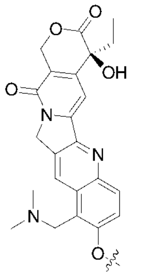 , 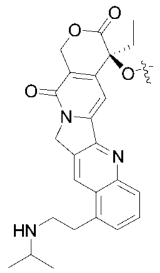 , 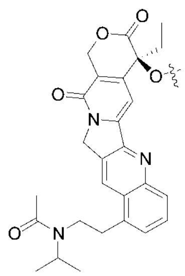 , 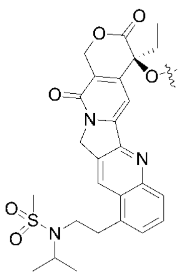 , 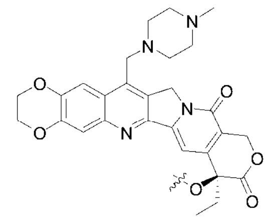 ,

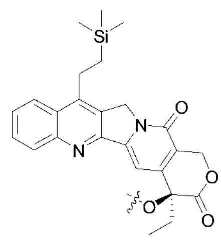 , 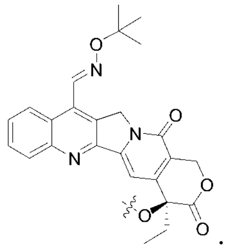 .

11. The compound of Formula II according to claim 2, which is selected from the group consisting of the following structures:

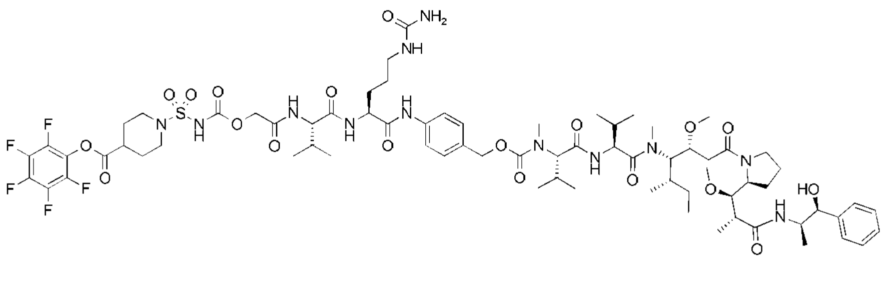

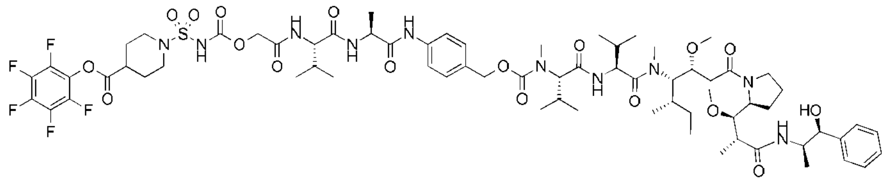

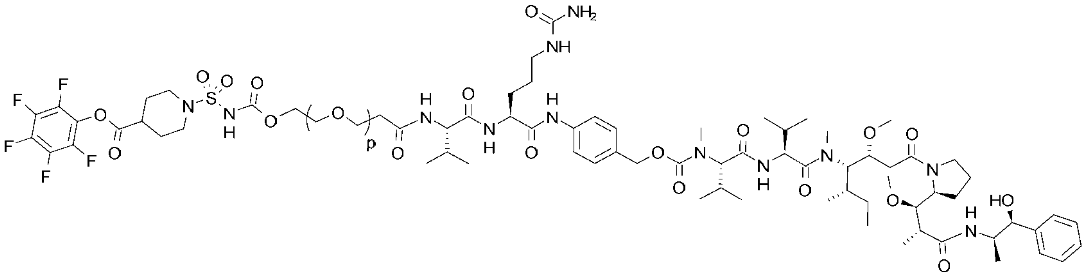

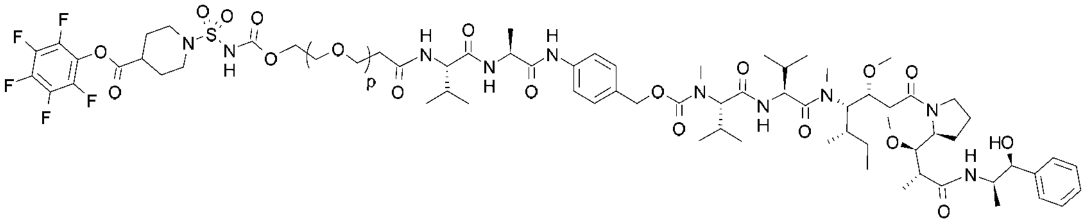

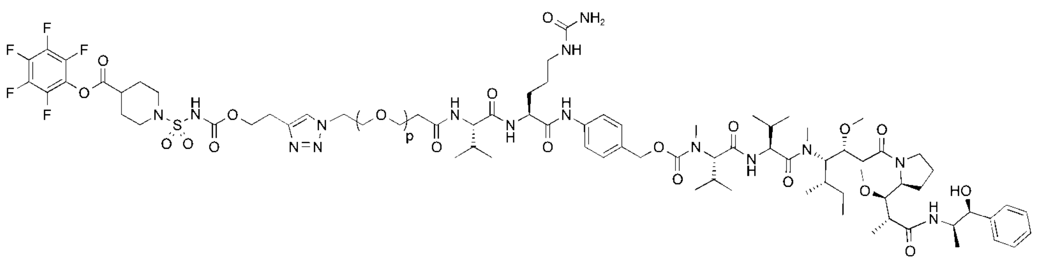

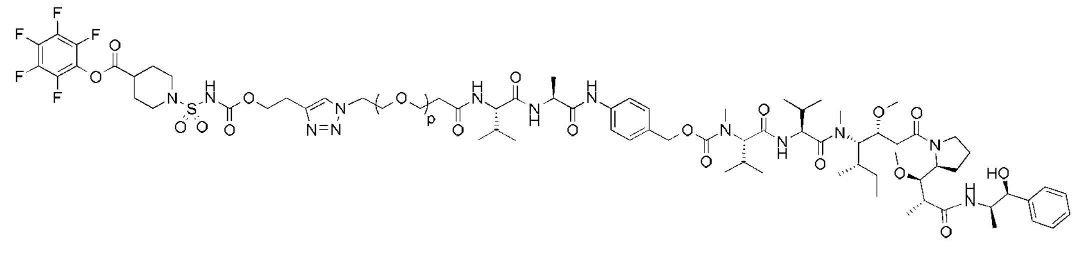

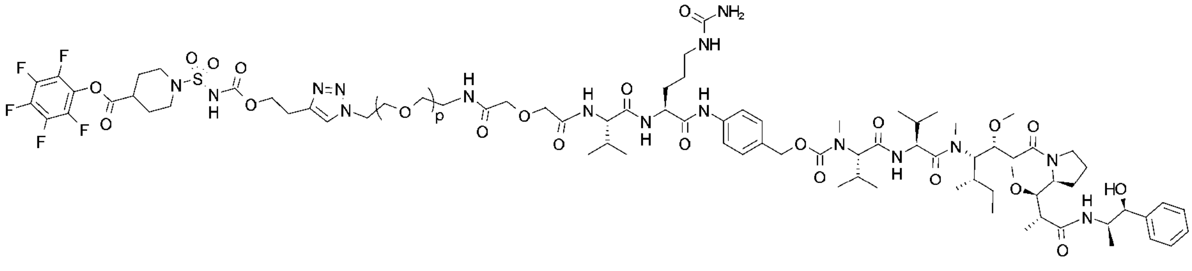

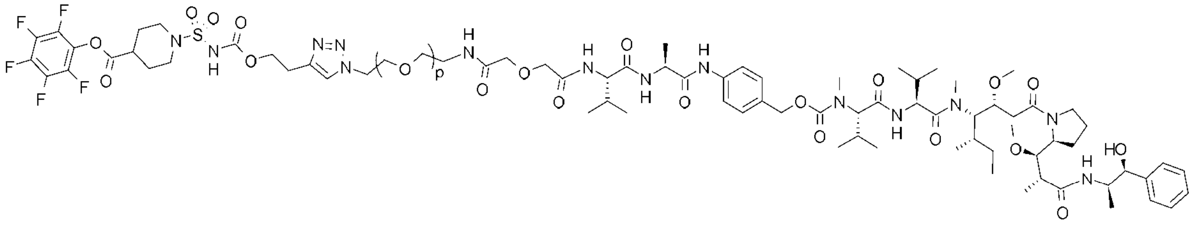

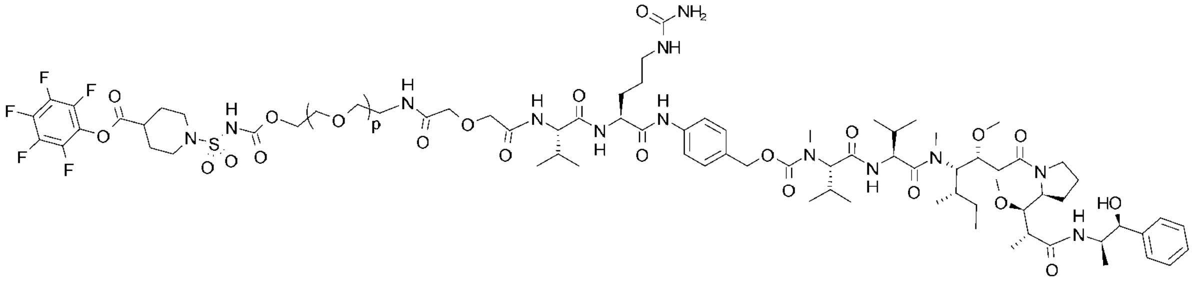

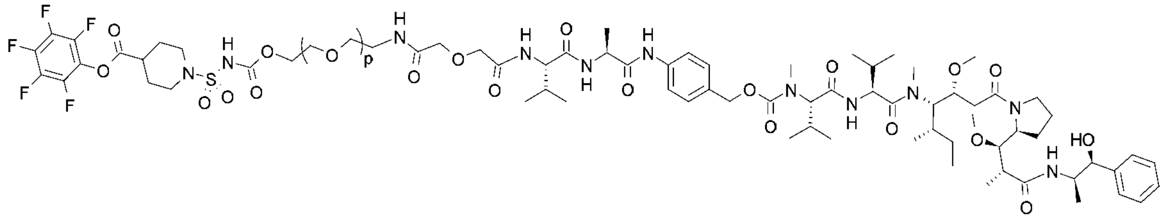

;

p is 0 or an integer of 1 to 20.

**12.** The compound of Formula II according to any one of claims 2, 6 to 11, which is represented by the following structure:

DL-1:

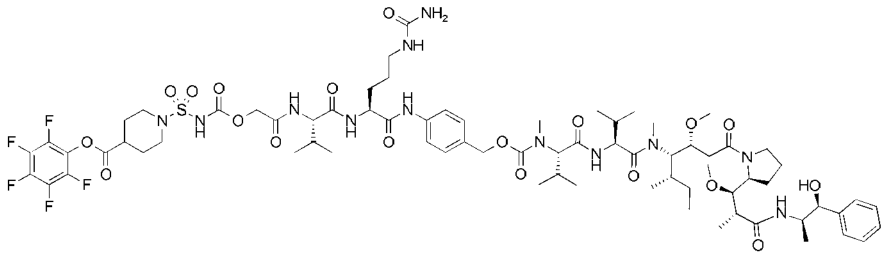

DL-2:

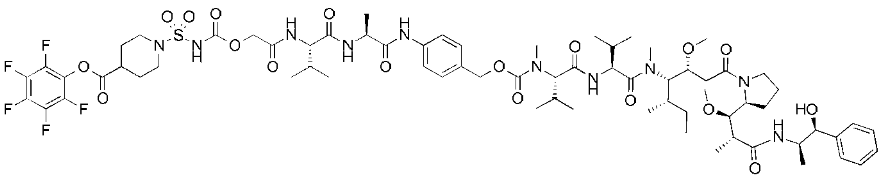

.

**13.** A bioactive conjugate, which has a structure represented by Formula III:

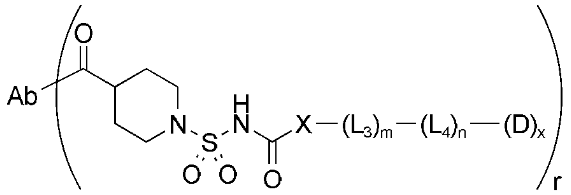

Formula III

wherein:

Ab is a targeting moiety (e.g., a small molecule ligand, a protein (e.g., an antibody), a polypeptide, a non-protein agent (e.g., a sugar, RNA, or DNA)); r is selected from the group consisting of 1 to 10; preferably, Ab is trastuzumab or pertuzumab; preferably, Ab is trastuzumab;
X, $L_3$, $L_4$, D, m, n, and x are defined in any one of claims 2 and 6 to 10.

**14.** The bioactive conjugate according to claim 13, which is selected from:

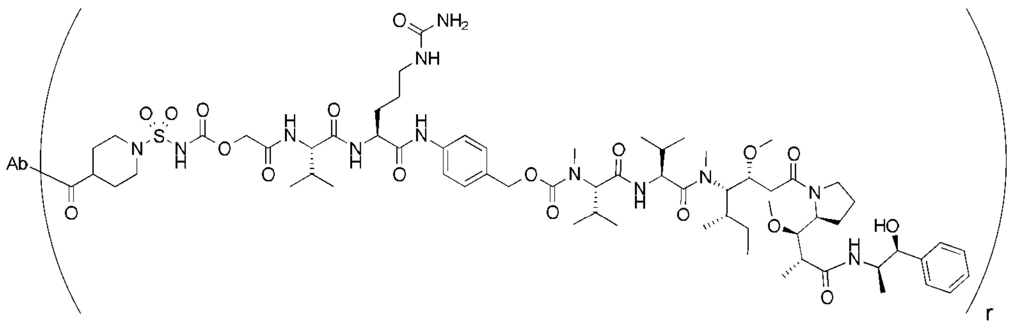

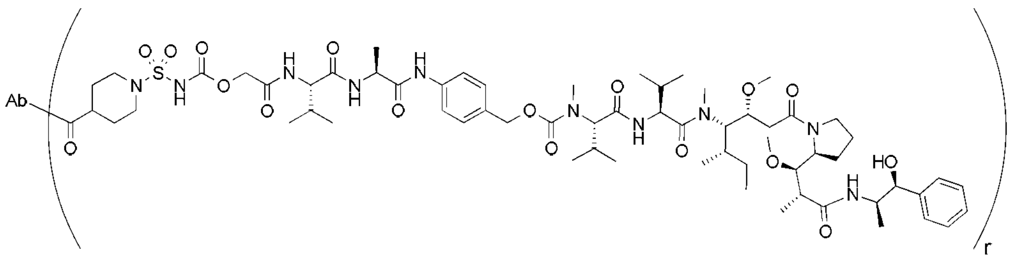

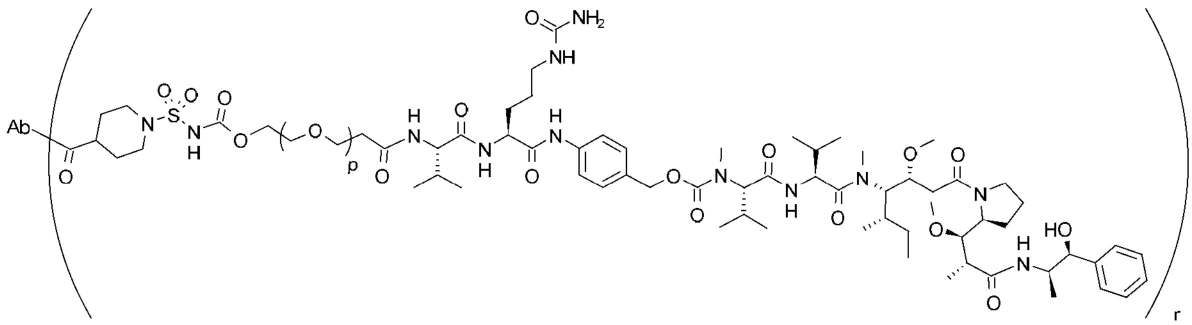

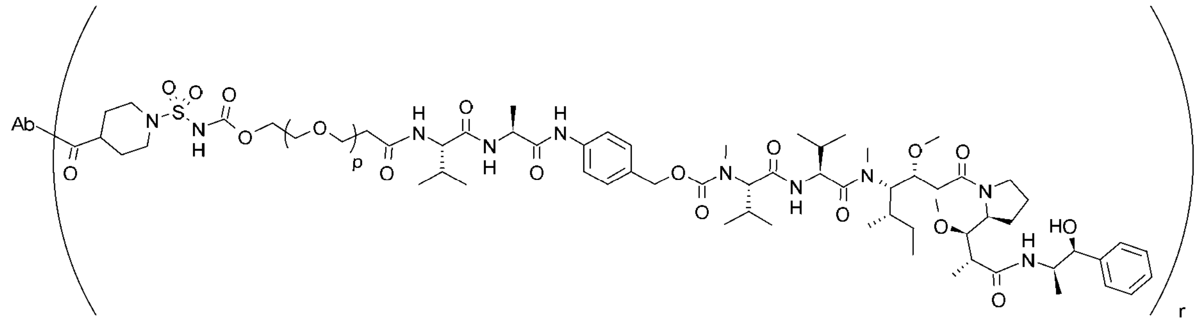

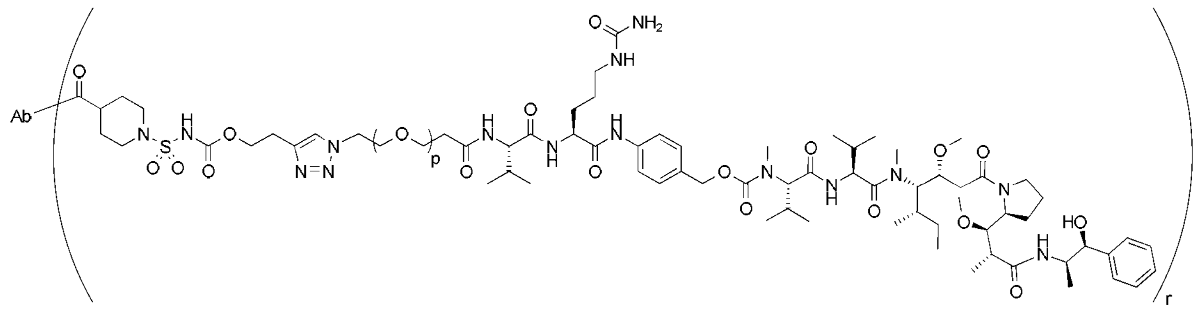

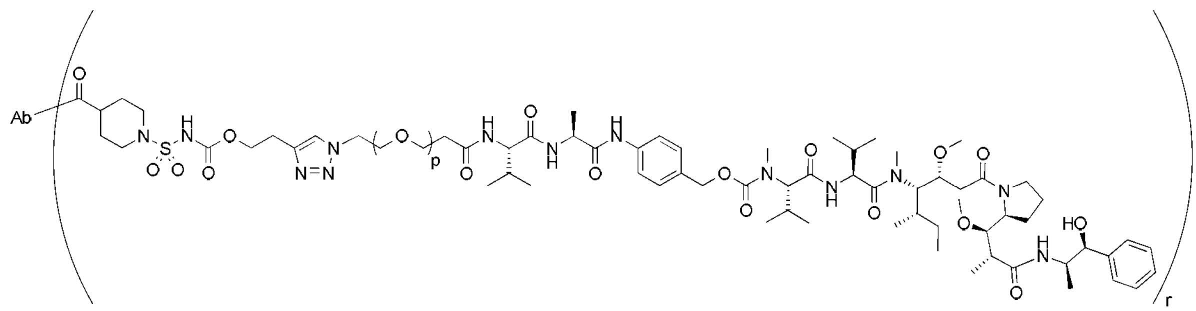

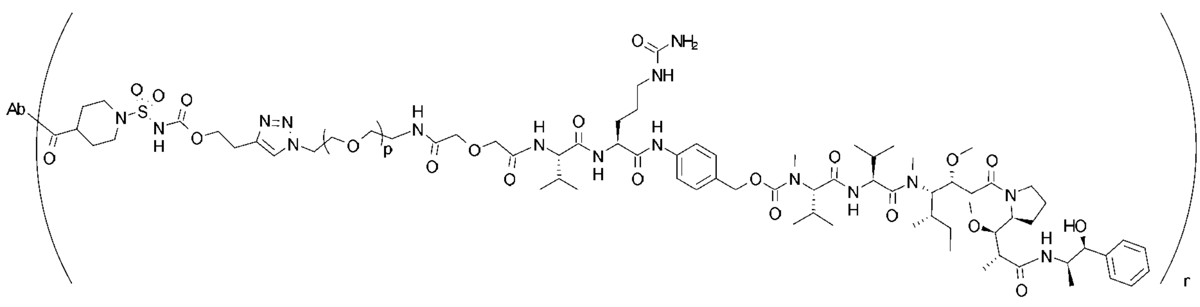

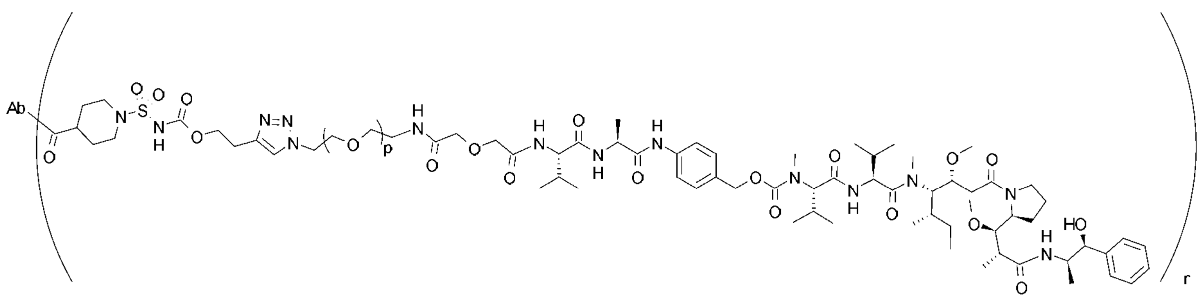

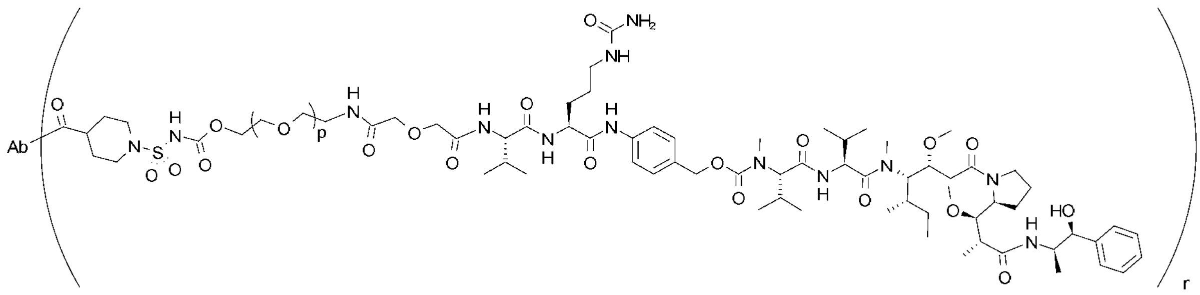

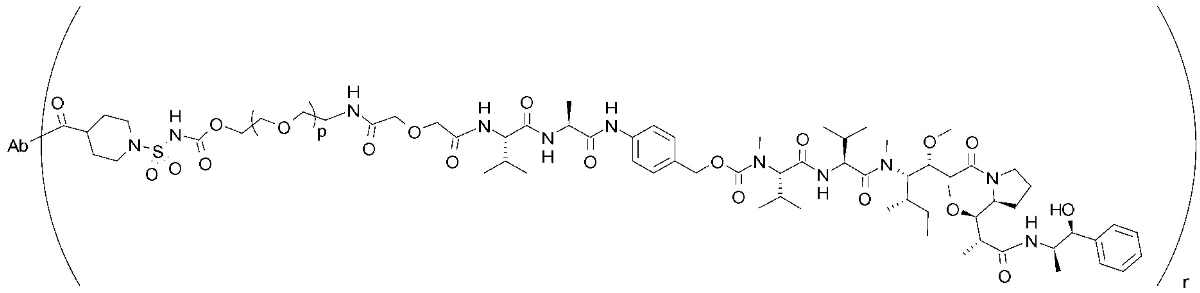

r is 1 to 10, preferably 1 to 3, more preferably about 2;
p is 0 or an integer of 1 to 20, preferably an integer of 0 or 1 to 10;
preferably, Ab is an antibody, and the light chain of the antibody is connected to the remaining portion of the compound represented by Formula III via an amide bond formed between the terminal amino group of lysine and the remaining portion of the conjugate represented by Formula III.

**15.** The bioactive conjugate according to claim 13 or 14, which has the following structure:

Trastuzumab-DL-1:

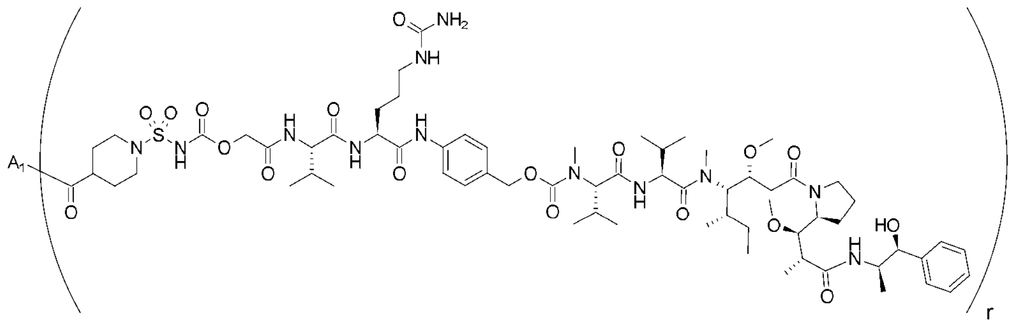

Trastuzumab-DL-2:

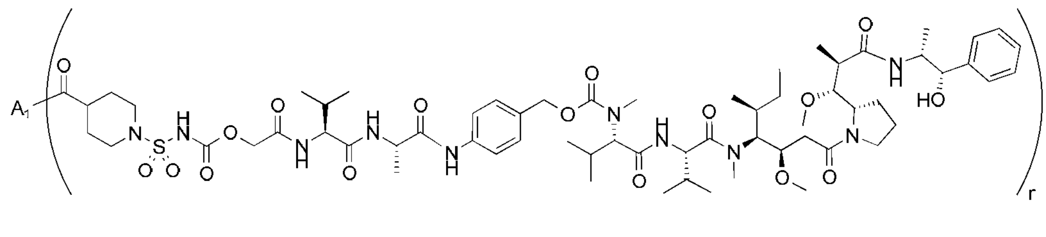

wherein, $A_1$ is trastuzumab, r is 1 to 10, preferably 1 to 3, and more preferably about 2; preferably, trastuzumab is connected to the other portion of the bioactive conjugate through the lysine residue on trastuzumab.

**16.** A composition, wherein the composition comprises one or more of the bioactive conjugate according to any one of claims 13 to 15;
preferably, the DAR of the composition is about 1 to 10, preferably about 1 to 5, and more preferably about 2.0 to 2.5,

such as 2.0, 2.1, 2.2, 2.3, 2.4 or 2.5.

**17.** A pharmaceutical composition, which comprises the bioactive conjugate according to any one of claims 13 to 15, and one or more pharmaceutically acceptable carriers.

**18.** A kit product, which comprises the bioactive conjugate according to any one of claims 13 to 15, the composition according to claim 16, or the pharmaceutical composition according to claim 17, and optional medication instructions.

**19.** Use of the bioactive conjugate according to any one of claims 13 to 15, the composition according to claim 16, or the pharmaceutical composition according to claim 17 in the manufacture of a medicament for preventing or treating a tumor or an autoimmune disease.

**20.** The bioactive conjugate according to any one of claims 13 to 15, the composition according to claim 16, or the pharmaceutical composition according to claim 17, for use in preventing or treating a tumor or an autoimmune disease.

**21.** A method for preventing or treating a tumor disease, comprising administering an effective amount of the bioactive conjugate according to any one of claims 13 to 15, the composition according to claim 16, or the pharmaceutical composition according to claim 17 to a subject in need thereof.

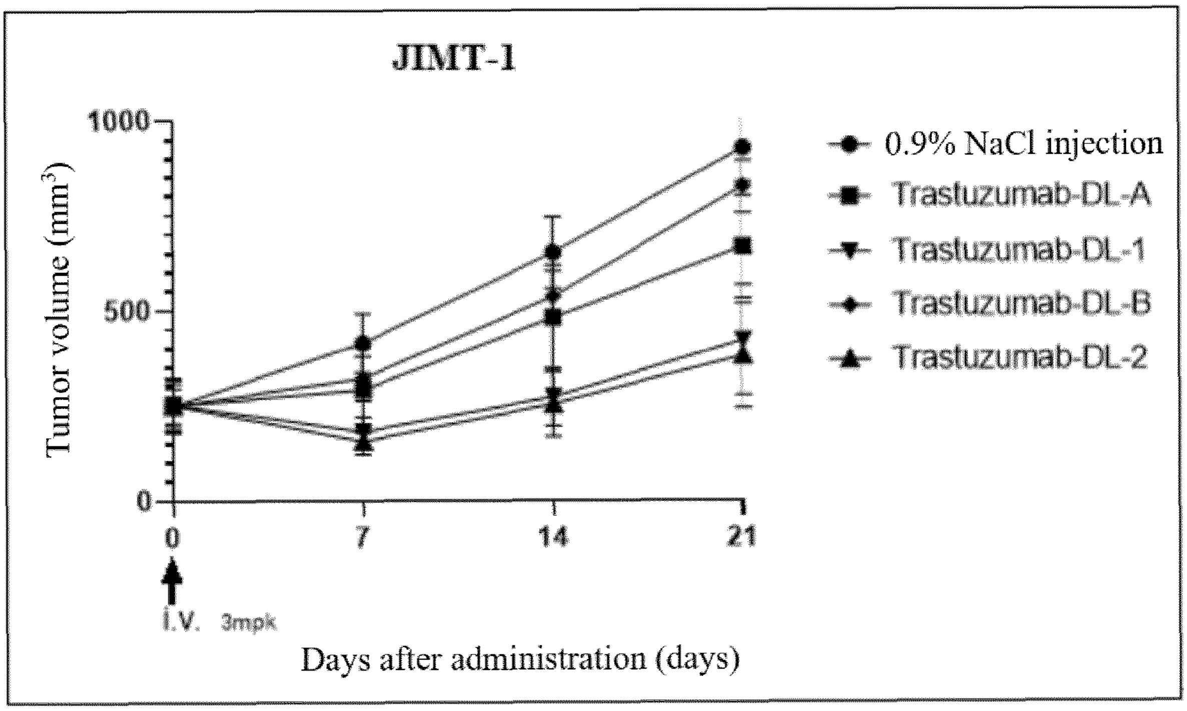


**Figure 1**

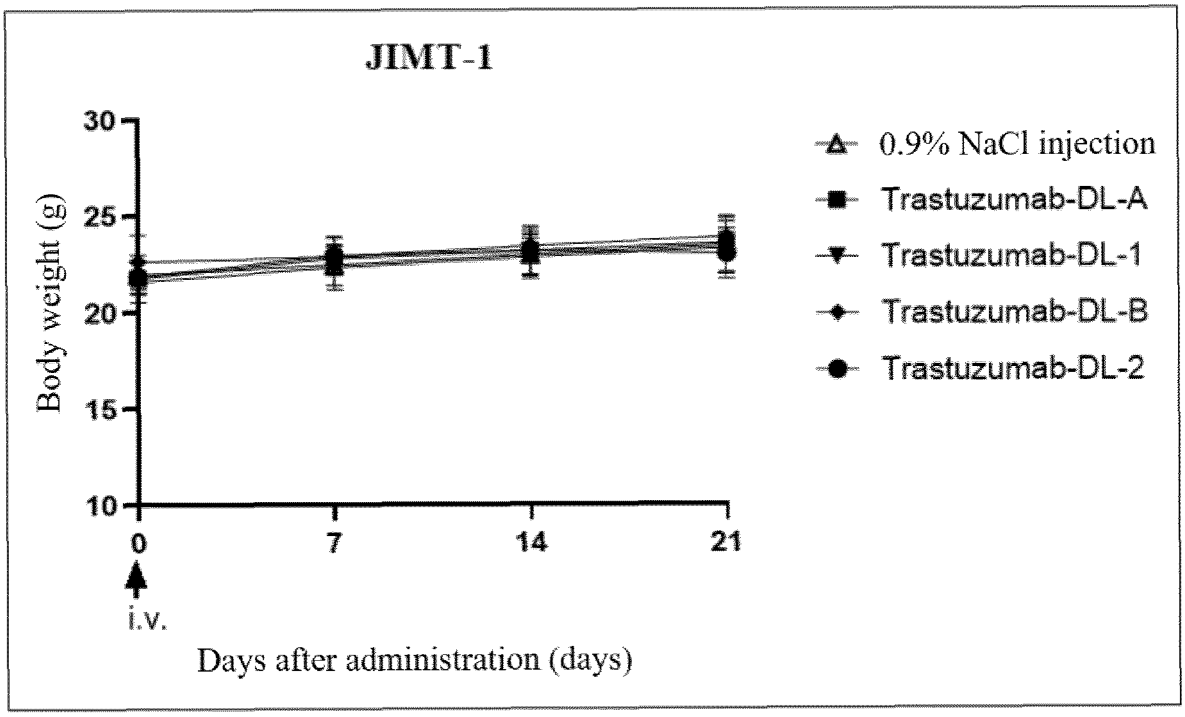


**Figure 2**

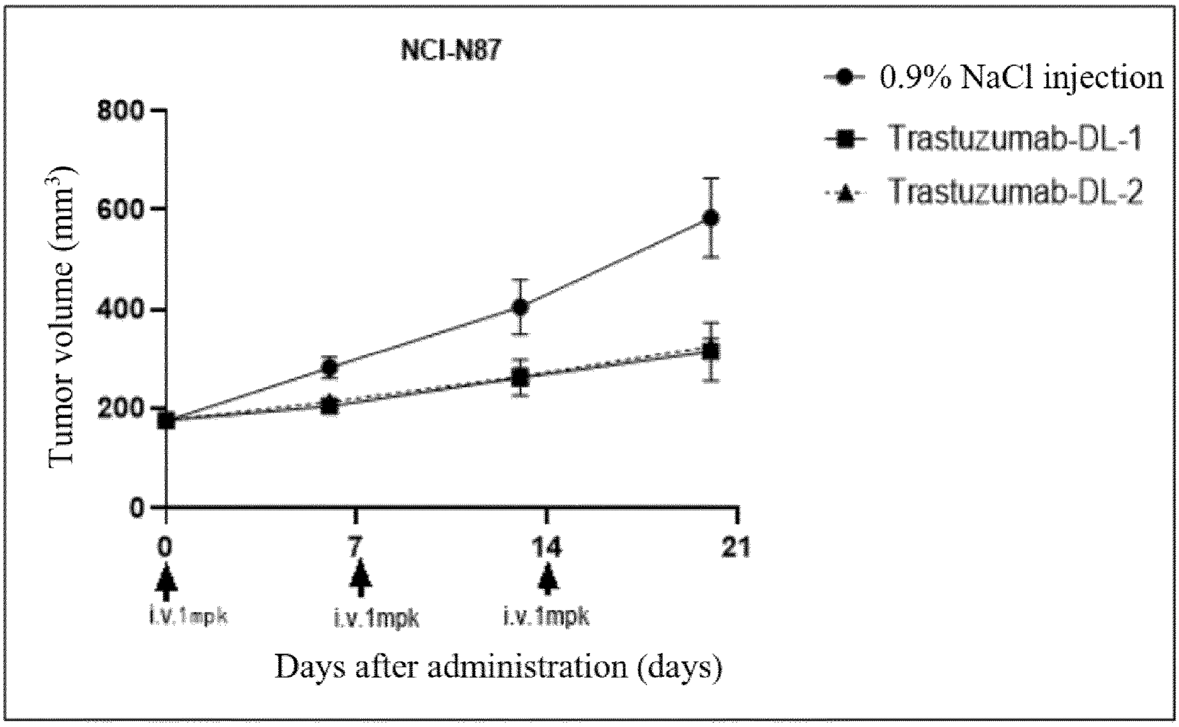


**Figure 3**

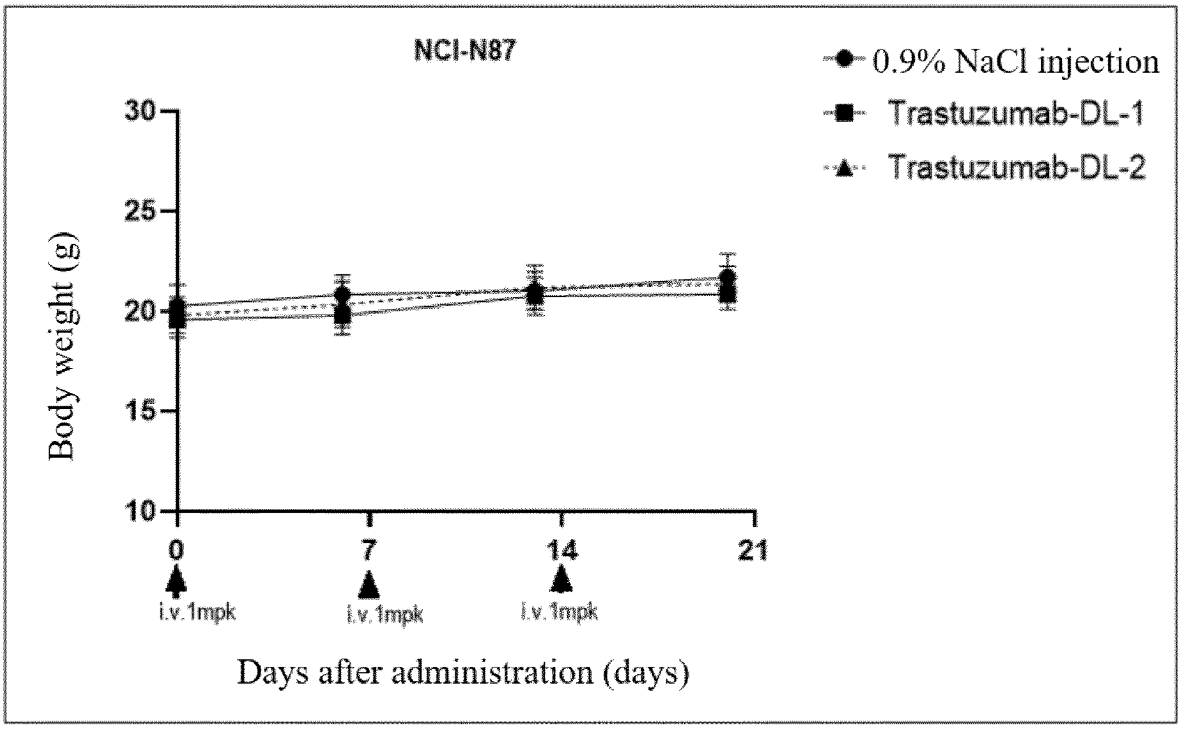


**Figure 4**

**INTERNATIONAL SEARCH REPORT**

International application No.
**PCT/CN2024/078293**

**A. CLASSIFICATION OF SUBJECT MATTER**

C07D 211/96(2006.01)i; A61K 31/445(2006.01)i; A61P 35/00(2006.01)i; A61P 37/00(2006.01)i

According to International Patent Classification (IPC) or to both national classification and IPC

**B. FIELDS SEARCHED**

Minimum documentation searched (classification system followed by classification symbols)

IPC: C07D211/-; A61K31/-; A61P35/-; A61P37/-

Documentation searched other than minimum documentation to the extent that such documents are included in the fields searched

Electronic data base consulted during the international search (name of data base and, where practicable, search terms used)

CNKI, CNTXT, ENTXT, DWPI, STN(CAPLUS, REGISTRY); 抗体, 偶联, 药物, 连接, 哌啶, 六氢吡啶, 磺酰, 酰胺, 胺, 酯, antibody, drug, conjugate, ADC, piperidine, sulfonyl, amide, amine, ester, 根据式I-III的结构式检索, search according to structural formulae I-III

**C. DOCUMENTS CONSIDERED TO BE RELEVANT**

| Category* | Citation of document, with indication, where appropriate, of the relevant passages | Relevant to claim No. |
|---|---|---|
| A | CN 114829345 A (JAPAN TOBACCO INC.) 29 July 2022 (2022-07-29) entire document, in particular description, paragraph [0514], Reference Production Example 2 | 1-20 |
| A | CN 110090308 A (SICHUAN KELUN-BIOTECH BIOPHARMACEUTICA CO., LTD.) 06 August 2019 (2019-08-06) entire document | 1-20 |
| A | CN 111295389 A (SICHUAN KELUN-BIOTECH BIOPHARMACEUTICA CO., LTD.) 16 June 2020 (2020-06-16) entire document | 1-20 |
| A | WO 2022253033 A1 (SICHUAN KELUN BIOTECH BIOPHARMACEUTICAL CO., LTD.) 08 December 2022 (2022-12-08) entire document | 1-20 |
| A | CN 1173179 A (SANOFI WINTHROP INC.) 11 February 1998 (1998-02-11) entire document | 1-20 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **31 May 2024** | **31 May 2024** |

| Name and mailing address of the ISA/CN | Authorized officer |
|---|---|
| **China National Intellectual Property Administration (ISA/CN)** **China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/078293**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **21**
because they relate to subject matter not required to be searched by this Authority, namely:
The subject matter of claim 21 is a method for preventing or treating a disease, and belongs to subject matter for which a search is not required (PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/078293**

| Patent document cited in search report | Publication date (day/month/year) | Patent family member(s) | Publication date (day/month/year) |
|---|---|---|---|
| CN 114829345 A | 29 July 2022 | EP 4082614 A1 | 02 November 2022 |
| | | EP 4082614 A4 | 24 January 2024 |
| | | WO 2021132577 A1 | 01 July 2021 |
| | | US 2023399319 A1 | 14 December 2023 |
| | | JPWO 2021132577 A1 | 01 July 2021 |
| CN 110090308 A | 06 August 2019 | WO 2019149116 A1 | 08 August 2019 |
| | | CN 111447939 A | 24 July 2020 |
| | | CN 110090308 B | 24 March 2023 |
| CN 111295389 A | 16 June 2020 | WO 2019114666 A1 | 20 June 2019 |
| | | EP 3725798 A1 | 21 October 2020 |
| | | EP 3725798 A4 | 10 November 2021 |
| | | US 2020347075 A1 | 05 November 2020 |
| | | US 2021101906 A2 | 08 April 2021 |
| | | US 11970506 B2 | 30 April 2024 |
| | | JP 2024038168 A | 19 March 2024 |
| | | KR 20200099123 A | 21 August 2020 |
| | | CA 3080236 A1 | 20 June 2019 |
| | | US 2023357259 A1 | 09 November 2023 |
| | | JP 2021506743 A | 22 February 2021 |
| | | JP 7446993 B2 | 11 March 2024 |
| WO 2022253033 A1 | 08 December 2022 | EP 4349832 A1 | 10 April 2024 |
| | | KR 20240017343 A | 07 February 2024 |
| CN 1173179 A | 11 February 1998 | US 5541168 A | 30 July 1996 |
| | | WO 9616970 A1 | 06 June 1996 |
| | | CA 2205950 A1 | 06 June 1996 |
| | | MX 9704035 A | 28 February 1998 |
| | | NZ 297344 A | 25 November 1998 |
| | | NO 972450 D0 | 29 May 1997 |
| | | AU 4248596 A | 19 June 1996 |
| | | AU 704233 B2 | 15 April 1999 |
| | | HUT 78043 A | 28 July 1999 |
| | | EP 0794956 A1 | 17 September 1997 |
| | | EP 0794956 A4 | 11 February 1998 |
| | | JP 2001520626 A | 30 October 2001 |
| | | FI 972310 A0 | 30 May 1997 |
| | | FI 972310 A | 21 July 1997 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 202310181337
- CN 202311231478
- WO 2012007896 A1
- WO 2013173392 A1


### Non-patent literature cited in the description


- **STAHL** ; **WERMUTH**. Handbook of Pharmaceutical Salts: Properties, Selection, and Use. Wiley-VCH, 2002
- **T. L. GILCHRIST**. Comprehensive Organic Synthesis. Academic Press, vol. 7, 748-750
- **G. W. H. CHEESEMAN** ; **E. S. G. WERSTIUK**. Advances in Heterocyclic Chemistry. Academic Press, vol. 22, 390-392
- **T. HIGUCHI** ; **V. STELLA**. Pro-drugs as Novel Delivery Systems. *ACS Symposium Series*, vol. 14
- **H. BUNDGAARD**. Design of Prodrugs. Elsevier, 1985
- **T.W. GREENE** ; **P.G.M. WUTS**. Protective Groups in Organic Synthesis. John Wiley & Sons, 1991
- **GREENE et al.** Protective Groups in Organic Synthesis. Wiley-Interscience, 2006